(19) Europäisches Patentamt
European Patent Office
Office européen des brevets

(11) EP 2 415 869 A1

(12) **EUROPEAN PATENT APPLICATION**
published in accordance with Art. 153(4) EPC

(43) Date of publication:
**08.02.2012 Bulletin 2012/06**

(21) Application number: **10757994.8**

(22) Date of filing: **30.03.2010**

(51) Int Cl.:
***C12N 15/113*** *(2010.01)* ***A61K 48/00*** *(2006.01)*
***A61K 31/7115*** *(2006.01)* ***A61K 31/712*** *(2006.01)*
***A61K 31/7125*** *(2006.01)* ***A61K 31/713*** *(2006.01)*
***A61P 35/00*** *(2006.01)* ***C12Q 1/68*** *(2006.01)*

(86) International application number:
**PCT/CN2010/000405**

(87) International publication number:
**WO 2010/111891 (07.10.2010 Gazette 2010/40)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **03.04.2009 CN 200910081145**
**03.04.2009 CN 200910081144**

(71) Applicant: **Biomics Biotechnologies Co., Ltd.**
**Nantong, Jiangsu 226016 (CN)**

(72) Inventors:
- **LIANG, Zicai**
**Beijing 100871 (CN)**
- **DU, Quan**
**Beijing 100871 (CN)**

(74) Representative: **Leathley, Anna Elisabeth**
**Dehns**
**St Bride's House**
**10 Salisbury Square**
**London**
**EC4Y 8JD (GB)**

# (54) MODIFIED OLIGO-NUCLEIC ACID MOLECULE, PREPARATION METHOD AND USES THEREOF

(57) The present invention relates to a modified oligonucleotide, its preparation and application. The invention eables stabilizing the oligonucleotide by introducing a relatively small amount of modified nucleotide at specific UA/UA and/or CA/UG and/or UG/CA site of the oligonucleotide, therefore to decrease the modification-related cytotoxicity and compromising effects on the biological activity.

EP 2 415 869 A1

Sequence Listing

<110> Peking University

<120> Modified Oligonucleotide and its Preparation and Application

<130> 20100320

<150> 200910081145.8
<151> 2009-04-03

<150> 200910081144.3
<151> 2009-04-03

<160> 10

<170> PatentIn version 3.3

<210> 1
<211> 1500
<212> DNA
<213> Homo sapiens

<400> 1
gccgagcccg ggcccagcgc cgcctgcagc ctcgggaagg gagcggatag cggagccccg 60

agccgcccgc agagcaagcg cggggaacca aggagacgct cctggcactg cagataactt 120

gtctgcattt caagaacaac ctaccagaga ccttacctgt caccttggct ctcccaccca 180

atggagatgg ctctaatggt ggcacaaacc aggaagggga aatctgtggt ttaaattctt 240

tatgcctcat cctctgagtg ctgaaggctt gctgtaggct gtatgctgtt aatgctaatc 300

gtgatagggg tttttgcctc caactgactc ctacatatta gcattaacag tgtatgatgc 360

ctgttactag cattcacatg gaacaaattg ctgccgtggg aggatgacaa agaagcatga 420

gtcaccctgc tggataaact tagacttcag gctttatcat ttttcaatct gttaatcata 480

atctggtcac tgggatgttc aaccttaaac taagttttga aagtaaggtt atttaaaaga 540

tttatcagta gtatcctaaa tgcaaacatt ttcatttaaa tgtcaagccc atgtttgttt 600

ttatcattaa cagaaaatat attcatgtca ttcttaattg caggttttgg cttgttcatt 660

ataatgttca taaacacctt tgattcaact gttagaaatg tgggctaaac acaaatttct 720

ataatatttt tgtagttaaa aattagaagg actactaacc tccagttata tcatggattg 780

tctggcaacg ttttttaaaa gatttagaaa ctggtacttt cccccaggta acgattttct 840

gttcaggcaa cttcagttta aaattaatac ttttatttga ctcttaaagg gaaactgaaa 900

ggctatgaag ctgaattttt ttaatgaaat atttttaaca gttagcaggg taaataacat 960

ctgacagcta atgagatatt ttttccatac aagataaaaa gatttaatca aaaaatttca 1020

tatttgaaat gaagtcccaa atctaggttc aagttcaata gcttagccac ataatacggt 1080

tgtgcgagca gagaatctac ctttccactt ctaagcctgt ttcttcctcc atatggggat 1140

aatactttac aaggttgttg tgaggcttag atgagataga gaattattcc ataagataat 1200

caagtgctac attaatgtta tagttagatt aatccaagaa ctagtcaccc tactttatta 1260

gagaagagaa aagctaatga tttgatttgc agaatattta aggtttggat ttctatgcag 1320

tttttctaaa taaccatcac ttacaaatat gtaaccaaac gtaattgtta gtatatttaa 1380

tgtaaacttg ttttaacaac tcttctcaac attttgtcca ggttattcac tgtaaccaaa 1440

taaatctcat gagtctttag ttgatttaaa ataaaaaaaa aaaaaaaaaa aaaaaaaaaa 1500

<210> 2
<211> 6047
<212> DNA
<213> Homo sapiens

<400> 2
ggggtctcgg cgatcgctgc tcctcctcct ccttctcctc ctctttttc tcctccgcct 60

cctccccccg ccgcctcgcc accgccgcgg ctagggctgg aggcgccgct gtcattcctg 120

tgccggagga accggcgctg ccggtgcctg gggtcgggg cgcgggcgaa gccgggccgc 180

ggaggacaca acaggtagag ccgggggtgc ccggccgcgc gccccccgcg catcatgcag 240

ctctttgtca cctctctcgc ccccaggcca aaatcctgag catgatggaa gacaataagc 300

agctcgcgct ccgcatcgat ggggcggtcc agtcggccag ccaggaggtg accaacctgc 360

gagccgaact cacggccacc aaccggagac tggcggaact gagcggcggc ggcggccccg 420

gcccgggccc gggagccgcg gccagcgcct cggcggcggg ggactcggcg gcgacgaaca 480

tggagaaccc ccagcttgga gcgcaagtgc tcctgcggga agaagtgtcg cggctccagg 540

aggaagttca ccttctccgg cagatgaagg agatgttggc gaaggacctg gaggagtcgc 600

agggcggcaa gtcctctgag gtcctctcgg ccaccgagct cagggtccag ctggcccaga 660

aggagcagga gctagccaga gccaaagaag ccttgcaggc catgaaagct gatcggaagc 720

gcttaaaggg cgagaagaca gacctggtga gccagatgca gcagctgtat gccacactgg 780

agagccgcga ggagcagctc cgagacttca tccgcaacta tgagcagcac cgcaaggaga 840

gcgaggatgc ggtcaaagcg ctggccaagg agaaggacct gctggagcgt gagaagtggg 900

agctgcggcg ccaagccaag gaggccacag accacgccac ggcactgcgc tcccagctgg 960

acctcaagga caaccggatg aaggagctgg aggccgagct ggccatggcc aaacagtcct 1020

tagctacgct gaccaaggac gtccccaagc ggcattccct cgccatgccg ggcgagacgg 1080

tgctcaatgg caaccaggag tgggtggtgc aggcggacct cccgctgacc gcagccatcc 1140

ggcagagtca acagactctc taccactcac acccccctca ccctgcggac cggcaagcgg 1200

tcagggtgag cccctgccac tcccggcagc cctctgtcat ctccgacgca tctgccgccg 1260

aaggcgaccg gtcgtccaca ccgagcgaca tcaactcccc tcgacaccgg acacactccc 1320

tctgcaacgg cgacagtccc ggcccagttc agaagaacct gcacaaccct attgtacagt 1380

cactagagga tcttgaagac caaaaacgga aaaagaagaa agagaagatg ggattcggct 1440

ccatctcccg cgtcttcgcc agagggaagc agcggaagtc cctcgacccc ggcctctttg 1500

atgactcgga cagccagtgc agccccacgc ggcagagcct cagcctgtcg gaaggcgagg 1560

agcagatgga ccggctgcag caggtggagc tggtgaggac cacccctatg tcccactgga 1620

aggcgggcac cgtccaggcc tggctggagg tggtgatggc catgcctatg tacgtcaagg 1680
cctgcacgga gaacgtgaag agcgggaagg tgctgctgag cctgagtgac gaggacctgc 1740
agctgggcct tggggtgtgc agctccctgc accggcgcaa gctgcgcctg gccatcgagg 1800
actaccgtga tgccgaggca ggccgcagcc tgtccaaagc tgccgagctg gaccatcact 1860
gggtggccaa ggcctggctg aatgacattg gcctgtccca gtactcccag gcctttcaga 1920
accacctggt tgatgggcgg atgctgaatt ccctgatgaa gcgagacctg gagaagcacc 1980
tgaacgtgtc caagaagttc caccaggtca gcatcctgct ggggatcgag ctgctgtacc 2040
aagtgaactt cagcagggag gccctccagg agcgccgggc ccgctgcgag acgcagaaca 2100
ttgaccccgt ggtgtggacc aaccagcggg tgctcaagtg ggttcgagac atcgacctga 2160
aggagtacgc agacaacctg accaacagcg gcgtccatgg tgctgtgctg gtgctggagc 2220
ccacattcaa tgccgaggcc atggccactg ccctgggcat ccccagtggg aagcacatcc 2280
tccggagaca cctggcagag gagatgagcg ccgtcttcca cccagccaac tccacaggca 2340
tccgggaggc tgagcgtttt ggaacgcccc ctggcagggc ctccagcgtc acgcgggcag 2400
gaaaggagga gaacagcagc ggtctcaagt acaaggctgg ccggctgccc ctggggaaga 2460
taggaagggg cttcagcagc aaagatcccg atttccatga tgactatggc tctcttcaaa 2520
acgaagattg cggagacgat gacccccaga gcaggctgga acagtgccgt ctggaaggct 2580
acaacagcct ggaggtcacc aacgtgtaag gaactggtgg ctccaccaga cccaacgtga 2640
gagacccagg aaggaagaga agccagatgg ccccaggtgt cgttctcact gtacatagcg 2700
gccgcaggct gaggatgtcc cttgctcctg ggcaaaatcc cgatggactc tgcggtttca 2760
gctccacagc gcccaggaga gagaagacac cagcccacct gtcttgggtg ggccatggac 2820
tttcctgttc agctggagat gggcccagag gacctgtcac agtgtccggc cctgcctcca 2880
tccaggatac acaggctcca cctcagagtg accgtcactg tggagcagcc aagcagtccc 2940
tggagcctta aacggagctg ccaaggtggg aggaggccca cagttcccta aaacaccctt 3000
ccggcgggag caggggggac cccaacccca caccccagcg cccagtgcat tggcagagcc 3060
gggtgcagga agtgctgcct cttgccgaga cgtcggacag ggcgggggtt ggggaactct 3120
cggctacagc atcttaccct tgactgagaa cttgggtcct gacttggctc actgaatctc 3180
tcttgggaga atgcaaaatc cttccacctg aaaagctctg tgacacatgg gggtggacgt 3240
attgaagagc tgtttgccga tccacccagg agtggctacg ctgagtgggg agccggtgaa 3300
tgatccgtgc aggagtgggg cttagcagcc acatttctag gagatgcaga tatcctatca 3360
ccagaatgaa agctattggg acaacaggat cggggatgac cgacggcccc atatggtgaa 3420
tctctggcct gtggtttggc tttactgaga ttccaaaccc cactatctgc actccgtgac 3480
agtggtatgg agtgtggcaa tgagtttggg gtctggggca gggaaatgct tgacattgtt 3540
aacccaacaa acctttgttg tgatgtccct gtcacctgaa acataggtga catagctcac 3600
caatgtccta accgagacac aaactccaca gagcaaaatc atttggtatt ggtggggaga 3660

accccagccc ttttcttgac ctgccactgt tatgctgtgt ggcttcttcc cagtggcctc 3720
acctctctgt gcctcgatgt cttcatctac gatacttctg gttccctccc agggacatcg 3780
tgaggattaa cacttgctaa tatctgtaac acaatttgta acctctcagg agacaatggg 3840
aagttatggg gtagctaatt tcccatttac aacacagaaa tgatatagag ctagttcgct 3900
ccaactcttt aggttgaagc agtgtgcaaa aggaagaaaa gaaatgttta atgttcagac 3960
ctgccaagag cctccaacag ggctcaagaa acatataaat cccatgagca cagccttgaa 4020
aaccagtttg actcaagcct tcgggcctca gttcattgac cagatgacag ccacgtgatg 4080
attagggaag gacggatgca ttgcgattct gcttacacat cgggttatca aagcgagtca 4140
cttgttggaa ccatgatgct cgacctcctt caaggccgtt tgcactgggg cttgagtttc 4200
caagattcac aacaggtgtc agcctctgag aaccctcaaa gcgtgtgttc ttcaacctgg 4260
caaattgttt cctctcatgg gggaagccga gctctgatga acttgagaat tacacctctc 4320
tcatgccgaa gaccgtggtg ttccccctaa tgacataaac gcagcctttc ttgctgtctg 4380
agaccaaatg tctagttggt agacaggtgg atgtttggcc tcctaagggc acacttctga 4440
tcctgggccc caggtggtga atctctggca tgtggcttgg ctttgttgag actccaaatt 4500
ccattatctt catgacattc ggcctcatcc atagggtcct gaagctgcag tccacagctc 4560
agaaaggaga ggtgagacct ccctccaacc tggtgccaca ggtctctccc aagccacatc 4620
cagcctggat gacctgggac cccagaaact gccgtttggg aggcagcaac agcaacgtgc 4680
ccaggcaggc agttattccc acagagtgag ccagaattgt agcagggcac ttgaatgcag 4740
agctgatgat ttgaaaccaa cgttcaccca acttgtcaga aatggcactt acatggttcg 4800
atcttgctgg agacaagtgg acaattgggg gtcactggca gagacagtat tgcccaaaat 4860
gttcacagca ggaggccagc aggcctgagg caacacgggc aaccgcgaat gcctcttttg 4920
gtttaaatta tgccatcaca accctctttc acccatgagg ctccccatcc ctgacagcca 4980
ggtgagcatt tggagctggt ttctcaacat gaggatgggt tggttgttaa attaacaacc 5040
tccacagtat cagattgagt gagctttgtc tgctggaaaa acctgaaacg tcaactctgc 5100
ttcaaggtcg gcaagaagaa cagaaggcgg agacttggca gagagactca agctgattgt 5160
cacaggctac agaggggcca gctccagaac agtgaccagc tacatcctgt ccaagcagcc 5220
cgagtgtggt cttggtccct gcagggcaat gtgggcatct ggacctgggg acgatgtgga 5280
tgcacttctt ggaaagctgt tgtagcttgt gcctgtgggt ggagaaggca cctgcctggt 5340
agactctcag ctttctgacc cccaggagcc tctgcgaggc ccctttgtcc ttggctgagc 5400
cggacctttc ttttggaaat ctgtctgtct gttggcatcg ctgttttcag accccaggct 5460
gcagaggagg ggagaagcca cacaacaatc tggacccaat aaagtggaga gaagggcgtc 5520
tctacacagc ccggccagcg tggagggccc caggacaggg acccaaaagc ttgacgtcac 5580
tgaacagggc tgggtactgg cagaacagga agatttggcc agaggtgacc tcagtgttcc 5640
ctccaggggc atccaggccc ctctgacctg ggagaagaa ggcccatgct caggcccacc 5700

tccctcttcc catcagagcc catgcgtcct gggcaccacc acttccactc tgcttttcga 5760

ggctccggag ggctcttcct gctgtgaaag gaaaggagaa gaaagcctgt gggcaatggc 5820

aacctctgag tctggcattc ttgccaatgg ctggccagcg aggagaatct cccgagccct 5880

gacacacaaa ggcattttgt ggctgcagag gaaatgggtt ggctctgaac aaagatgcag 5940

tttctagggc cgtggcccca aatcgcttcc ccgagagtga attttaacac tgtaacaata 6000

aatactactg cacagcactt taaaaaaaaa aaaaaaaaaa aaaaaaa 6047

<210> 3
<211> 2413
<212> DNA
<213> Homo sapiens

<400> 3
cttcttcgtc agcctccctt ccaccgccat attgggccac taaaaaaagg gggctcgtct 60

tttcggggtg tttttctccc cctcccctgt ccccgcttgc tcacggctct gcgactccga 120

cgccggcaag gtttggagag cggctgggtt cgcgggaccc gcgggcttgc acccgcccag 180

actcggacgg gctttgccac cctctccgct tgcctggtcc cctctcctct ccgccctccc 240

gctcgccagt ccatttgatc agcggagact cggcggccgg gccggggctt ccccgcagcc 300

cctgcgcgct cctagagctc gggccgtggc tcgtcggggt ctgtgtcttt tggctccgag 360

ggcagtcgct gggcttccga gaggggttcg ggctgcgtag gggcgctttg ttttgttcgg 420

ttttgttttt ttgagagtgc gagagaggcg gtcgtgcaga cccgggagaa agatgtcaaa 480

cgtgcgagtg tctaacggga gccctagcct ggagcggatg gacgccaggc aggcggagca 540

ccccaagccc tcggcctgca ggaacctctt cggcccggtg gaccacgaag agttaacccg 600

ggacttggag aagcactgca gagacatgga agaggcgagc cagcgcaagt ggaatttcga 660

ttttcagaat cacaaacccc tagagggcaa gtacgagtgg caagaggtgg agaagggcag 720

cttgcccgag ttctactaca gacccccgcg gccccccaaa ggtgcctgca aggtgccggc 780

gcaggagagc caggatgtca gcgggagccg cccggcggcg cctttaattg gggctccggc 840

taactctgag gacacgcatt tggtggaccc aaagactgat ccgtcggaca gccagacggg 900

gttagcggag caatgcgcag gaataaggaa gcgacctgca accgacgatt cttctactca 960

aaacaaaaga gccaacagaa cagaagaaaa tgtttcagac ggttccccaa atgccggttc 1020

tgtggagcag acgcccaaga agcctggcct cagaagacgt caaacgtaaa cagctcgaat 1080

taagaatatg tttccttgtt tatcagatac atcactgctt gatgaagcaa ggaagatata 1140

catgaaaatt ttaaaaatac atatcgctga cttcatggaa tggacatcct gtataagcac 1200

tgaaaaacaa caacacaata acactaaaat tttaggcact cttaaatgat ctgcctctaa 1260

aagcgttgga tgtagcatta tgcaattagg tttttcctta tttgcttcat tgtactacct 1320

gtgtatatag tttttacctt ttatgtagca cataaacttt ggggaaggga gggcagggtg 1380

gggctgagga actgacgtgg agcggggtat gaagagcttg ctttgattta cagcaagtag 1440

ataaatattt gacttgcatg aagagaagca attttgggga agggtttgaa ttgttttctt 1500

taaagatgta atgtcccttt cagagacagc tgatacttca tttaaaaaaa tcacaaaaat 1560

ttgaacactg gctaaagata attgctattt atttttacaa gaagtttatt ctcatttggg 1620

agatctggtg atctcccaag ctatctaaag tttgttagat agctgcatgt ggctttttta 1680

aaaaagcaac agaaacctat cctcactgcc ctccccagtc tctcttaaag ttggaattta 1740

ccagttaatt actcagcaga atggtgatca ctccaggtag tttggggcaa aaatccgagg 1800

tgcttgggag ttttgaatgt taagaattga ccatctgctt ttattaaatt tgttgacaaa 1860

atttctcat tttcttttca cttcgggctg tgtaaacaca gtcaaaataa ttctaaatcc 1920

ctcgatattt ttaaagatct gtaagtaact tcacattaaa aaatgaaata ttttttaatt 1980

taaagcttac tctgtccatt tatccacagg aaagtgttat ttttcaagga aggttcatgt 2040

agagaaaagc acacttgtag gataagtgaa atggatacta catctttaaa cagtatttca 2100

ttgcctgtgt atggaaaaac catttgaagt gtacctgtgt acataactct gtaaaaacac 2160

tgaaaaatta tactaactta tttatgttaa aagatttttt ttaatctaga caatatacaa 2220

gccaaagtgg catgttttgt gcatttgtaa atgctgtgtt gggtagaata ggttttcccc 2280

tcttttgtta aataatatgg ctatgcttaa aaggttgcat actgagccaa gtataatttt 2340

ttgtaatgtg tgaaaaagat gccaattatt gttacacatt aagtaatcaa taaagaaaac 2400

ttccatagct att 2413

<210> 4
<211> 600
<212> DNA
<213> Pan troglodytes

<400> 4
atgaagcaca gcctccccga cctgccctac gactacggcg ccctggaacc tcacatcaac 60

gcgcagatca tgcagctgca ccacagcaag caccacgcgg cctacgtgaa taacctgaac 120

gtcaccgagg agaagtacca ggaggcgttg gccaagggag atgttacagc ccagatagct 180

cttcagcctg cactgaagtt caatggtggt ggtcatatca atcatagcat tttctggaca 240

aacctcagcc ctaacggtgg tggagaaccc aaaggggagt tgctggaagc catcaaacgt 300

gactttggtt cctttgacaa gtttaaggag aagctgacgg ctgcatctgt tggtgtccaa 360

ggctcaggtt ggggttggct tggtttcaat aaggaacggg gacacttaca aattgctgct 420

tgtccaaatc aggatccact gcaaggaaca acaggcctta ttccactgct ggggattgat 480

gtgtgggagc acgcttacta ccttcagtat aaaaatgtca ggcctgatta tctaaaagct 540

atttggaatg taatcaactg ggagaatgta actgaaagat acatggcttg caaaaagtaa 600

<210> 5
<211> 3233
<212> DNA
<213> Macaca mulatta

<220>

<221> misc_feature
<222> (2570)..(2654)
<223> n is a, c, g, or t

<400> 5
aaaacttctg ggagcctcca aactcctagc tgtcttgtcc ctcgccctgg agagaaggca 60
gaaccatggc attttattgc tgcttctggg tcctcttggc actcacctgg cacacctctg 120
cctatgggcc cgaccagcga gcccaaaaga aaggggacat tatacttggc gggctctttc 180
ctattcattt tggagtagca gctaaagatc aagatctcaa atcaaggccg gagtctgtgg 240
aatgcatcag gtataatttc cgtgggtttc gctggttaca ggctatgata tttgccatag 300
aggagataaa cagcagccca gcccttcttc ccaacttgac gctgggatac aggatatttg 360
acacttgcaa caccgtttct aaggccttgg aagccaccct gagttttgtt gctcaaaaca 420
aaattgattc tttgaacctt gatgagttct gcaactgctc agagcacatt ccctctacga 480
ttgctgtggt gggagcaact ggctcaggcg tctccacggc agtggcaaat ctgctggggc 540
tcttctacat tccccaggtc agttacgcct cctccagcag actccttagc aacaagaatc 600
aattcaagtc cttcctccga accatcccca atgatgagca ccaggccact gccatggcag 660
acatcattga gtatttccgc tggaactggg tgggcacaat tgcagctgat gatgactatg 720
ggcggccagg gattgagaag ttccgagagg aagctgagga aagggacatc tgcattgact 780
tcagtgaact catctcccag tactctgatg aggaagagat ccagcatgtg gtggaggtga 840
ttcaaaattc cacggccaaa gtaatcgtgg ttttctccag tggcccagac cttgagcccc 900
tcatcaagga gattgtccgg cgcaatatca caggcaagat ctggctggcc agtgaggcct 960
gggccagctc ctccctgatc gccatgcccg agtacttcca cgtggttgga ggcaccattg 1020
gatttgctct gaaggctggg cagatcccag gctttcggga attcctgaag aaggtccatc 1080
ccaggaagtc tgtccacaat ggttttgcca aggagttttg ggaagaaaca tttaactgcc 1140
acctccaaga aggtgccaaa ggacctttac ctgtggacac ctttctgaga ggtcacgaag 1200
aaagtggagg caggtttagc aacagttcga cagccttccg acccctctgt acaggggatg 1260
agaacatcag cagtgttgag accccttaca tagattacac acatttacgg atatcctaca 1320
atgtgtactt agcagtctac tccattgccc atgccttgca agatatatat acctgcttac 1380
ctgggagagg gctcttcacc aacggctcct gtgcagacat caagaaagtt gaggcgtggc 1440
aggtcctgaa gcacctacgg cacctaaact ttaccaacaa tatgggggag caggtgacct 1500
ttgatgagtg tggtgacctg gtggggaact attccatcat caactggcac ctctccccag 1560
aggatggctc catcgtgttt aaggaagtcg ggtattacaa cgtctatgcc aagaagggag 1620
aaagactctt catcaacgag gagaaaatcc tgtggagtgg gttctccagg gaggtgccct 1680
tctccaactg cagccgagac tgtctggcag ggaccaggaa agggatcatt gagggggagc 1740
ccacctgctg ctttgagtgt gtggagtgtc ctgatgggga gtacagcgat gagacagatg 1800
ccagtgcctg taacaagtgc ccagatgact tctggtccaa tgagaaccac acctcctgca 1860
ttgccaagga gatcgagttt ctgtcgtgga cagagccctt tgggattgca ctcaccctct 1920

ttgccgtgct gggcattttc ctgacagcct ttgtgctggg tgtgtttatc aagttccgca 1980
acacgcccat cgtcaaggcc acgaaccgag agctctccta ccttctcctc ttctccctgc 2040
tctgctgctt ctccagctcc ctgttcttca ttggggagcc ccaggactgg acttgccgcc 2100
tgcgccaacc ggcctttggc atcagcttcg tgctctgcat ctcgtgcatc ctggtgaaaa 2160
ccaaccgtgt cctcctggtg tttgaggcca agatccccac cagcttccac cgcaagtggt 2220
gggggctcaa cctgcagttt ctgctggttt tcctctgcac cttcatgcag attgtcatct 2280
gtgtgatctg gctctacacc gcgccccct caagctaccg caaccacgag ctggaggatg 2340
agatcatctt catcacgtgc cacgagggct cactcatggc cctgggcttc ctgatcggct 2400
acacctgcct gctggctgcc atctgcttct tctttgcctt caagtcccgg aagctgccag 2460
agaacttcaa tgaagccaag ttcatcacct tcagcatgct catcttcttc attgtctgga 2520
tctccttcat tccagcctat gccagcacct acggcaagtt tgtctctgcn nnnnnnnnnn 2580
nnnnnnnnnn nnnnnnnnnn nnnnnnnnnn nnnnnnnnnn nnnnnnnnnn nnnnnnnnnn 2640
nnnnnnnnnn nnnnccatcc cgcaacacca tcgaggaggt gcgttgcagc accgcagctc 2700
acgctttcaa ggtggctgcc cgggccacgc tgcgccgcag caacgtctcc cgcaagcggt 2760
ccagcagcct tgggggctcc actggatcca ccccctcctc ctccatcagc agcaagagca 2820
acagcgaaga cccattccca cagcccgaga ggcagaagca gcagcagccg ctgccaagat 2880
gcaagcagaa ggtcatcttc ggcagtggca cggtcacctt ctcactgagc ttcgatgagc 2940
ctcagaagaa cgccatggcc cacaggaatt ctacacacca gaactccctg gaggcccaga 3000
aaagcagcga tacgctggcc cgacaccagg cattactccc gctgcagtgc ggggaagcgg 3060
actcagatct gagcgtccaa gaaacaggtc tgcaaggacc agtgggtgga gaccaccggc 3120
cagaggtgga ggtccctgaa gagttgtccc cagcacttgt agtgtccagt tcacagagct 3180
ttgtcatcag cggtggagga agcactgtta cagaaaatgt actgcattca taa 3233

<210> 6
<211> 2694
<212> DNA
<213> Canis familiaris

<400> 6
gggggggggg ggccggactc aactctactg tggagtcggg gccatccctc atttttctca 60
ctcaggagcg gctggctggc ggcggctggc tggcggcagg agctggcaat gtttggcctc 120
aagagaaacg cagtaatccg gactcaactc tactgtgggg gggccgggct gggggccggc 180
agcggcggcg cctcctcttc gggagggcgg cttttggctt cggggaggga ggccacgacc 240
agacgggagg gagggggagg ggaagccggt gcggtgattg gcggaagcgc cggcgcaagt 300
cccccgacca ctctggcgcc ggacgcccgg agggtcgcgc ggccctcacc cattggcgct 360
gagggcccca acgtcagcgc gacccccccg aggctgctgc tgctcgcgcc cccctgccgc 420
gcgtcgccgc ctgaagagat ggaaggcccg gccgccgacg ccatcatgtc gcccgaagag 480

```
gagctagacg ggtacgagcc ggaacctttg gggaagcggc cggcggtcct gcctctgctg    540
gagctggtgg gggaggccag cagtggcccc ggcatggacg gctcgctacc ctcgacgcca    600
cccccggcgg aggaggagga agatgagttg taccggcagt ccctggagat tatctctcgg    660
taccttcggg aacaggccac aggcgccaag gacgcgaaac cactgggcgg gtctcgggcg    720
gccagccgga aggcgttaga gaccctccag cgagtcgggg acggggtaca gcgcaaccac    780
gagacagcct tccaaggcat gcttcggaaa ctggacatca aaaacgaaga cgatgtcaaa    840
tcgttgtctc gagtgattgt ccatgttttc agtgacggag taacaaactg ggcaggatt     900
gtgactctta tttcctttgg tgcctttgtg gccaaacact tgaagagtat aaaccaagaa    960
agctgcatcg aaccattagc agaaagcatc acagatgttc tcgtaaggac gaaacgagac   1020
tggctagtca aacaaagagg ctgggatggg tttgtggagt tcttccatgt agaggaccta   1080
gaaggcggca tcagaaatgt gctgctggct tttgcaggtg ttgctggagt aggagctggt   1140
ttggcatatc taataagata gccttttaag tgcaataatt aacttttaaa caaccccagg   1200
caccaaaacc acatatgact gctgtgaaat caaatgtatt tatgaagttg gactttgagc   1260
tgtccaggct gtaacctcgg agagttctac tctagcaacg tagaaaagca agtggcaaga   1320
ggattatggc taacaggaat aaatacatgg gagaagtagt ccccccttgaa gagtcactgt   1380
ctaaaagaag cagagctcag tttcagcaac aggcaaactt ggggaggcca tggaggagga   1440
cttttagatt tagtaaagtt ggtagggttg aagagactta attttcttgt ctagaacagg   1500
agagtggcca gtagccaggc tagtcataga gtccattaaa tatgtccact gaattcatta   1560
acttcccata tagtgttaaa agagaagcac taacaatggc attgatctgt atgaaatgga   1620
tttaagctac aggtgataga actatgacta gaagccgcag tactgtacaa cagtgtgaag   1680
gaaagctttt tctctgtaat tagctttccc aagtatactt cttgaaagtc caagtgctca   1740
ggacgtttta cctgttctac tttggcttgg tttgagtggt ttagtttatt agcctagtaa   1800
tggccaacaa tacttgactt agggtcaata attacaattg caaatgtggg aatagcctca   1860
atttttaagg caaaaacaaa ttataaatgt atttgtctgt aaaaattgtg tatattttta   1920
cagaaagtct atttctttga aatgtaaagg gatgaagagt ctcaaaatat attagttttt   1980
ttttcatgcc cgtttgaaat ttacaacttc tgtagttagg aatctatttc ttacagcttt   2040
tctaaatttt gtcctggtca gttctagatt gtatacagaa ccaattgatg taattgtatg   2100
caacttggtt gtagtggaac aattccaatt cataactatg cagacttttt aattttccta   2160
tctgattggt aagtattctt tagtggtttt gggtgttttg ttttgtttta aacctgggat   2220
tgagaagttg atgaatggaa attcatcctt taacttcatt acatgtgggt ttacaataat   2280
tgagtcaagg tgaagtttga agtttggggg caggggcgt gtgtgggttg ataacttaaa    2340
aaaataatgg gctctgattg ggcagctact catttgagtt ccttccattt gacctaattt   2400
aactggtgaa atttaaattg agtttatgag ctcatcttca aaacttttgc tagaagattt   2460
tcagctgttc caaatgggac ttactaacag tatgtatata aaaagatcac atcagtggat   2520
```

gagagacatt tgatcccttg tttgcttaat aaattgtaaa atgatggctt ggaaaagcag 2580

gcttatagtc taaccatggt gctattatta ggcttgcttg ttaaacacag gtctaagcct 2640

agtatatcaa taaaacaaat acttatttca tttgaaaaaa aaaaaaaaaa aaaa 2694

<210> 7
<211> 3863
<212> DNA
<213> Rattus norvegicus

<400> 7
ctgcgccgga ggagccggcg cttgccggtg cctgggggtc ggggcgcggg gaaacccggg 60

ccgctggggt gacccaacag gtagagccgg ggccacggcc ggccacgcgc cccccgcgtg 120

catcatgcag ctctttgtca cctctctcgc ccccaggcca aaatcctgag catgatggaa 180

gacaataagc agctcgcgct ccgcatcgat ggggcggtcc agtcggccag ccaggaggtg 240

accaacctgc gagccgaact cacggccacc aaccggagac tggcggaatt gagcggcgga 300

ggcggcggcc ccggctcggg cccgggagct gcgaccagcg cctcggcagc agcggtgacg 360

gtggctgact cggcggtggc gaccatggag aaccatcagc acggagctca agtgctcctg 420

cgggaggaag tggtccagct tcaggaggaa gtccaccttc tccgacagat gaaggaaatg 480

ctggcaaagg acctagagga gtcacagggc ggcaaatgct ccgaggtgct gtcggccact 540

gaactccggg tgcagctggt acagaaggag caggagctgg ccagagccag ggaagccttg 600

caagccatga aagctgaccg gaagcgctta aagggcgaga agaccgacct ggtgagccag 660

atgcagcagt tgtacgccac gctggagagc cgcgaggagc agttgcgaga cttcatccgc 720

aactatgaac aacaccgcaa ggagagtgag gatgctgtca aagcgctggc caaggagaaa 780

gacctgctgg agcgagagaa gtgggagctc cgacggcagg ccaaggaggc cacggaccat 840

gctgcggccc tgcgctccca gctggacctc aaggacaacc gcatgaagga gctggaggcc 900

gagctagcca tggccaagca gtccttagcc acactgacca aggatgttcc caagaggcat 960

tcactcgcca tgcccggcga gacggtgctc aatggcaacc aggaatgggt ggtgcaggct 1020

gacctcccac tgactgccgc catccggcag agccaacaga ctctctacca ctcccaccct 1080

ccccaccctg cagaccgaca agcggtcagg gtgagcccct gccactcaag acagccttcc 1140

gtcatctctg atgcttccgc tgctgaaggt gaccggtcat ctacaccgag cgacatcaac 1200

tccccaagac accggacaca ctccctctgc aacggcgaca gtcccggccc agttcagaag 1260

agcctacaca accctattgt acagtcacta gaggatcttg aagaccaaaa acggaaaaag 1320

aagaaagaga agatgggatt tggctccatc tctcgagtct tcgccagagg gaaacaacgg 1380

aagtccctcg acccgggcct ctttgatggt accgcccctg attattacat agaggaagac 1440

gcggactggt gataacccac cgccctccgc ccgttgtcca cgggcgtgtc tgtgtgtgga 1500

tgagccttgg agagtgtgcg agagtgtgcg tgcgcgtggg gatgcggagc ctgtgtgtgg 1560

ggtgtgtgtg cacggaggca ctcgccctcg tgggtgcgtg tagccaagcg cctggaacag 1620

acagaaacac aaatgtgacc cccaaccccc ctccgcgtcg tcacctctgt aatcgctgta 1680

cataccacaa accgtgtgtg actctgtcaa ccccgttgtc tctgagcgat acacttgtgt 1740
ttgtttctct cgtttgtttt gtttttcgat gttttttggg gggggggtt gtttgtttgt 1800
ttctgtttaa atccgtcgtg tttttgtttt taatttgccc ctttctcctc cctcctccct 1860
ttttatgaaa cttgaacact tgaaggactg ctgtgtattt gtaaataaca aaagtagtgt 1920
gcactttgtg cttgtaaagt ccctggtcca gccgctactc ctacagccca cgtcgccaag 1980
gacgcgcggt ctgtgtttca tgtcccctcc ttcttcggtg tgaacgtgtg ggaccagacc 2040
ttgttctcgg ggtgtgcccg agtcacttta accacaaaaa cacccccgcc atcgtcgtca 2100
gggtaaggtc actctgcaga gacccgtgag cccatccaag actttttggc tgggccacag 2160
tgtttataag gggtcatat gggctctttt taatggcctg cctgttttta aattgtgttg 2220
ctgtttcttt ctttatggag caaaaagaga gaaagaaaac ttgcatttaa tttatttgca 2280
caaatgcaga aagcgtattc tatctaaatt attacgtaaa tattggaatg tatatttttc 2340
cacggggtgg gcgggaggcg ggcgtttctg ctgacttgtc tattctgtgt tcatgccgct 2400
gcagacactg tgcaaggtag aattcaggtg gctagagctg gggacctcag acgaccagct 2460
tgcttcttct ttttttctct tttctgtccc cccccccccg gctgctgtcc ctgcatgaga 2520
ggactcccac cttctgctct ggcctttctc tctagatatt ctctctcttt atatatatat 2580
ctatatattt atttatctga catacagatc atgacttcgg tgagcagaga acactaacag 2640
tcaaattccc cttctcttgg acctgtcttt ggaaccatgt tgattgagac aagggaaggt 2700
tagcctgggg cacatctgtg gggtctaacc agacatctgg gcttgagtca gtgggtaaga 2760
ggctcctggt ggcacataca caatgcctct ctatccttag cttattccaa acatagggca 2820
agccagtgtc aacaaagatg atgcccacct tctgggaggg gctgttgggg gcagtatcta 2880
gcaggcaagc cctttgccct ctttcgtcca gttgacttct ttgcccacac atggactttc 2940
tcataatccc tggatccctg tcctctgatt atatcagcga ctcaagcaga ctaagcccaa 3000
aagggctggc aggtgcctgt gcctgtttgt gggggtagca ctgtccctat ggggcctcac 3060
ctggggcatg gagaacccac aagtttccag ccagtaccac cccaccccg aactcagttt 3120
cctctttctc tcctccccat cccctttttaa ctgatcctaa accactaata actcagccca 3180
caccccttat ggccactgct gtgctccctc taacagaaac attctcaact cttgaaagat 3240
ttttcagtac cggatggaaa atctggaatg tataggatct tgtgtccaaa gtctctaaaa 3300
aaacaagcca aaaagcccct caccaggtca cctcttccct cccgacctgg cccagaggcc 3360
ggagagagag caggcaggga caccagatgg gccttcctgg gtacacaaca accctttcgg 3420
ctcagctgat taacccagcc tggtggtgag ggagagaggc cagagctggc atcagaggga 3480
ggtcttagaa aacactaccc ccaccccatg aattttcggg ttttcttttc ctttcttttt 3540
tttatggttc ccccacacac acccttcctt ttaacttaca agaaaataag gttgcaacca 3600
ctcccggtta atggcttagt cactcctctt cgtttcagtt ttttggaaat taggagatta 3660
tcctgtgagt tactaaggat ggtttatttt cgagaaccac atcagatagg gaatgagtta 3720

tgggtaactc tagatgaaaa tgacccttcc cccccccccg ggagaggttt ctcgaaggca 3780

tggatgcaaa atataaaata ttaaaaaaaa gtctaaataa agcttatttt aaaataaaaa 3840

aaaaaaaaaa aaaaaaaaaa agg 3863

<210> 8
<211> 903
<212> DNA
<213> Rattus norvegicus

<400> 8
gatcatgggt gctacggcgc tgcccccgat ctggcagatg taccttaagg accaccggat 60

ctacaccttc aagaactggc ccttcctgga ggattgctcc tgcaccccag agcggatggc 120

ggaggctggc ttcatccact gccctaccga gaatgagcct gatttggccc agtgtttttt 180

ctgctttaag gaactggaag gctgggaacc ggatgacaac cctatagagg agcataggaa 240

gcactcccct ggctgcgcct tccttacagt caagaagcag gtggaagaac tgaccgtcag 300

cgagtttttg aaactggaca aacaaagagc caagaacaaa attgcaaagg agaccaacaa 360

caaacagaaa gagttcgagg agacccggag gaccgtccgg cagtccattg agcagctggc 420

tgccttacgc tgagcctttg ctgggcaacc tggacctgag tgacatgcca cggctaagcc 480

acttgtccca gctttccagg cctgcctggc cgccttggtg tcttacagga gaccgtgaca 540

tttcgaaact ggacgtcaga tgatttgggg ttttgcttta aaggggctca gcctgcgtgg 600

ccacctctct ttggttttgt ggctttgctc tattgtgacc tggacttaag cactgaggaa 660

gggagtggat gagggacagg attctctgac aggatctatg ggtggggagg ggggtgaagg 720

gagggttgtg caaggccttt ctggtcttga tgtttccatg cctggcagct gtcgcagccc 780

atgtgtaggt gttggtttat atatgtttgt gctgataatt cttcttctgt ccttctgatg 840

agtcctccta ccatggggta atggaataaa ataacttaac aaaaaaaaaa aaaaaaaaaa 900

aaa 903

<210> 9
<211> 8229
<212> DNA
<213> Mus musculus

<400> 9
gcggcaggat acgcgcttgg gcgtcgggac gcggctgcgc tcagctctct cctctcggaa 60

gctgcagcca tgatggaagt ttgagagttg agccgctgtg aggccaggcc cggcgcaggc 120

gagggagatg agagacggcg gcggccacgg cccagagccc ctctcagcgc ctgtgagcag 180

ccgcgggggc agcgccctcg gggagccggc cgggcggcgg cggcggcagc ggcggcgggc 240

ctcgcctcct cgtcgtctgt tctaaccggg cagcttctga gcagcttcgg agagagacgg 300

tggaagaagc cgtgggctcg agcgggagcc ggcgcaggct cggcggctgc acctcccgct 360

cctggagcgg gggggagaag cggcggcggc ggccgcggct ccggggaggg ggtcggagtc 420

gcctgtcacc attgccaggg ctgggaacgc cggagagttg ctctctcccc ttctcctgcc 480

```
tccaacacgg cggcggcggc ggcggcacgt ccagggaccc gggccggtgt taagcctccc    540
gtccgccgcc gccgcacccc ccctggcccg ggctccggag gccgccggag gaggcagccg    600
ctgcgaggat tatccgtctt ctccccattc cgctgcctcg gctgccaggc ctctggctgc    660
tgaggagaag caggcccagt ctctgcaacc atccagcagc cgccgcagca gccattaccc    720
ggctgcggtc cagggccaag cggcagcaga gcgaggggca tcagcgaccg ccaagtccag    780
agccatttcc atcctgcaga agaagcctcg ccaccagcag cttctgccat ctctctcctc    840
ctttttcttc agccacaggc tcccagacat gacagccatc atcaaagaga tcgttagcag    900
aaacaaaagg agatatcaag aggatggatt cgacttagac ttgacctata tttatccaaa    960
tattattgct atgggatttc ctgcagaaag acttgaaggt gtatacagga acaatattga   1020
tgatgtagta aggtttttgg attcaaagca taaaaaccat tacaagatat acaatctatg   1080
tgctgagaga cattatgaca ccgccaaatt taactgcaga gttgcacagt atccttttga   1140
agaccataac ccaccacagc tagaacttat caaacccttc tgtgaagatc ttgaccaatg   1200
gctaagtgaa gatgacaatc atgttgcagc aattcactgt aaagctggaa agggacggac   1260
tggtgtaatg atttgtgcat atttattgca tcggggcaaa tttttaaagg cacaagaggc   1320
cctagatttt tatggggaag taaggaccag agacaaaaag ggagtcacaa ttcccagtca   1380
gaggcgctat gtatattatt atagctacct gctaaaaaat cacctggatt acagacccgt   1440
ggcactgctg tttcacaaga tgatgtttga aactattcca atgttcagtg gcggaacttg   1500
caatcctcag tttgtggtct gccagctaaa ggtgaagata tattcctcca attcaggacc   1560
cacgcggcgg gaggacaagt tcatgtactt tgagttccct cagccattgc ctgtgtgtgg   1620
tgatatcaaa gtagagttct tccacaaaca gaacaagatg ctcaaaaagg acaaaatgtt   1680
tcacttttgg gtaaatacgt tcttcatacc aggaccagag gaaacctcag aaaaagtgga   1740
aaatggaagt ctttgtgatc aggaaatcga tagcatttgc agtatagagc gtgcagataa   1800
tgacaaggag tatcttgtac tcaccctaac aaaaaacgat cttgacaaag caaacaaaga   1860
caaggccaac cgatacttct ctccaaattt taaggtgaaa ctatacttta caaaaacagt   1920
agaggagcca tcaaatccag aggctagcag ttcaacttct gtgactccag atgttagtga   1980
caatgaacct gatcattata gatattctga caccactgac tctgatccag agaatgaacc   2040
ttttgatgaa gatcagcatt cacaaattac aaaagtctga ttttttttt cttatcaaga    2100
gggataaaat accatgaaaa aaaaaaaact tgaataaact gaaatggacc tttttttttt   2160
tttttttttt ttaaatggca ataggacatt gtgtcagatt gcagttatag gaacaattct   2220
cttctcctga ccaatcttgt tttaccctat acatccacag ggttttgaca cttgttgtcc   2280
agttaaaaaa aggttgtgta gctgtgtcat gtatatacct ttttgtgtca aaggacattt   2340
taaaattcaa ttaggataaa taaaagatgg cactttccca ttttattcca gttttataaa   2400
aagtggagac aggctgatgt gtatacgcag gagtttttcc tttattttct gtcaccagct   2460
gaagtggctg aagagctctg attcccgggt tcacgtccta cccctttgca cttgtggcaa   2520
```

cagataagtt tgcagttggc taaggaagtt tctgcagggt tttgttagat tctaatgcat 2580
gcacttgggt tgggaatgga gggaatgctc agaaaggaat gtttctacct gggctctgga 2640
ccatacacca tctccagctc cttagatgca cctttcttta gcatgctcca cttactaatc 2700
tggacatccg agagattggc tgctgtcctg ctgtttgttt gtgcatttta aagagcatat 2760
tggtgctaga caaggcagct agagtgagta tatttgtagt ggggtacagg aatgaaccat 2820
ctacagcatc ttaagaatcc acaaaggaag ggatataaaa aaagtggtca tagatagata 2880
aaagacacag cagcaatgac ttaaccatac aaatgtggag gctttcaaca aaggatgggc 2940
tggaaacaga aaatttgaca atgatttatt cagtatgctt tctcagttgt aatgactgct 3000
ccatctccta tgtaatcaag gccagtgcta agagtcagat gctattagtc cctacatcag 3060
tcaacacctt acctttattt ttattaattt tcaatcatat acctactgtg gatgcttcat 3120
gtgctggctg ccagtttgtt tttctcctta aatattttat aattcttcac aggaaatttc 3180
aacttgagat tcaacagtaa gcaggttttg tttttttttt ttcctagaga ttgatgatgc 3240
gcgtcctcag tccagtggct gtcagacgtt cagccccttt gaccttacac attctattac 3300
aatgagtttt gcagttttgc acattttttt taaatgtcat taactgttag ggaattttac 3360
ttgaatactg aatacatata atgtgtatat taaaaaagtc attgtttgtg ttaaaaaaga 3420
aattagagtt gcagtaaatt tacagcactg cacgaataat aaggcattga agtttttcag 3480
tagaaattgt cctacagatg ctttatcgac ttgctattgg aagaatagat cttcttaaat 3540
gtgcagtgtt gagtcacttc gttatagtgg tagagttggg attagggctt caattttact 3600
tcttaaatat cattctatgt ttgatatgcc cagactgcat acaatttaaa gcaagagtac 3660
aactactatc gtaatggtaa tgtgaagatg ctattacaaa ggatctcctc ccaacccctc 3720
gggaatttgg tgtctttcaa attatatctt gaccttgaca tttgaatatc cagccattat 3780
tagatttctt aatggtgtga agtcccattt tcaataactt attggtgctg aaattgttca 3840
ctagctgtgg tctgacctag ttaatttaca agtacagatt gcataggacc cactagagaa 3900
gcatttatag tttgatggta agtagattag gcagaacgcc atctaaaata ttcttagaaa 3960
ataatgttga tgtattttcc atacctcatc agtttcactc aaccaataaa gtttttaaaa 4020
ttgtaacaaa gctcttagga tttacacatt tatatttaaa cattgataca tgaatattga 4080
ctgactgttg ataaagtcag agacaacttt tcctgagatc tcaccatgga aatctgtaca 4140
ccccctgtc tttcctaaaa gctgaaagtg gctgactaaa atgcaaagca gctgttgatg 4200
ttttgaagat agtgataaac actgttcttt gttagttttg ggcacagcat gctaaactat 4260
aacttgtatt gttccaatat gtaacacaga gggccaggtc atgaataatg acattacaat 4320
gggctgttgc actgttaata tttttccttt ggaatgtgaa ggtctgaatg agggttttga 4380
ttttgaatgt ttcagtgttt ttgagaagcc ttgcttacat tttatggtgt agtcattgga 4440
aatggaaaaa tggcattata tatatattat atatatataa atatatatat tatacatact 4500
ctccttactt tatttcagtt accatcccca tagaatttga caagaattgc tatgactgaa 4560

agggttttga gtcctaattc aaactttctt tatgacagta ttcacgatta gcctgaagtg 4620
cattctgtag gtgatctctc ccgtgtttct ggaatgcttt cttagactct tggatgtgca 4680
gcagcttatg tgtctgaaat gacttgaagg catcaccttt aagaaggctt acagttgggc 4740
cccgtacatc ccaagtcctc tgtaattcct cttggacatt tttgccataa ttgtaaaagg 4800
gtagttgaat taaatagcgt caccattctt tgctgtggca caggttataa acttaagtgg 4860
agtttaccgg cagcatcaaa tgtttcagct ttaaaaataa aagtaggtta caagttacat 4920
gtttagtttt agaaaatttg tgcaatatgt tcataacgat ggctgtggtt gccacaaagt 4980
gcctcgttta cctttaaata ctgttaatgt gtcgtgcatg cagacggaag ggtggatct 5040
gtgcactaaa cggggggctt ttactctagt attcggcaga gttgccttct acctgccagc 5100
tcaaaagttc gatctgtttt catatagaat atatatacta aaaccatcca gtctgtaaaa 5160
cagccttacc ccgattcagc ctcttcagat actcttgtgc tgtgcagcag tggctctgtg 5220
tgtaaatgct atgcactgag gatacacaaa tatgacgtgt acaggataat gcctcatacc 5280
aatcagatgt ccatttgtta ctgtgtttgt taacaaccct ttatctctta gtgttataaa 5340
ctccacttaa aactgattaa agtctcattc ttgtcattgt gtgggtgttt tattaaatga 5400
gagtatttat aattcaaatt gcttaaatcc attaaaatgt tcagtaatgg gcagccacat 5460
atgattacaa agttcctgtg cattttttcta tttttcccc tccttgctat ccttccaagc 5520
aaagcatctt tctgtcatct tggtagacac atacctgtct actcatggtt aagaagagca 5580
ctttaagcct tagtcatcac ttaataagtt attccaggca cagtaaaaag ttcaaggttc 5640
ttggaaaacg gtgcttattt ctcttcttat aagccagatg tctgaagata gccctaaccc 5700
caagaacggg cttgatgtct caggtctgtt ctgtggcttt ctgttttttt taacactgca 5760
gttggccatc agcacatggg aggtttcatc gggacttgtc cagagtagta ggctcaaata 5820
tactatctcc tttctaatat tcttaaaggc taaggagtcc tttcaatata acagtaagat 5880
aacttgtgat gttttagaag taagcagacc attaatgtca atgtggagtc ttaatgttac 5940
atgaagttga tagtttctct gtgacccatt taaaaataca aaccgagtag catgcaatta 6000
tgtaaagaaa tatgaagatt atatgtagtc acacattttc tttagaattc ttagtttggt 6060
gaaaacttga atataaaggt attttgattt atatgacatt ttgatgatat ttgaaaaaaa 6120
ggaatttcct gacattttgc ttttagatca tgtcccccat tgtgctgtaa tttaagccaa 6180
cttggttcag tgaatgccat caccatttcc attgagaatt taaaactcac cagtgtttaa 6240
catgcaggct tctgagggct cccggagaat cagaccttaa gcccagttga tttacttcta 6300
acgtgaaact tcgagttcct gtatactttg ctagataatt tgtggtacat ctaaagctta 6360
gtcttaagtg gcttgtgtgt ggattttatt caacattctt gttgctaggg tagagagaaa 6420
tgttgctgag tagaaacaag agtacccagt tcaatgtggt acagagagca gtccctaaaa 6480
tctgtacaca gtgtaatgga ccactttagg agtcaagagg ctgatttttc ctatgaaatt 6540
acattgcaac aggaagcctt ctagtatagt tccttttact gttagaatat gtttttatgc 6600

atacgctata gctgctttcc catcttccaa caacaggtat caggatgtaa gcaagcttta 6660

aacagtgtga agatggcagg atagtgtcat cggtaacagt cctctgactc taaatgtagt 6720

tgctctgtaa cactttgtga atataacatc acaattctca tgtccttggg gggggggggc 6780

atacccagta ttagtatgtt ttagtgacta agcaatcatt tttctgttta ctcatgtaca 6840

ttttctcttt aaaactaaaa cctgtactgt gtatgtctcc aaagcctttt agcttagttt 6900

ttaggaaatg aacactgaat ggatcacttt ttagtgtagc aggtatggga tatgtgcatt 6960

atagagagac cttgtcagct ctctgggcct atttgaatgt ttattgttgg tgtgaggatg 7020

gtaggggaat cagtaaatac aagttacgtt ggtttagcag agcaagctca gtgtgggtat 7080

ttctctttga agcgtggtgc gtgacgcact gtgagtagag aatttggtca ccctttgagt 7140

cctcttgcat tttgcaaact tgctcagcaa atgcgtacct accttgcccc ctaggtaaaa 7200

gcaggaacta ctactgattt atctgtcact cagctgtctt tatatgtgtg cttctgtgac 7260

ttgtatcaca caagaatctt aaagatttca caaattgtta ccttttagct ctgaatgttg 7320

agtattctgg tgggctaaca acaagacaaa ctcttgacag tcatttgaga attttcatga 7380

aacatttagc tgaaaacatt ttataattta tgaaaaaaat gtgttacctt aaacttttac 7440

atatgtggga gacattaact gccatatttg agcatactga attttaaatt taaaataaag 7500

ctgcatattt ttaaatgaaa tgtttaacaa ggattcatat tttttgtttt ttaagattaa 7560

aaataattta tgtcttctca tgtggaacct catctgtcac aatggttaga ttatacagaa 7620

tggagcaagg cttgtagtgg tttagcttac agtaaaattc ttaatgttta gatgtgttta 7680

cttactggct gttatgtata cttttgagat tttccacctg ttctgtgtag ttttctaaat 7740

gatactccta cttaaaaaca gcattttagt atctattttc tgtctccatt aaatggtcct 7800

cattttctat tgagtttgga agtgtgcaca ttgtgtgtgt gtgtgtgtgt gtgtgtgtgc 7860

acacgtgtgc gcgcccgtgc gtgtgtctat ttgtggagtt tgtatgggag aattagtttt 7920

gaaagtgcta gaatagagat gaaatttggt tcaagtaaaa ttttcccact gggattttac 7980

agtttattgt aataaaatgt taattttgga tgaccttgaa tattaatgaa tttgttagcc 8040

tcttgatgtg tgcattaatg agatatatca aagttgtata ttaaaccaaa gttggagttg 8100

tggaagtgtt tttatgaagt tccgtttggc taccaatgga cataagacta gaaatacctt 8160

cctgtggaga atatttttcc tttaaacaat taaaaaggtt cattattttt gaaaaaaaaa 8220

aaaaaaaaa 8229

<210> 10
<211> 1329
<212> DNA
<213> Aspergillus fumigatus

<400> 10
atgagtcaac cacagagagg tggaggtatc tctgccatct tcgtccatgt aggggcggga 60

tatcacggcc atggaaacga gagagcccac cttcaggtat gcgaaaacgc ttgtaaagcc 120

```
gcaatgggca ttttaaaaag cggtggatca gctctggatg ctgttgagat ggccatcatt    180
gtaatggagg atgacgagat tacaaatgct ggctatggaa gcaacctgac gattgaagga    240
gctgtcgaat gtgatgctac tattgtcgac caccatggca ggagcggcgc agctggagcc    300
gtctcgccgc gaatgctact tgaaacctct gcaaagcctt taacttgcca cagagtgcct    360
ccaagcttcc tcgttggtga gggtgcaacg gactttgctt atgagcaagg attggtcata    420
ctgcctcctg acgggctcat atcgcgcttt tcgaaagaga gatggcaccg ctggctgcag    480
gatctcgagg ctgctgaact agtcgaaaga aaacgggatc cttcacgttt ccgcatggaa    540
gaagatagag catccttcct tcgtcggcca atgctgaatc gtaatccagc ccgcctcatt    600
gacaatacgc gtttacgccc acatctaccc agctctccct tcaccggagt cgacttgctg    660
aacccccttc ctcgtctcca ggctcttgga agcagacaaa ccatagctcc tgcaccacca    720
gctatgcctt cgaaccaagg acacggcatg ggtcctgcaa ttggacaacc tatggctgtg    780
gcaggtggac aaagtatggc ttctgcacct gagcaaggta tggctgcctc ggccaacacc    840
catgcagatg gtgcgacttt ccgcgcgtgc cctacaaaag caccttgtgc acctgacact    900
tcgacgactg ctcacccggg cgctgcagac gaggatgaag atatgatcag cgatacagtc    960
ggggccatag ctgtagactg ttatggcaat attgcggcag gttcttcgtc tggcgggatt   1020
ggcgcgaagc atcgcggtcg aattgggcct gctgcgctgg tgggcatcgg cacctatgtg   1080
attcctgtgg atcccagcga tcccgaacaa gtatcagtcg cttcagttac atcgggcaca   1140
ggcgagcaca ttgctacaac tatggctgcg cagacgtcag ctgcaagact ctattactgt   1200
caaaagaagt gtaaggacgg tactttcgaa agtgtatccg aagacgaagc tatgaatgca   1260
atcatagcca ctgagttcat gggtgagtta ttgtattcca tattccttac gaccattgat   1320
agagaataa                                                           1329
```

## Description

Field of the Invention

This invention relates to nucleic acid technology, specifically to modified oligonucleotide and its preparation, its application in RNA interference, pharmaceutical compositions, and disease treatment.

Background of the Invention

RNA interference (RNAi) is a gene silencing phenomenon mediated by double-stranded RNA (dsRNA) molecules, shutting down homologous gene expression at mRNA level. RNAi is a post-transcriptional gene regulation mechanism, also named post-transcriptional gene silencing (PTGS), target gene knockdown, or gene silencing.

RNAi phenomenon was first reported in plants by two different research groups in 1990. Later on, this phenomenon was further observed in almost all eukaryotes, including C. elegans, Drosophila, zebrafish and mice.

In 1999, RNA fragments of 21 to 25 nucleotides in length were identified in plant RNAi by Hamilton and Baulcombe. These small RNA fragments were demonstrated to be the mediator necessary for RNAi, and thus named as small interfering RNA (siRNA).

Double-stranded siRNA conjugates with endogenous enzymes and proteins, and then forms RNA-induced silencing complex (RISC). In the process of RNAi, while the sense RNA strand of double-stranded siRNA is excluded from the complex, the antisense RNA strand functions to guide RISC to target mRNA at homologous locus, resulting in the degradation of target mRNA and gene silencing mediated by RNase III component within RISC complex.

However, due to the vulnerability of siRNA to serum ribonuclease that resulting in serum instability, synthetic siRNAs are often chemically modified so as to increse their serum stability and gene silencing activity.

Even though, in consideration of the poor understanding of the degradation process and mechanism of RNAi, random modification strategy is performed to stabilize siRNA in body fluids. Although this strategy can improve serum stability of the modified siRNAs to some extent, it usually ends up with excessive chemical modifications and results in increased cytotoxicity and compromised biological activity in many cases, due to the lack of theoretical guidance. This therefore restricts the *in vivo* applications of the modified siRNAs. In addition, the extensive siRNA modification strategy also limits the use of many chemical modifications that have great stabilizing effect, however with relatively high cytotoxicity.

Therefore, there is an urgent need to develop a specific modification strategy for synthetic siRNAs, to achieve optimum serum stability with minimal chemical modifications.

Summary of the Invention

The present invention is to overcome technical problem caused by the extensive siRNA modification commonly performed in the art, by providing a kind of modified oligonucleotide. The oligonucleotides provided in the present invention include, without limitation, RNA and DNA oligonucleotides, also include a variety of chemically modified nucleic acid derivatives including single-stranded and double-stranded nucleic acid molecules. That is, the modified oligonucleotides disclosed in the invention include but do not limit to single-stranded RNA molecules (ssRNA), single-stranded DNA molecule (ssDNA), double-stranded RNA molecules (dsRNA), double-stranded DNA molecule (dsDNA), as well as a variety of nucleic acid derivatives modified from the above molecules. Preferably, the oligonucleotides are double-stranded RNA molecules (dsRNA); more preferably, the oligonucleotides are small interfering nucleic acids (siRNA).

To address these problems, the present invention discloses a modified oligonucleotide comprising a first nucleic acid fragment and a second nucleic acid fragment. Said first nucleic acid fragment and second nucleic acid fragment can form double-stranded region, wherein the modified oligonucleotide has at least one of the following modifications, the modification(s) improve the stability of the modified oligonucleotide in mammalian body fluids:

(a) The first nucleic acid fragment contains at least one contiguous UA sequence, the second nucleic acid fragment contains at least one contiguous UA sequence complementary to the UA sequence in the first nucleic acid fragment. The UA sequence(s) of the first nucleic acid fragment and the complementary UA sequence(s) of the second nucleic acid fragment pair to form UA/UA site(s); wherein at least one of the nucleotides in at least one of the UA/UA site (s) is a modified nucleotide;

(b) The first nucleic acid fragment contains at least one contiguous CA or UG sequence, the second nucleic acid fragment contains at least one contiguous CA or UG sequence complementary to the CA or UG sequence in the first nucleic acid fragment, the CA or UG sequence(s) of the first nucleic acid fragment and the CA or UG sequence (s) of the second nucleic acid fragment pair to form CA/UG and/or UG/CA site(s); wherein at least one of the

nucleotides in at least one of the CA/UG and/or UG/CA site(s) is a modified nucleotide;

(c) The first nucleic acid fragment contains at least one contiguous UA sequence and at least one contiguous CA or UG sequence, the second nucleic acid fragment contains at least one contiguous UA sequence complementary to the UA sequence of the first nucleic acid fragment and one contiguous CA or UG sequence complementary to the CA or UG sequence of the first nucleic acid fragment, the UA sequence(s) of the first nucleic acid fragment and the UA sequence(s) of the second nucleic acid fragment pair to form UA/UA site(s), the CA or UG sequence(s) of the first nucleic acid fragment and the UG or CA sequence(s) of the second nucleic acid fragment pair to form CA/UG or UG/CA site(s); wherein at least one of the nucleotides in at least one of the UA/UA site(s) is a modified nucleotide, and at least one of the nucleotides in at least one of the CA/UG or UG/CA site(s) is a modified nucleotide.

According to the present invention, in the cases that the first nucleic acid fragment contains at least one contiguous UA sequence, the second nucleic acid fragment contains at least one contiguous UA sequence complementary to the UA sequence in the first nucleic acid fragment, the UA sequence(s) of the first nucleic acid fragment and the complementary UA sequence(s) of the second nucleic acid fragment pair to form UA/UA site(s), modification can be made for at least one of the nucleotides in one UA/UA site, modification can also be made for at least one of the nucleotides in multiple UA/UA sites, modifications can also be made for multiple nucleotides in multiple UA/US sites. Preferably, only one uracil nucleotide is modified in each UA/UA site.

In the cases that first nucleic acid fragment contains at least one contiguous CA or UG sequence, the second nucleic acid fragment contains at least one contiguous CA or UG sequence complementary to the CA or UG sequence in the first nucleic acid fragment, the CA or UG sequence(s) of the first nucleic acid fragment and the CA or UG sequence (s) of the second nucleic acid fragment pair to form CA/UG and/or UG/CA site(s), modification can be made for at least one of the nucleotides in one CA/UG or UG/CA site, modification can also be made for at least one of the nucleotides in multiple CA/UG or UG/CA site(s), furthermore, modifications can also be made for multiple nucleotides in multiple CA/UG or UG/CA sites. Preferably, only one cytosine nucleotide is modified in each CA/UG or UG/CA site.

In the cases that the first nucleic acid fragment contains at least one contiguous UA sequence and at least one contiguous CA or UG sequence, the second nucleic acid fragment contains at least one contiguous UA sequence complementary to the UA sequence of the first nucleic acid fragment and one contiguous CA or UG sequence complementary to the CA or UG sequence of the first nucleic acid fragment, the UA sequence(s) of the first nucleic acid fragment and the UA sequence(s) of the second nucleic acid fragment pair to form UA/UA site(s), the CA or UG sequence(s) of the first nucleic acid fragment and the UG or CA sequence(s) of the second nucleic acid fragment pair to form CA/UG or UG/CA site(s), modification can be made for at least one of the nucleotides in UA/UA and CA/UG or UG/CA site; modification can also be made for at least one of the nucleotides in at least one of the UA/UA sites, at the same time, modification is also made for at least one of the nucleotides in at least one of the CA/UG or UG/CA sites.

In the present invention, the term "first nucleic acid fragment" refers to a nucleic acid fragment partly or completely homologous to the coding region of a target gene, the term "second nucleic acid fragment" refers to a nucleic acid fragment complementary to the coding region of a target gene. The term "complementary" refers to that two nucleotides can pair with each other in hybridization condition. For example, adenine (A) can pair with thymine (T) or uracil (U), cytosine (C) can pair with guanine (G).

In the present invention, said oligonucleotides can be small interfering RNA (siRNA) against a variety of target genes. For example, target genes can be an endogenous gene to be studied, a gene whose expression to be inhibited; for example, a gene associated with a disease or a disorder, such as oncogenes, viral genes, cell surface receptors, nuclear receptors, or genes implicated in cellular signaling pathway.

Based on the sequence of a target gene, specific siRNAs can be designed by person having ordinary skill in the art to which this invention belongs. For example, input target gene sequences or their NCBI Genbank sequence number into various siRNA design softwares, such as Insert Design Tool for the shRNA Vectors (Ambion), shRNA Explorer (Gene Link), siDirect (Yuki Naito et al. University of Tokyo), SiRNA at Whitehead (Whitehead Institute for Biomedical Research), BLOCK-iT RNAi Designer (invitrogen), RNAi Design (IDT), RNAi Explorer (Gene Link), siRNA Target Finder (Ambion), or siSearch (Stockholm Bioinformatics Center) etc., the software can design gene-specific siRNAs according to the requirements and siRNA design principles.

In addition, several siRNA design software can also perform genome-wide or transcriptome-wide homology search, so as to obtain target gene-specific or sequence-specific siRNAs. Said siRNA design software and principles are commonly understood by person having ordinary skill in the art to which this invention belongs. All these information are therefore included in the present invention as references.

In a preferred embodiment, the invention relates to a modified oligonucleotide, wherein all the nucleotides are unmodified nucleotides except the nucleotides in said CA/UG and/or UA/UA and/or UG/CA site(s). In these cases, incorporation of minimal chemical modifications into the modified oligonucleotide not only increase its stability, maintain its biological activity, further reduces potential cytocoxicity caused by extensive chemical modifications.

In the present invention, the modified oligonucleotide can be single-stranded or double-stranded oligonucleotide. In the cases of single-stranded oligonucleotide, said first nucleic acid fragment and second nucleic acid fragment can form double-stranded region by base-pairing of their complementary sequences. In the cases of double-stranded oligonucleotide, said modified oligonucleotide can contain a sense strand and antisense strand. The sense strand can be a contiguous fragment, or several discontiguous fragments; the antisense strand is a contiguous fragment.

In one aspect of the present invention, the invention relates to a modified oligonucleotide comprising a sense strand and an antisense chain. Said sense strand and antisense strand are contiguous nucleic acid fragments, said first nucleic acid fragment is in the sense strand, and said second nucleic acid fragment is in the antisense strand.

In another aspect of the present invention, the invention relates to a modified oligonucleotide comprising a sense strand and an antisense strand. Said sense strand comprises two or more discontiguous sense-strand fragments, said antisense strand is a contiguous nucleic acid fragment. Said first nucleic acid fragment is in the one or more sense-strand fragments, and the second nucleic acid fragment is in the antisense strand.

In the present invention, the term "sense strand" refers to a nucleic acid fragment partly or completely homologous to the coding region of a target gene, in the cases that the modified oligonucleotide is a double-stranded molecule. The term "second nucleic acid fragment" refers to a nucleic acid fragment complementary to the coding region of a target gene, in the cases that the modified oligonucleotide is a double-stranded molecule. In addition, in the cases that said modified oligonucleotide is a double-stranded molecule, said sense strand and antisense strand are contiguous nucleic acid fragments, the term "sense strand" and the term "first nucleic acid fragment" are used interchangeably, the term "antisense strand" and the "second nucleic acid fragment" are used interchangeably.

In the present invention, the term "sense-strand fragment" refers to the component fragments of the sense strand, in the cases that said sense strand comprises discontiguous fragments; the total length of all sense-strand fragments is equal to length of the sense strand. In addition, in the cases that said sense strand contains UA/UA site (s), the term "sense-strand fragment" and "first nucleic acid fragment" are used interchangeably.

In the present invention, said modified oligonucleotide can comprise only ribonucleotides, can also be a hybrid molecule comprising ribonucleotides and at least one deoxyribonucleotide.

In present invention, the term "potential toxicity" refers to the cellular toxicity effects caused by involved chemical modification(s) made on the modified nucleotide(s). In another word, it refers to the inhibition effects on cell growth, and thus named as "growth inhibition ratio" throughout this invention. The higher the growth inhibition ratio is, the greater the potential cytotoxicity is.

In the invention, said modifications are commonly understood by person having ordinary skill in the art to which this invention belongs. For example, the modification(s) are one or a combination of the following modifications:

(a) modifying the phosphodiester bond(s) connecting nucleotides in the oligonucleotide;

(b) modifying the sugar(s) of the nucleotide in the oligonucleotide;

(c) modifying the base(s) of the nucleotide in the oligonucleotide.

Said modification of phosphodiester bond is made by modifying the oxygen of the phosphodiester bond, including phosphorothioate modification and boranophosphate modification. As shown in the figures, these modifications replace the oxygen of the phosphodiester bond by sulfur or boron, respectively. Both modifications can stabilize the structure of siRNA, maintain high specificity and affinity of base pairing. However, boranophosphate siRNAs have stronger hydrophobicity and readily to form protein conjugate in the plasma, and less toxic to human body than phosphorothioate siRNAs.

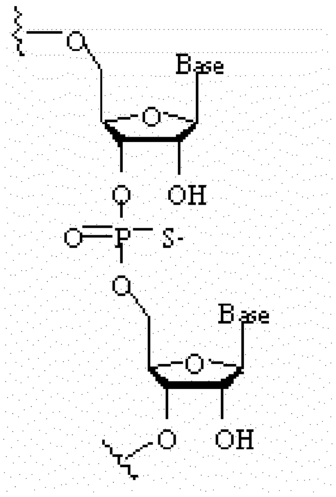

phosphorothioate modification

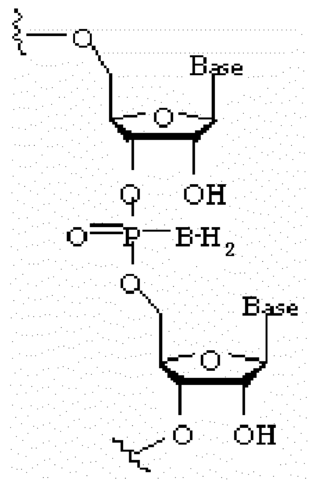

boranophosphate modification

Said modification of ribose refers to modifications of hydroxyl at 2'-position of the pentose sugars in siRNAs. Replacing the 2'-OH by methoxy or fluorine interfers with the interaction between the modified siRNA and serum ribonuclease, render the siRNA more stable in serum, and increase its resistance to nuclease degradation. Modifications of the 2'-hydroxyl of the pentose sugars comprises 2'-deoxy-2'-fluoro modification (2'-F), 2'-O-methyl modification (2'-OME), 2'-methoxyethyl-modification (2'-MOE), 2,4'-dinitrophenol-modification (2'-DNP), locked nucleic acid (LNA), 2'-amino modification (Amina modification), 2'-deoxy modification, etc.

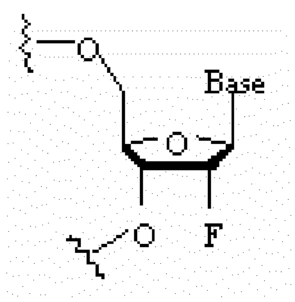

2'-deoxy-2'-fluoro modification

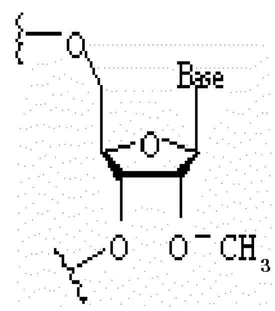

2'-O-methyl modification

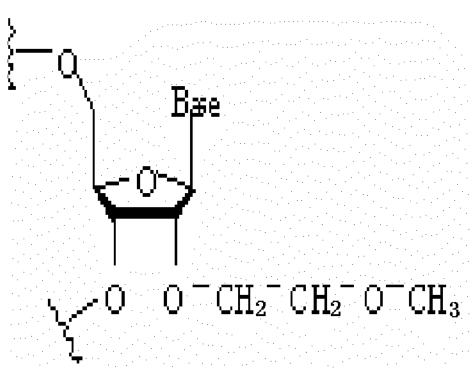

2'-methoxyethyl-modification

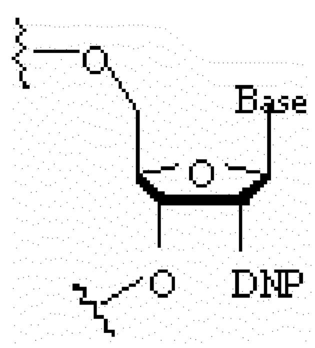

2,4'-dinitrophenol-modification

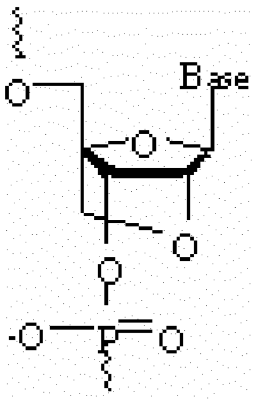

LNA

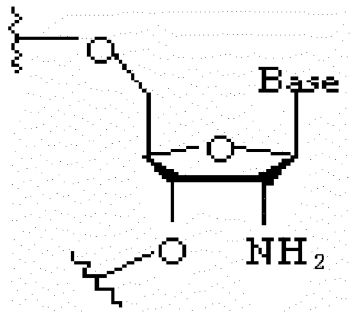

2'-amino modification

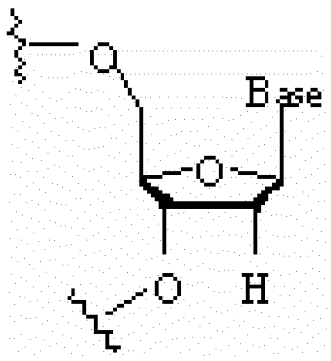

2'-deoxy modification

Said base modification refers to modifications of the base of the nucleotide, including commonly used modifications such as 5'-bromo-uracil and 5'-iodo-uracil, and other modifications such as N3-methyl-uracil and 2, 6-diaminopurine, and so on.

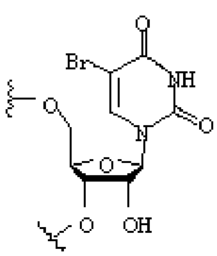

5'-bromo-uracil

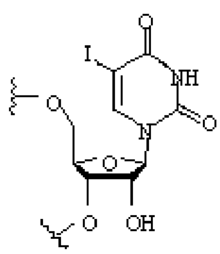

5'-iodo-uracil

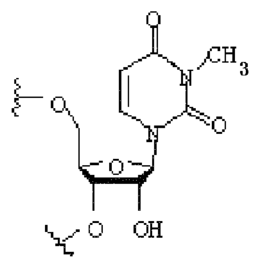

N3-methyl-uracil

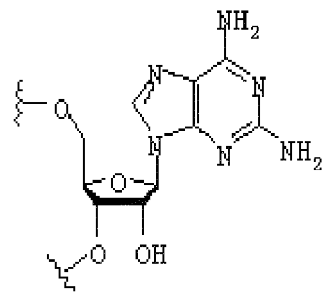

2, 6-diaminopurine

Preferably, modifed oligonucleotide provided in the present invention is a 2'-modified nucleotide. More preferably, the modified nucleotide is a 2'-OMe-modified or 2'-Fluoro-modified nucleotide. These modifications are made to increase the stability of the modified oligonucleotides in mammalian body fluids and enhance their resistance to nuclease degradation.

The present invention also relates to a method for preparing said modified oligonucleotide, comprising the steps of: selecting UA/UA and/or CA/UG site(s) from the oligonucleotide sequence to be prepared, synthesizing the oligonucleotide, wherein repalcing nucleotide(s) in the selected site(s) by corresponding modified nucleotide(s). The resulting oligonucleotide comprises a first nucleic acid fragment and a second nucleic acid fragment, the first nucleic acid fragment and the second nucleic acid fragment form double-stranded region. (a) said first nucleic acid fragment contains at least one contiguous UA sequence, the second nucleic acid fragment contains at least one contiguous UA sequence complementary to the UA sequence in the first nucleic acid fragment, the UA sequence(s) of the first nucleic acid fragment and the complementary UA sequence(s) of the second nucleic acid fragment pair to form UA/UA site(s), wherein at least one of the nucleotides in said UA/UA site(s) is a modified nucleotide; more preferably, only one uracil nucleotide in each UA/UA site is a modified nucleotide.

(b) The first nucleic acid fragment contains at least one contiguous CA or UG sequence, the second nucleic acid fragment contains at least one contiguous CA or UG sequence complementary to the CA or UG sequence in the first nucleic acid fragment, the CA or UG sequence(s) of the first nucleic acid fragment and the CA or UG sequence (s) of the second nucleic acid fragment pair to form CA/UG and/or UG/CA site(s), wherein at least one of the nucleotides in said CA/UG and/or UG/CA site(s) is a modified nucleotide; more preferably, only cytosine nucleotide (s) in CA/UG or UG/CA site(s) is/are modified nucleotide(s).

(c) The first nucleic acid fragment contains at least one contiguous UA sequence and at least one contiguous CA or UG sequence, the second nucleic acid fragment contains at least one contiguous UA sequence complementary to the UA sequence of the first nucleic acid fragment and at least one contiguous CA or UG sequence complementary to the CA or UG sequence of the first nucleic acid fragment, the UA sequence(s) of the first nucleic acid fragment and the UA sequence(s) of the second nucleic acid fragment pair to form UA/UA site(s), the CA or UG sequence (s) of the first nucleic acid fragment and the UG or CA sequence(s) of the second nucleic acid fragment pair to form CA/UG or UG/CA site(s); wherein at least one of the nucleotides in said UA/UA and CA/UG and/or UG/CA site(s) is a modified nucleotide; or, at least one of the nucleotides in said UA/UA site is a modified nucleotide, and at the same time, at least one of the nucleotides in said CA/UG and/or UG/CA site(s) is a modified nucleotide.

In a preferred embodiment, the present invention relates to a modified oligonucleotide prepared by the claimed method in the invention, wherein all the nucleotides are unmodified nucleotides except the nucleotides in said UA/UA and/or CA/UG and/or UG/CA site(s).

In the invention, the structures of said oligonucleotides prepared by the provided method are commonly understood by person having ordinary skill in the art to which this invention belongs. Said structures can be any stucture of existing nucleotide or oligonucleotide.

For example, the invention relates to a modified oligonucleotide that is prepared by the claimed method in the present invention can be a hairpin-structured and single-stranded nucleic acid molecule, the complementary nucleic acid sequences of the first nucleic acid fragment and the second nucleic acid fragment pair to form double-stranded region. In another aspect, the invention relates to a modified oligonucleotide that is prepared by the claimed method in the invention can contain a sense strand and an antisense strand, the sense strand and the antisense strand are

contiguous nucleic acid fragments. Said first nucleic acid fragment is in the sense strand, said second nucleic acid fragment is in the antisense strand.

In yet another aspect, the invention relates to a modified oligonucleotide that is prepared by the claimed method in the present invention can contain a sense strand and an antisense strand. The sense strand contains two or more discontiguous sense-strand component fragments, the antisense strand is a contiguous nucleic acid fragment. Said first nucleic acid fragment is in one or more sense-strand component fragments, the second nucleic acid fragment is in the antisense strand.

In yet another aspect, the invention relates to a modified oligonucleotide that is prepared by the claimed method in the present invention can be a hybrid nucleic acid molecule comprising ribonucleotides and at least one deoxyribonucleotide.

In general, the oligonucleotides of the present invention can be synthesized using conventional protocols known in the art. Alternatively, synthesis can be performed by specialized biotechnology companies providing oligonucleotide synthesis service, such as Shanghai GenePharma Ltd, Guangzhou Ribo Biotechnology Ltd, or Invitrogen Ltd.

In general, the procedure of oligonucleotide synthesis comprises the steps of: (a) Synthesis of oligonucleotide; (b) Deprotection; (c) Purification; and (d) Desalination.

For example, detailed procedure of synthesizing a siRNA comprises the steps of:

(a) Synthesis of oligonucleotide. Single-stranded RNA oligonucleotide is produced by solid phase synthesis on a scale of 1 mmol, using an automatic DNA/RNA synthesizer (for example, Applied Biosystems EXPEDITE8909). Coupling time is set to 10-15 minutes for each synthesis cycle. From the starting material of 5'-O-dimethoxy-thymidine solid support, the first nucleotide is connected to the support in the first synthesis cycle; and for each following synthesis cycle, a new nucleotide is connected to the product of cyclte n-1. Repeat the synthesis cycle until the completion of the synthesis of the oligonucleotide.

(b) Deprotection. Move siRNA-conjugated solid support into a test tube, and add 1 ml of ethanol/triethylamine (volume ratio of 1:3) in the test tube; seal the tube and place it in an incubator at 55 to 70˚C, incubate for 2 to 30 hours; take out the siRNA-conjugated solid support and wash twice with double-distilled water (1 ml for each wash); collect the eluate and dry at room temperature for 30 minutes. Then, add 1 ml of 1M tetrabutylammonium fluoride in tetrahydrofuran solution, react at room temperature for 4 to 12 hours; add 2 ml ethanol and collect the sediment for obtaining the crude product of synthesized oligonucleotide.

(c) Purification. Dissolve the obtained crude oligonucleotide in 2 ml ammonium acetate solution (1 mole/ml), separate the oligonucleotide using C18 high pressure liquid chromatography for purified product oligonucleotide.

(d) Desalination. To further remove salt from the oligonucleotide, wash the purified oligonucleotide product 2 to 4 times by ethanol solution (2 ml per wash, 75% in weight ratio), and dry the oligonucleotide product at room temperature. Then, dissolve the sense strand and the antisense strand in 1 to 2 ml annealing buffer (10mM Tris, pH 7.5-8.0, 50 mM NaCl), heat the solution to 95˚C and let it cool down slowly to room temperature, and keep the solution at room temperature for 16 to 22 hours for obtaining desalted siRNA solution.

The present invention also relates to a pharmaceutical composition for inhibiting the expression of a target gene in a mammal. Said pharmaceutical composition comprises at least one of the modified oligonucleotides disclosed in the invention, and at least one of pharmaceutically acceptable carriers. As used herein, "pharmaceutically acceptable carrier" should be compatible with the oligonucleotide(s) included in said pharmaceutical composition. In general, the "pharmaceutically acceptable carrier" can blend with the oligonucleotide(s) and do not significantly compromise the inhibition activity of the pharmaceutical composition on gene expression, under normal circumstances.

Examples of the substance of "pharmaceutically acceptable carrier" or components of the "pharmaceutically acceptable carrier" are sugars, such as lactose, glucose and sucrose; starch such as corn starch and potato starch; cellulose and its derivatives such as carboxymethyl fiber sodium, ethyl cellulose and methyl cellulose; West tragacanth powder; malt; gelatin; talc; solid lubricant such as stearic acid and magnesium stearate; calcium sulfate; vegetable oils such as peanut oil, cottonseed oil, sesame oil, olive oil, corn oil and cocoa butter; polyols such as propylene glycol, glycerol, sorbitol, mannose alcohol and polyethylene glycol; alginate; emulsifiers such as Tween; wetting agents, such as lauryl sulfate sodium; colorants; flavoring agent; pressure tablets, stabilizers; antioxidants; preservatives; pyrogen-free water; isotonic salt solution; and phosphate buffer, etc. Preferably, the carriers are selected from saline, glycerol and phosphate phosphate buffer saline.

The pharmaceutical composition provided by the invention can be made in a variety of medically acceptable formulations, and administrated by physicians in an optimal dosage according to patient's type, age, weight, disease condition, delivery approach and other factors. The formulations provided by the present invention includes a variety of liquid formulations such as oral, injection, sublingual agent, tablet formulations prepared by adding suitable excipient, capsule formulation, or a variety of other formulations, etc. Preferred formulations of said pharmaceutical composition comprise injection, capsules, sublingual, oral liquid, aerosol, or patch.

In another aspect, the invention also provides a method for preparing the pharmaceutical composition, said method comprises formulating the modified oligonucleotides and pharmaceutically acceptable carriers to obtain the said pharmaceutical compositions. No particular order or rules need to be applied to the mixing of the modified oligonucleotides and the pharmaceutically acceptable carriers.

In another aspect, the invention also relates to a method for inhibiting the expression of a target gene in a cell, compreseing:

(a) introducing the modified oligonucleotide(s) into at least one of the cells; and

(b) incubating the cells for a time sufficient to obtain inhibition of the expression of the target gene in the cell.

Preferably, the cells are mammalian cells.

The present invention also relates to a method for preparing an oligonucleotide that is highly stable in a biological sample and can inhibite the expression of a target gene, comprising the steps of:

(a) selecting one or more nucleic acid sequences of 18 to 30 nucleotides in length from the nucleotide sequence of the mRNA resulting from the transcription of the target gene; and

(b) synthesizing the selected sequences, wherein one strand comprises a sequence complementary to selected sequence in (a); and

(c) testing one or more oligonucleotides of (b) for their activity to inhibit the expression of the target gene in a biological sample; and

(d) selecting one or more oligonucleotides of (c) possessing the activity to inhibit the expression of the target gene in a biological sample; and

(e) in the oligonucleotides selected in (d), identifying in the nucleotide sequences of all occurrences of the UA/UA, CA/UG and UG/CA site(s); and

(f) synthesizing one or more oligonucleotides selected in (d), wherein at least one nucleotide in the UA/UA, CA/UG and UG/CA site(s) identified in (e) is replaced by its corresponding modified nucleotide.

Preferably, only cytidine nucleotide(s) in all occurrences of the CA/UG or UG/CA site is/are modified, only one uracil nucleotide in each UA/UA site is modified; more preferably, all the nucleotides are unmodified nucleotides except the nucleotides in said CA/UG and/or UA/UA and/or UG/CA site(s).

A method of treating a disease caused by expression of a target gene in a subject is provided in the present invention. Said method comprises administering to said subject a pharmaceutical composition comprising at least one of said oligonucleotides and a pharmaceutically acceptable carrier. Preferably, the subject is mammalian; more preferably, the subject is human being.

The present invention also relates to a method for preparing an oligonucleotide with nuclease resistance, said method comprises the steps of:

(a) identifying in the nucleotide sequence of the oligonucleotide all occurrences of UA/UA, CA/UG and UG/CA site (s); and

(b) synthesizing the oligonucleotide, wherein replaces nucleotide(s) in the UA/UA, CA/UG and UG/CA site(s) identified in (a) by corresponding modified nucleotide(s). Preferably, only cytidine nucleotide(s) in all occurrences of the CA/UG or UG/CA site(s) identified in (a) is/are modified, only one uracil nucleotide in each UA/UA site identified in (a) is modified. More preferably, all the nucleotides are unmodified nucleotides except the nucleotides in said CA/UG and/or UA/UA and/or UG/CA site(s).

The invention also relates to a method to identify an oligonucleotide with stability in biological samples, comprising the steps of:

(a) providing a first modified oligonucleotide, and a second oligonucleotide identical in sequence to the first oligonucleotide except that it dose not have modified nucleotide(s); and

(b) determining the stability and the degradation process of said first and second oligonucleotide in the biological sample by contacting the two oligonucleotides with the biological sample under indentical conditions,

Whereby, where the first modified oligonucleotide is degraded less rapidly than the second oligonucleotide, the oligonucleotide with stability in a biological sample is identified.

By specifically modifying the UA/UA and/or CA/UG and/or UG/CA site(s) identified in an oligonucleotid, the invention achieves stabilizing the modified oligonucleotide by introducing only a relatively small amount chemically modified nucleotides into the oligonucleotide. Compared to randomly modified oligonucleotide in the art, the specific modification approach disclosed in the present invention greatly reduces the potential cytotoxicity and influence on the biological activity of the modified oligonucleotide.

Inventors of the present invention performed comprehensive investigation on the *in vivo* degradation of synthetic oligonucleotide, and found that replacing a relatively small amount of the nucleotides in UA/UA and/or CA/UG site(s) identified in a oligonucleotide sequence by corresponding chemically modified nucleotide(s) can greatly increase the stability of the modified oligonucleotide in biological samples, and at the same time, reduces the cytotoxicity caused by extensive and random modifications usually performed in the art, as well as the effects on biological activity.

The present invention is further illustrated using the following embodiments. Should be understood that the materials, methods, and examples described in the present invention are illustrative only and not intented to be limiting. Unless otherwise defined, all reagents and culture medium used in the invention are commercially available.

Example 1. Synthesis of siRNA oligonucleotides

siRNA oligonucleotides against target genes in table 1-182 were synthesized by Genepharma Ltd (Shanghai, China).

Example 2. Assays of serum stability, silencing efficacy and cytotoxicity of variously modified siRNA

Serum stability, silencing efficacy and cytotoxicity assay of modified and unmodified siRNAs in table 1-182 were tested using the methods of:

1. Serum stability assay

Serum degradation assays were performed at 37˚C by incubating 4 μL 20 μM modified or unmodified siRNA in 32 μL 1×PBS solution, containing 4 μL fetal bovine serum (Sigma). The final serum concentration was 10%. After serum treatment of 0, 3 or 6 hours, 10μL reaction solution was removed and frozen immediately in liquid nitrogen to terminate the reaction. The samples were then stored at -80˚C until analysis.

Prepare a 20% polyacrylamide gel; mix 3 uL 3× loading buffer (30mM EDTA, 36% glycerol, 0.06% bromophenol blue) with serum-treated siRNA sample; then load the sample into the gel and run electrophoresis at 80 mA under constant current condition. After electrophoresis, add 1 × Sybr Gold (Invitrogen, Cat. 11494) into the sample and let it stain for 10 minutes before imaging and analysis. The results were presented in Table 1-182.

2. Silencing efficacy assay

Human embryonic kidney cells (HEK293) were grown in Dulbecco's modified Eagle's medium supplemented with 10% fetal bovine serum, 2 mM L-glutamine, 100 units/ml penicillin and 100 μg/ml streptomycin (Life Technologies, Gibco), and seeded into 24-well plates at a density of $1\times10^5$ cells (0.5mL culture medium/well). After 24 hours incubation and the cell density reached 50% confluence, the culture medium was changed to Opti-MEM (Gibco). Lipofectamine™ 2000 transfection reagent (Invitrogen) was used to co-transfect siRNA and reporter plasmids. Report vector (0.17 μg/well) carrying the target site of tested siRNA and *firefly* luciferase gene was transfected into the cells together with pRL-TK control vector carrying *renilla* luciferase gene. The final concentration of siRNA was 13 nM. Each siRNA was parallely transfected in three wells, with the same amount of the two reporter plasmids. The three wells without siRNA treatment were used as control. Four hours after transfection, the culture medium was changed to one milliliter DMEM growth medium (10% fetal bovine serum, 2 mM L-glutamine, 100 units/ml penicillin and 100 μg/ml streptomycin). Twenty-four later, the cells were lysated by adding 10 uL cell lysis buffer, and the activity of both luciferases was measured with a fluorometer (Synergy HT, BioTek, USA), using Dual-Luciferase reporter assay system (Promega) according to the manufacturer's instructions. Silencing efficacy of the siRNA was calculated by the following formula, using siRNA-untreated cells as control. All the experiments were performed in triplicate and repeated for at least twice. The results were shown in table 1-182.

Silencing efficacy= (the expressional amount of firefly luciferase in test group/ the expressional amount of

renilla luciferase in test group)/ (the expressional amount of firefly luciferase in control group/ the expressional amount of renilla luciferase in control group)

3. Cytotoxicity assay of modified siRNA

Human embryonic kidney cells (HEK293) were grown in Dulbecco's modified Eagle's medium supplemented with 10% fetal bovine serum, 2 mM L-glutamine, 100 units/ml penicillin and 100 μg/ml streptomycin (Life Technologies, Gibco), and seeded into 24-well plates at a density of $1\times10^5$ cells (0.5mL culture medium/well). After 24 hours incubation and the cell density reached 50% confluence, the culture medium was changed to Opti-MEM (Gibco). Lipofectamine™ 2000 transfection reagent (Invitrogen) was used to transfect chemically synthetic siRNA into the cells at a final concentration of 13 nM. Each siRNA was parallely transfected in three independent wells, with three other siRNA-untreated wells as control. Four hours after transfection, the culture medium was changed to one milliliter DMEM growth medium (10% fetal bovine serum, 2 mM L-glutamine, 100 units/ml penicillin and 100 μg/ml streptomycin).

Twenty-four later, removed the culture medium and washed the cells once with PBS. Add 1 mL PBS and 10 μL MTT into each well, incubate in a 5% $CO_2$ incubator at 37°C for 4-6 hours; add 0.1 mL acidified isopropanol into each well, mix by vortexing; measure the absorbance value (A value) at 570nm for each well, using a microplate reader. The growth inhibition ratio was calculated using the following formula, the results were shown in table 1-182.

$$\text{Growth inhibition ratio (\%)} = (\text{A value of test group} - \text{A value of control group}) / \text{A value of control group} \times 100\%$$

Table 1

| Gene | Human KAZRIN (NM_201628) (SEQ ID NO:2) siRNA | Locus Stability | 718-738 BP Expression inhibition ratio | Growth inhibition ratio |
|---|---|---|---|---|
| 1-1 | Unmodified siRNA<br>5-AGCGCUUAAAGGGCGAGAAGAUU<br>UUUCGCGAAUUUCCCGCUCUUCU-5 | + | 84% | 0% |
| 1-2A | Random modification (2'-deoxy-2'-fluoro)<br>5-A**G**C**G**C**UU**AAA**GGG**C**G**A**G**AA**G**AUU<br>UUUCGCGAAUUUCCCGCUCUUCU-5 | ++ | 27% | 37% |
| 1-2B | Random modification (2'-O-methyl)<br>5-AGCGCUUAAAGGGCGAGAAGAUU<br>**UUU**C**G**C**G**AA**UUU**CCC**G**C**U**C**UU**C**U**-5 | ++ | 0% | 32% |
| 1-3A | Specific modification (2'-O-methyl)<br>5-AGCGCU**UA**AAGGGCGAGAAGAUU<br>UUUCGCGA**AU**UUCCCGCUCUUCU-5 | ++ | 27% | 15% |
| 1-3B | Specific modification (2'-deoxy-2'fluoro)<br>5-AGCGCU**U**AAAGGGCGAGAAGAUU<br>UUUCGCGA**AU**UUCCCGCUCUUCU-5 | +++ | 44% | 12% |
| 1-3C | Specific modification (2'-O-methyl) | | | |

(continued)

| Gene | Human KAZRIN (NM_201628) (SEQ ID NO:2) | Locus | 718-738 BP | |
|---|---|---|---|---|
| | siRNA | Stability | Expression inhibition ratio | Growth inhibition ratio |
| | 5-AGCGCU**UA**AAGGGCGAGAAGAUU<br>UUUCGCGA**A**UUUCCCGCUCUUCU-5 | ++ | 56% | 10% |
| 1-3D | Specific modification (2'-O-methyl)<br>5-AGCGCU**U**AAAGGGCGAGAAGAUU<br>UUUCGCGA**A**UUUCCCGCUCUUCU-5 | ++ | 81% | 11% |
| 1-3E | Specific modification (2'-O-methyl)<br>5-AGCGCU**U**AAAGGGCGAGAAGAUU<br>UUUCGCGAA**U**UUCCCGCUCUUCU-5 | +++ | 49% | 10% |
| 1-3F | Specific modification (2'-deoxy-2'-fluoro)<br>5-AGCGCUUAAAGGGCGAGAAGAUU<br>UUUCGCGA**AU**UUCCCGCUCUUCU-5 | ++ | 49% | 14% |
| 1-3G | Specific modification (2'-deoxy-2'-fluoro)<br>5-AGCGCU**U**AAAGGGCGAGAAGAUU<br>UUUCGCGAAUUUCCCGCUCUUCU-5 | ++ | 85% | 0% |
| 1-3H | Specific modification (2'-O-methyl)<br>5-AGCGCUUAAAGGGCGAGAAGAUU<br>UUUCGCGA**A**UUUCCCGCUCUUCU-5 | ++ | 84% | 6% |
| 1-31 | Specific modification (2'-O-methyl)<br>5-AGCGCU**UA**AAGGGCGAGAAGAUU<br>UUUCGCGAA**U**UUCCCGCUCUUCU-5 | ++ | 23% | 10% |
| 1-3J | Specific modification (2'-O-methyl)<br>5-AGCGCUU**A**AAGGGCGAGAAGAUU<br>UUUCGCGA**AU**UUCCCGCUCUUCU-5 | ++ | 41% | 14% |
| 1-3K | Specific modification (2'-deoxy-2'-fluoro)<br>5-AGCGCUU**A**AAGGGCGAGAAGAUU<br>UUUCGCGAA**U**UUCCCGCUCUUCU-5 | ++ | 47% | 15% |
| 1-3L | Specific modification (2'-O-methyl)<br>5-AGCGCUU**A**AAGGGCGAGAAGAUU<br>UUUCGCGA**A**UUUCCCGCUCUUCU-5 | ++ | 83% | 12% |

(continued)

| Gene | Human KAZRIN (NM_201628) (SEQ ID NO:2) siRNA | Locus Stability | 718-738 BP Expression inhibition ratio | Growth inhibition ratio |
|---|---|---|---|---|
| 1-3M | Specific modification (2'-O-methyl)<br>5-AGCGCU**UA**AAGGGCGAGAAGAUU<br>UUUCGCGAAUUUCCCGCUCUUCU-5 | ++ | 59% | 7% |
| 1-3N | Specific modification (2'-deoxy-2'-fluoro)<br>5-AGCGCUUAAAGGGCGAGAAGAUU<br>UUUCGCGAA**U**UUCCCGCUCUUCU-5 | ++ | 49% | 0% |
| 1-3O | Specific modification (2'-O-methyl)<br>5-AGCGCUU**A**AAGGGCGAGAAGAUU<br>UUUCGCGAAUUUCCCGCUCUUCU-5 | ++ | 85% | 5% |
| 1-3P | Unmodified siRNA(hairpin, unmodified)<br>5-AGCGCUUAAAGGGCGAGAAGAUU**N**<br>UUUCGCGAAUUUCCCGCUCUUCU**NNN** | +++ | 85% | 0% |
| 1-3Q | Specific modification (hairpin, 2'-O-methyl)<br>5-AGCGCU**U**AAAGGGCGAGAAGAUU**N**<br>UUUCGCGAA**U**UUCCCGCUCUUCU**NNN** | +++ | 53% | 10% |
| 1-3R | Specific modification (discontiguous sense strand, 2'-deoxy-2'-fluoro)<br>5-AGCGCU**U**AAAGG**\|**GCGAGAAGAUU<br>UUUCGCGAAUUUCCCGCUCUUCU-5 | +++ | 78% | 2% |
| 1-3S | Specific modification (hybrid molecule, 2'-O-methyl)<br>5-AGCGCU**U**AAAGGGCG(**DA**)GAAGAUU<br>UUUCGCGAA**U**UUCCCGCUCUUCU-5 | ++ | 50% | 7% |
| 1-3T | Specific modification (hybrid molecule, 2'-deoxy-2'-fluoro)<br>5-AGCGCUU**A**AAGGGCG(**DA**)GAAGAUU<br>UUUCGCGA**A**UUUCCCGCUCUUCU-5 | ++ | 79% | 9% |
| 1-3U | Specific modification (phosphodiester bond modification)<br>5-AGCGCU**U**AAAGGG**-**CGAGAAGAUU<br>UUUCGCGAA**U**UUCCCGCUCUUCU-5 | +++ | 45% | 11% |
| 1-3V | Specific modification (2'-deoxy-2'-fluoro) | | | |

(continued)

| Gene | Human KAZRIN (NM_201628) (SEQ ID NO:2) siRNA | Locus Stability | 718-738 BP Expression inhibition ratio | Growth inhibition ratio |
|---|---|---|---|---|
| | 5-AGCGCU**U**AAAGGGCGAGAAGAUU<br>UUUCGCGAA**U**UUCCCGCUCUUCU-5 | +++ | 51% | 8% |

Table 2

| Gene | Mouse (NM_008960.2) (SEQ ID NO:9) SIRNA | Locus Stability | 1148-1166 BP Expression inhibition ratio | Growth inhibition ratio |
|---|---|---|---|---|
| 2-1 | Unmodified siRNA<br>5-AACCCACCACAGCUAGAACUU<br>UUGGGUGGUGUCGAUCUUGAA-5 | + | 86% | 0% |
| 2-2A | Random modification (2'-O-methyl)<br>5-AACCCACCACAGCUAGAACUU<br>**UU**G**GGUGGUGU**C**G**A**U**C**UUG**AA-5 | ++ | 15% | 34% |
| 2-2B | Random modification (2'-deoxy-2'-fluoro)<br>5-**AA**CCC**A**CC**A**C**A**GC**UA**G**AA**C**UU**<br>UUGGGUGGUGUCGAUCUUGAA-5 | ++ | 37% | 38% |
| 2-3A | Specific modification (2'-deoxy-2'-fluoro)<br>5-AACCCACCACAGC**UA**GAACUU<br>UUGGGUGGUGUCG**AU**CUUGAA-5 | ++ | 57% | 17% |
| 2-3B | Specific modification (2'-O-methyl)<br>5-AACCCACCACAGC**UA**GAAC**UU**<br>**UU**GGG**U**GG**U**G**U**CG**AU**C**UU**GAA-5 | +++ | 24% | 34% |
| 2-3C | Specific modification (2'-O-methyl)<br>5-AACCCACCACAGC**UA**GAAC**UU**<br>UUGGGUGGUGUCG**AU**CUUGAA-5 | +++ | 51% | 17% |
| 2-3D | Specific modification (2'-O-methyl)<br>5-**AA**CCC**A**CC**A**C**A**GC**UA**G**AA**C**UU**<br>UUGGGUGGUGUCG**AU**CUUGAA-5 | ++ | 44% | 37% |
| 2-3E | Specific modification (2'-O-methyl) | | | |

(continued)

| Gene | Mouse (NM_008960.2) (SEQ ID NO:9) SIRNA | Locus Stability | 1148-1166 BP Expression inhibition ratio | Growth inhibition ratio |
|---|---|---|---|---|
| | 5-**AA**CCC**A**CC**A**C**A**GC**UA**G**AA**C**UU**<br>**UU**GGG**U**GG**U**G**U**CG**AU**C**UU**G**AA**-5 | +++ | 7% | 52% |
| 2-3F | Specific modification (2'-O-methyl)<br>5-AACCCACCACAGC**U**AGAAC**UU**<br>UUGGGUGGUGUCGA**U**CUUGAA-5 | ++ | 81% | 14% |
| 2-3G | Specific modification (2'-O-methyl)<br>5-**AA**CCC**A**CC**A**C**A**GC**UA**G**AA**CUU<br>UUGGGUGGUGUCGA**U**CUUGAA-5 | +++ | 32% | 35% |
| 2-3H | Specific modification (2'-O-methyl)<br>5-AACCCACCACAGC**UA**GAACUU<br>UUGGGUGGUGUCGAUCUUGAA-5 | ++ | 79% | 6% |
| 2-3I | Specific modification (2'-O-methyl)<br>5-**AA**CCC**A**CC**A**C**A**GC**UA**G**AA**C**UU**<br>UUGGGUGGUGUCGAUCUUGAA-5 | ++ | 51% | 42% |
| 2-3J | Specific modification (2'-deoxy-2'-fluoro)<br>5-AACCCACCACA**G**C**UAG**AACUU<br>UUGGGUGGUGUCGAUCUUGAA-5 | ++ | 75% | 16% |
| 2-3K | Specific modification (2'-O-methyl)<br>5-AACCCACCACAGC**U**AGAACUU<br>UUGGGUGGUGUCGAUCUUGAA-5 | ++ | 84% | 4% |
| 2-3L | Specific modification (2'-O-methyl)<br>5-AACCCACCACAGC**U**AGAAC**UU**<br>UUGGGUGGUGUCGAUCUUGAA-5 | ++ | 81% | 3% |
| 2-3M | Specific modification (2'-deoxy-2'-fluoro)<br>5-**AA**CCC**A**CC**A**C**A**GC**UA**G**AA**C**UU**<br>UUGGGUGGUGUCGAUCUUGAA-5 | +++ | 54% | 33% |
| 2-3N | Specific modification (2'-O-methyl)<br>5-AACCCACCACAGC**U**AGAACUU**NN**<br>UUGGGUGGUGUCGAUCUUGAA**NN** | +++ | 82% | 5% |
| 2-3O | Specific modification (2'-deoxy-2'-fluoro) | | | |

(continued)

| Gene | Mouse (NM_008960.2) (SEQ ID NO:9) SIRNA | Locus Stability | 1148-1166 BP Expression inhibition ratio | Growth inhibition ratio |
|---|---|---|---|---|
| | 5-AACCCACCACAGCUAGAACUU<br>**UU**GGG**U**GG**U**G**U**CGA**U**C**UU**GAA-5 | ++ | 14% | 23% |
| 2-3P | Unmodified siRNA (hairpin, unmodified)<br>5-AACCCACCACAGC**U**AGAACUU**NN**<br>UUGGGUGGUGUCGAUCUUGAA**NN** | +++ | 78% | 5% |
| 2-3Q | Specific modification (hairpin, 2'-O-methyl)<br>5-**AA**CCC**A**CC**A**C**A**GCU**A**G**AA**C**UU**<br>UUGGGUGGUGUCGAUCUUGAA-5 | +++ | 42% | 34% |

Table 3

| gene | Human BIC(NR 001458.3)(SEQ ID NO:1) SIRNA | Locus Stability | 289-310 BP Expression inhibition ratio | Growth inhibition ratio |
|---|---|---|---|---|
| 3-1 | Unmodified siRNA<br>5-UUAAUGCUAAUCGUGAUAGGGGUU<br>AUAAUUACGAUUAGCACUAUCCCC-5 | + | 88% | 0% |
| 3-2A | Random modification (2'-O-methyl)<br>5-**UUAAU**GC**UAAU**CG**U**G**AUA**GGGG**UU**<br>AUAAUUACGAUUAGCACUAUCCCC-5 | ++ | 49% | 41% |
| 3-2B | Random modification (2'-deoxy-2'-fluoro)<br>5-UUAAUGCUAAUCGUGAUAGGGGUU<br>**AUAAUUA**CG**AUUA**GC**A**C**UAU**CCCC-5 | ++ | 15% | 37% |
| 3-3A | Specific modification (2'-O-methyl)<br>5-U**U**AAUGC**U**AAUCGUGAUAGGGGUU<br>AUAA**U**UACGA**U**UAGCACUAUCCCC-5 | +++ | 80% | 11% |
| 3-3B | Specific modification (2'-O-methyl)<br>5-U**U**AAU**G**C**U**AAUC**G**U**G**AUA**GGGG**UU<br>AUAA**U**UACGA**U**UAGCACUAUCCCC-5 | +++ | 51% | 31% |
| 3-3C | Specific modification (2'-deoxy-2'-fluoro) | | | |

(continued)

| gene | Human BIC(NR 001458.3)(SEQ ID NO:1) SIRNA | Locus Stability | 289-310 BP Expression inhibition ratio | Growth inhibition ratio |
|---|---|---|---|---|
| | 5-**UU**AA**U**GC**U**AA**U**CG**U**GA**U**AGGGG**UU**<br>AUAA**U**UACGA**U**UAGCACUAUCCCC-5 | +++ | 45% | 57% |
| 3-3D | Specific modification (2'-deoxy-2'-fluoro) | | | |
| | 5-U**UAA**UGC**UAA**UCGUG**A**U**A**GGGGUU<br>AUAA**U**UACGA**U**UAGCACUAUCCCC-5 | ++ | 65% | 32% |
| 3-3E | Specific modification (2'-O-methyl) | | | |
| | 5-U**UA**AUGC**UA**AUCGUGAUAGGGGUU<br>AUAA**U**UACGA**U**UAGCACUAUCCCC-5 | ++ | 75% | 27% |
| 3-3F | Specific modification (2'-O-methyl) | | | |
| | 5-**UUAAU**GC**UAAU**CG**U**G**AUA**GGGG**UU**<br>AUAA**U**UACGA**U**UAGCACUAUCCCC-5 | ++ | 49% | 48% |
| 3-3G | Specific modification (2'-O-methyl) | | | |
| | 5-**UUA**A**U**GC**UA**A**U**CG**U**GA**U**AGGGG**UU**<br>A**U**AA**UU**ACGA**UU**AGCAC**U**A**U**CCCC-5 | ++ | 23% | 52% |
| 3-3H | Specific modification (2'-O-methyl) | | | |
| | 5-U**UA**AU**G**C**UA**AUC**G**U**G**AUA**GGGG**UU<br>AUAA**U**UACGA**U**UAGCACUAUCCCC-5 | ++ | 17% | 42% |
| 3-3I | Specific modification (2'-deoxy-2'-fluoro) | | | |
| | 5-U**UA**AUGC**UA**AUCGUGAUAGGGGUU<br>AUA**AU**UACG**AU**UAGCACUAUCCCC-5 | ++ | 65% | 21% |
| 3-3J | Specific modification (2'-O-methyl) | | | |
| | 5-**UUA**A**U**GC**UA**A**U**CG**U**GA**U**AGGGG**UU**<br>A**U**A**AUU**ACG**AUU**AGCAC**U**AUCCCC-5 | ++ | 17% | 51% |
| 3-3K | Specific modification (2'-O-methyl) | | | |
| | 5-**UUA**A**U**GC**UA**A**U**CG**U**GA**U**AGGGG**UU**<br>AUA**AU**UACG**AU**UAGCACUAUCCCC-5 | ++ | 35% | 48% |
| 3-3L | Specific modification (2'-deoxy-2'-fluoro) | | | |
| | 5-**UUAAU**GC**UAAU**CG**U**G**AUA**GGGG**UU**<br>AUA**AU**UACG**AU**UAGCACUAUCCCC-5 | ++ | 53% | 45% |
| 3-3M | Specific modification (2'-deoxy-2'-fluoro) | ++ | 28% | 39% |

(continued)

| gene | Human BIC(NR 001458.3)(SEQ ID NO:1) SIRNA | Locus | 289-310 BP | |
|---|---|---|---|---|
| | | Stability | Expression inhibition ratio | Growth inhibition ratio |
| | 5-U**UA**AUGC**UA**AUCGUGAUAGGGGUU<br>A**U**A**AUU**ACG**AUU**AGCAC**U**A**U**CCCC-5 | | | |
| 3-3N | Specific modification (2'-O-methyl)<br>5-U**UA**AUGC**UA**AUCGUGAUAGGGGUU<br>**AUAAUUA**CG**AUUA**GC**A**C**UAU**CCCC-5 | +++ | 8% | 49% |
| 3-3O | Specific modification (2'-O-methyl)<br>5-U**U**AAUGC**U**AAUCGUGA(**DT**)AGGGGUU<br>AUAA**U**UACGA**U**UAGCACUAUCCCC-5 | ++ | 70% | 9% |
| 3-3P | Specific modification (2'-O-methyl)<br>5-U**U**AAUGC**U**AAUCGUGAAGG(**DG**)GUU<br>AUAA**U**UACGA**U**UAGCACUAUCCCC-5 | ++ | 72% | 12% |

Table 4

| gene | Human BIC(NR 001458.3)(SEQ ID NO:1) SIRNA | Locus | 745-763 BP | |
|---|---|---|---|---|
| | | Stability | Expression inhibition ratio | Growth inhibition ratio |
| 4-1 | unmodified<br>5-AGAAGGACUACUAACCUCCTT<br>TTUCUUCCUGAUGAUUGGAGG-5 | ++ | 83% | 0% |
| 4-2 | Random modification (2'-O-methyl)<br>5-AGAAGGAC**U**AC**U**AACC**U**CCTT<br>TT**U**C**UU**CC**U**GA**U**GA**UU**GGAGG-5 | ++ | 49% | 41% |
| 4-3A | Specific modification (2'-O-methyl)<br>5-AGAAGGA**CU**A**CU**AA**CCUCC**TT<br>TTUCUUCCUGAUGAUUGGAGG-5 | ++ | 80% | 15% |
| 4-3B | Specific modification (2'-O-methyl)<br>5-AGAAGGAC**U**AC**U**AACCUCCTT<br>TTUCUUCCUGA**U**GAUUGGAGG-5 | +++ | 81% | 5% |

Table 5

| gene | Human BIC(NR 001458.3)(SEQ ID NO:1) SIRNA | Locus Stability | 749-767 BP Expression inhibition ratio | Growth inhibition ratio |
|---|---|---|---|---|
| 5-1 | Unmodified siRNA<br>5-GGACUACUAACCUCCAGUUTT<br>TTCCUGAUGAUUGGAGGUCAA-5 | + | 69% | 0% |
| 5-2 | Random modification (2'-O-methyl)<br>5-GGA**CU**A**CU**AA**CCUCC**AG**UU**TT<br>TT**CCU**GA**U**GA**UU**GGAGG**UC**AA-5 | ++ | 7% | 53% |
| 5-3A | Specific modification (2'-O-methyl)<br>5-**GGA**CU**A**CU**AA**CCUCC**AG**UUTT<br>TTCCUGAUGAUUGGAGGUCAA-5 | ++ | 65% | 21% |
| 5-3B | Specific modification (2'-O-methyl)<br>5-GGAC**U**AC**U**AACCUC**C**AGUUTT<br>TTCCUGA**U**GA**U**UGGAGG**U**CAA-5 | +++ | 69% | 9% |

Table 6

| gene | Human BIC(NR 001458.3)(SEQ ID NO:1) SIRNA | Locus Stability | 807-825 BP Expression inhibition ratio | Growth inhibition ratio |
|---|---|---|---|---|
| 6-1 | Unmodified siRNA<br>5-GAAACUGGUACUUUCCCCCTT<br>TTCUUUGACCAUGAAAGGGGG-5 | + | 69% | 0% |
| 6-2 | Random modification (2'-deoxy-2'-fluoro)<br>5-G**A**AA**CU**GG**U**A**CUUUCCCCC**TT<br>TTCUUUGACCAUGAAAGGGGG-5 | ++ | 60% | 37% |
| 6-3A | Specific modification (2'-deoxy-2'-fluoro)<br>5-GAAACUGGUACUUUCCCCCTT<br>TTC**UUU**GACCA**U**GAAAGGGGG-5 | ++ | 26% | 12% |
| 6-3B | Specific modification (2'-deoxy-2'-fluoro)<br>5-GAAACUGG**U**ACUUUCCCCCTT | ++ | 58% | 8% |

(continued)

| gene | Human BIC(NR 001458.3)(SEQ ID NO:1) | Locus | 807-825 BP | |
|---|---|---|---|---|
| | SIRNA | Stability | Expression inhibition ratio | Growth inhibition ratio |
| | TTCUUUGACCA**U**GAAAGGGGG-5 | | | |

Table 7

| gene | Human BIC(NR 001458.3)(SEQ ID NO:1) | Locus | 818-836 BP | |
|---|---|---|---|---|
| | SIRNA | Stability | Expression inhibition ratio | Growth inhibition ratio |
| 7-1 | Unmodified siRNA<br>5-UUUCCCCCAGGUAACGAUUTT<br>TTAAAGGGGGUCCAUUGCUAA-5 | + | 70% | 0% |
| 7-2 | Random modification (2'-deoxy-2'-fluoro)<br>5-UUU**CCCCC**AGGUAA**C**GAUUTT<br>TTAAAGGGGGU**CC**AUUG**C**UAA-5 | ++ | 68% | 21% |
| 7-3A | Specific modification (2'-deoxy-2'-fluoro)<br>5-UUUCCCCC**AGG**U**AA**C**GA**UUTT<br>TTAAAGGGGGUCCAUUGCUAA-5 | ++ | 78% | 15% |
| 7-3B | Specific modification (2'-deoxy-2'-fluoro)<br>5-UUUCCCCCAGG**U**AACGAUUTT<br>TTAAAGGGGGUCCA**U**UGCUAA-5 | ++ | 75% | 8% |

Table 8

| gene | Human BIC(NR_001458.3)(SEQ ID NO:1) | Locus | 822-840 BP | |
|---|---|---|---|---|
| | SIRNA | Stability | Expression inhibition ratio | Growth inhibition ratio |
| 8-1 | Unmodified siRNA<br>5-CCCCAGGUAACGAUUUUCUTT<br>TTGGGGUCCAUUGCUAAAAGA-5 | + | 86% | 0% |
| 8-2 | Random modification (2'-O-methyl)<br>5-C**C**C**CA**G**G**G**U**A**A**C**G**AUU**U**UCUTT<br>TTGGGGU**CC**AUUG**C**UAAAAGA-5 | ++ | 69% | 53% |
| 8-3A | Specific modification (2'-O-methyl) | | | |

(continued)

| gene | Human BIC(NR_001458.3)(SEQ ID NO:1) SIRNA | Locus Stability | 822-840 BP Expression inhibition ratio | Growth inhibition ratio |
|---|---|---|---|---|
| | 5-CCCC**AGG**U**AA**C**GA**UUUUCUTT<br>TTGGGGUCCAUUGCUAAAAGA-5 | ++ | 79% | 12% |
| 8-3B | Specific modification (2'-O-methyl)<br>5-CCCCAGG**U**AACGAUUUUCUTT<br>TTGGGGUCCA**U**UGCUAAAAGA-5 | ++ | 70% | 5% |

Table 9

| gene | Human BIC(NR_001458.3)(SEQ ID NO:1) SIRNA | Locus Stability | 889-907 BP Expression inhibition ratio | Growth inhibition ratio |
|---|---|---|---|---|
| 9-1 | Unmodified siRNA<br>5-GGGAAACUGAAAGGCUAUGTT<br>TTCCCUUUGACUUUCCGAUAC-5 | + | 57% | 0% |
| 9-2 | Random modification (2'-deoxy-2'-fluoro)<br>5-GGGAAACUGAAAGGCUAUGTT<br>TT**CCCUUU**GA**CUUUCC**GA**U**A**C**-5 | + | 6% | 47% |
| 9-3A | Specific modification (2'-deoxy-2'-fluoro)<br>5-GGGAAA**CU**GAAAGG**C**UA**U**GTT<br>TTCCCUUUGACUUUCCGAUAC-5 | ++ | 51% | 14% |
| 9-3B | Specific modification (2'-deoxy-2'-fluoro)<br>5-GGGAAACUGAAAGGC**U**AUGTT<br>TTCCCUUUGACUUUCCGAUAC-5 | ++ | 50% | 4% |

Table 10

| gene | Human BIC(NR_001458.3)(SEQ ID NO:1) SIRNA | Locus Stability | 1084-1102 BP Expression inhibition ratio | Growth inhibition ratio |
|---|---|---|---|---|
| 10-1 | Unmodified siRNA<br>5-GCGAGCAGAGAAUCUACCUTT<br>TTCGCUCGUCUCUUAGAUGGA-5 | + | 97% | 0% |
| 10-2 | Random modification (2'-O-methyl) | ++ | 65% | 41% |

(continued)

| gene | Human BIC(NR_001458.3)(SEQ ID NO:1) SIRNA | Locus Stability | 1084-1102 BP Expression inhibition ratio | Growth inhibition ratio |
|---|---|---|---|---|
| | 5-**G**C**GAG**C**AGAGAA**UCU**A**CCUTT<br>TTCGCUCGUCUCUUAGAUGGA-5 | | | |
| 103A | Specific modification (2'-O-methyl)<br>5-GCGAG**C**AGAGAAUC**U**A**C**CUTT<br>TTCGCUCG**U**CUCUUAGA**U**GGA-5 | ++ | 90% | 9% |
| 10-3B | Specific modification (2'-O-methyl)<br>5-GCGAGCAGAGAAUC**U**ACCUTT<br>TTCGCUCGUCUCUUAGA**U**GGA-5 | ++ | 90% | 5% |

Table 11

| gene | Human BIC(NR_001458.3)(SEQ ID NO:1) SIRNA | Locus Stability | 1088-1106 BP Expression inhibition ratio | Growth inhibition ratio |
|---|---|---|---|---|
| 11-1 | Unmodified siRNA<br>5-GCAGAGAAUCUACCUUUCCTT<br>TTCGUCUCUUAGAUGGAAAGG-5 | + | 79% | 0% |
| 11-2 | Random modification (2'-O-methyl)<br>5-G**C**AGAGAA**UCU**A**CCUUUCC**TT<br>TT**C**G**UCUCUU**AGA**U**GGAAAGG-5 | ++ | 13% | 38% |
| 11-3A | Specific modification (2'-O-methyl)<br>5-**G**C**AGAGAA**UCU**A**CCUUUCCTT<br>TTCGUCUCUUAGAUGGAAAGG-5 | +++ | 65% | 12% |
| 11-3B | Specific modification (2'-O-methyl)<br>5-G**C**AGAGAAUC**U**ACCUUUCCTT<br>TTCG**U**CUCUUAGA**U**GGAAAGG-5 | ++ | 75% | 5% |

Table 12

| gene | Human BIC(NR_001458.3)(SEQ ID NO:1) SIRNA | Locus Stability | 1099-1117 BP Expression inhibition ratio | Growth inhibition ratio |
|---|---|---|---|---|
| 12-1 | Unmodified siRNA | | | |

(continued)

| gene | Human BIC(NR_001458.3)(SEQ ID NO:1) SIRNA | Locus Stability | 1099-1117 BP Expression inhibition ratio | Growth inhibition ratio |
|---|---|---|---|---|
| | 5-ACCUUUCCACUUCUAAGCCTT<br>TTUGGAAAGGUGAAGAUUCGG-5 | + | 84% | 0% |
| 12-2 | Random modification (2'-O-methyl)<br>5-A**CCUUUCC**A**CUUCU**AAG**CC**TT<br>TTUGGAAAGGUGAAGAUUCGG-5 | ++ | 75% | 41% |
| 12-3A | Specific modification (2'-O-methyl)<br>5-ACCUUUCCACUUCUAAGCCTT<br>TT**U**GGAAAGG**U**GAAGA**UU**CGG-5 | ++ | 80% | 15% |
| 12-3B | Specific modification (2'-O-methyl)<br>5-ACCUUUC**C**ACUUC**U**AAGCCTT<br>TTUGGAAAGG**U**GAAGA**U**UCGG-5 | ++ | 75% | 6% |

Table 13

| gene | Human BIC(NR_001458.3)(SEQ ID NO:1) SIRNA | Locus Stability | 1111-1129 BP Expression inhibition ratio | Growth inhibition ratio |
|---|---|---|---|---|
| 13-1 | Unmodified siRNA<br>5-CUAAGCCUGUUUCUUCCUCTT<br>TTGAUUCGGACAAAGAAGGAG-5 | + | 90% | 0% |
| 13-2 | Random modification (2'-deoxy-2'-fluoro)<br>5-C**U**A**A**G**C**C**U**G**U**U**U**C**U**U**C**C**U**CTT<br>TTG**A**UUCGG**A**C**AAA**G**AA**GG**A**G-5 | ++ | 75% | 57% |
| 13-3A | Specific modification (2'-deoxy-2'-fluoro)<br>5-CU**AA**GCCUG**U**UUCUUCCUCTT<br>TTGA**U**UCGGACAAAGAAGGAG-5 | ++ | 85% | 12% |
| 13-3B | Specific modification (2'-deoxy-2'-fluoro)<br>5-C**U**AAGCCUGUUUCUUCCUCTT<br>TTGAUUCGGACAAAGAAGGAG-5 | ++ | 82% | 4% |

Table 14

| gene | Human KAZRIN(NM_201628)(SEQ ID NO:2) SIRNA | Locus Stability | 2456-2474 BP Expression inhibition ratio | Growth inhibition ratio |
|---|---|---|---|---|
| 14-1 | Unmodified siRNA<br>5-GAAGAUAGGAAGGGGCUUCTT<br>TTCUUCUAUCCUUCCCCGAAG-5 | + | 55% | 0% |
| 14-2 | Random modification (2'-deoxy-2'-fluoro)<br>5-GAAGAUAGGAAGGGGCUUCTT<br>TT**CUUCU**A**UCCUUCCCC**GAAG-5 | ++ | 5% | 23% |
| 14-3A | Specific modification (2'-deoxy-2'-fluoro)<br>5-GAAGA**U**AGGAAGGGG**CUUC**TT<br>TTCUUCUAUCCUUCCCCGAAG-5 | ++ | 45% | 9% |
| 14-3B | Specific modification (2'-deoxy-2'-fluoro)<br>5-GAAGA**U**AGGAAGGGGCUUCTT<br>TTCUUCUA**U**CCUUCCCCGAAG-5 | ++ | 56% | 4% |

Table 15

| gene | Human KAZRIN(NM_201628)(SEQ ID NO:2) SIRNA | Locus Stability | 294-312 BP Expression inhibition ratio | Growth inhibition ratio |
|---|---|---|---|---|
| 15-1 | Unmodified siRNA<br>5-AAUAAGCAGCUCGCGCUCCTT<br>TTUUAUUCGUCGAGCGCGAGG-5 | + | 73% | 0% |
| 15-2 | Random modification (2'-O-methyl)<br>5-**AA**U**AAG**C**AG**CUC**G**C**G**CUCCTT<br>TTUUAUUCGUCGAGCGCGAGG-5 | ++ | 64% | 23% |
| 15-3A | Specific modification (2'-O-methyl)<br>5-AA**U**AAG**C**AGCUCGCGCUCCTT<br>TTUUA**U**UCG**U**CGAGCGCGAGG-5 | ++ | 68% | 12% |
| 15-3B | Specific modification (2'-O-methyl)<br>5-AA**U**AAGCAGCUCGCGCUCCTT<br>TTUUA**U**UCGUCGAGCGCGAGG-5 | ++ | 69% | 4% |

Table 16

| gene | Human KAZRIN(NM_201628)(SEQ ID NO:2) | Locus | 673-691 BP | |
|---|---|---|---|---|
| | SIRNA | Stability | Expression inhibition ratio | Growth inhibition ratio |
| 16-1 | Unmodified siRNA<br>5-UAGCCAGAGCCAAAGAAGCTT<br>TTAUCGGUCUCGGUUUCUUCG-5 | + | 93% | 0% |
| 16-2 | Random modification (2'-O-methyl)<br>5-**U**A**G**C**C**A**G**A**G**C**C**A**A**AG**A**AGCTT<br>TTA**U**CGG**U**C**U**CGG**UUU**C**UU**CG-5 | ++ | 75% | 57% |
| 16-3A | Specific modification (2'-O-methyl)<br>5-**U**AGC**C**AGAGC**C**AAAGAAGCTT<br>TTA**U**CGG**U**CUCGGUUUCUUCG-5 | ++ | 85% | 12% |
| 16-3B | Specific modification (2'-O-methyl)<br>5-**U**AGCCAGAGCCAAAGAAGCTT<br>TTA**U**CGGUCUCGGUUUCUUCG-5 | ++ | 82% | 4% |

Table 17

| gene | Human KAZRIN(NM_201628)(SEQ ID NO:2) | Locus | 1012-1030 BP | |
|---|---|---|---|---|
| | SIRNA | Stability | Expression inhibition ratio | Growth inhibition ratio |
| 17-1 | Unmodified siRNA<br>5-AACAGUCCUUAGCUACGCUTT<br>TTUUGUCAGGAAUCGAUGCGA-5 | + | 49% | 0% |
| 17-2 | Random modification (2'-O-methyl)<br>5-**A**A**C**A**G**U**C**C**U**UA**G**C**U**AC**G**CUTT<br>TTUUGUCAGGAAUCGAUGCGA-5 | + | 35% | 57% |
| 17-3A | Specific modification (2'-O-methyl)<br>5-AA**C**AGUCCU**U**AGC**U**ACGC**U**TT<br>TTUUG**U**CAGGAA**U**CGA**U**GCGA-5 | ++ | 85% | 12% |
| 17-3B | Specific modification (2'-O-methyl)<br>5-AACAGUCCU**U**AGC**U**ACGC**U**TT<br>TTUUGUCAGGAAUCGA**U**GCGA-5 | ++ | 82% | 4% |

Table 18

| gene | Human KAZRIN(NM_201628)(SEQ ID NO:2) SIRNA | Locus Stability | 1383-1401 BP Expression inhibition ratio | Growth inhibition ratio |
|---|---|---|---|---|
| 18-1 | Unmodified siRNA<br>5-CUAGAGGAUCUUGAAGACCTT<br>TTGAUCUCCUAGAACUUCUGG-5 | + | 72% | 0% |
| 18-2 | Random modification (2'-deoxy-2'-fluoro)<br>5-**CU**A**G**AG**G**A**UCUU**GA**A**GA**CC**TT<br>TTGA**UCUCCU**AGAA**CUUCU**GG-5 | ++ | 12% | 42% |
| 18-3A | Specific modification (2'-deoxy-2'-fluoro)<br>5-C**U**AGAGGAUCU**U**GAAGACCTT<br>TTGA**U**CUCCUAGAA**C**UUCUGG-5 | ++ | 67% | 12% |
| 18-3B | Specific modification (2'-deoxy-2'-fluoro)<br>5-CU**A**GAGGAUCUUGAAGACCTT<br>TTGA**U**CUCCUAGAACUUCUGG-5 | ++ | 82% | 4% |

Table 19

| gene | Human KAZRIN(NM_201628)(SEQ ID NO:2) SIRNA | Locus Stability | 2151-2169 BP Expression inhibition ratio | Growth inhibition ratio |
|---|---|---|---|---|
| 19-1 | Unmodified siRNA<br>5-AUCGACCUGAAGGAGUACGTT<br>TTUAGCUGGACUUCCUCAUGC-5 | + | 52% | 0% |
| 19-2 | Random modification (2'-deoxy-2'-fluoro)<br>5-**A**UC**GA**CCU**GAAGGAG**U**A**C**G**TT<br>TTU**AG**CU**GGA**CUUCCUC**A**U**G**C-5 | ++ | 21% | 49% |
| 19-3A | Specific modification (2'-deoxy-2'-fluoro)<br>5-AUCGACC**U**GAAGGAG**U**ACGTT<br>TTUAGCUGGA**C**UUCCUCA**U**GC-5 | ++ | 46% | 9% |
| 19-3B | Specific modification (2'-deoxy-2'-fluoro)<br>5-AUCGACCUGAAGGAG**U**ACGTT<br>TTUAGCUGGACUUCCUC**A**UGC-5 | ++ | 82% | 4% |

Table 20

| gene | Human KAZRIN(NM_201628)(SEQ ID NO:2) SIRNA | Locus Stability | 2459-2477 BP Expression inhibition ratio | Growth inhibition ratio |
|---|---|---|---|---|
| 20-1 | Unmodified siRNA<br>5-GAUAGGAAGGGGCUUCAGCTT<br>TTCUAUCCUUCCCCGAAGUCG-5 | + | 53% | 0% |
| 20-2 | Random modification (2'-O-methyl)<br>5-GAUAGGAAGGGGCUUCAGCTT<br>TT**CU**A**UCCUUCCCC**GAAG**UC**G-5 | ++ | 12% | 52% |
| 20-3A | Specific modification (2'-O-methyl)<br>5-GA**U**AGGAAGGG**G**CUU**C**AGCTT<br>TTCUA**U**CCUUCCCCGAAG**U**CG-5 | ++ | 42% | 16% |
| 20-3B | Specific modification (2'-O-methyl)<br>5-GA**U**AGGAAGGGGCUUCAGCTT<br>TTCU**AU**CCUUCCCCGAAGUCG-5 | ++ | 41% | 6% |

Table 21

| gene | Human KAZRIN(NM_201628)(SEQ ID NO:2) SIRNA | Locus Stability | 2935-2953 BP Expression inhibition ratio | Growth inhibition ratio |
|---|---|---|---|---|
| 21-1 | Unmodified siRNA<br>5-AGUCCCUGGAGCCUUAAACTT<br>TTUCAGGGACCUCGGAAUUUG-5 | + | 72% | 0% |
| 21-2 | Random modification (2'-O-methyl)<br>5-**A**G**U**C**C**C**U**G**G**A**G**C**C**UUA**A**A**C**TT<br>TTUCAGGGACCUCGGAAUUUG-5 | + | 68% | 41% |
| 21-3A | Specific modification (2'-O-methyl)<br>5-AGUCCC**U**GGAGCCU**U**AAACTT<br>TTUCAGGGA**C**CUCGGAA**U**UUG-5 | ++ | 65% | 12% |
| 21-3B | Specific modification (2'-O-methyl)<br>5-AGUCCCUGGAGCCU**UA**AACTT<br>TTUCAGGGACCUCGGA**A**UUUG-5 | ++ | 62% | 5% |

Table 22

| gene | Human KAZRIN(NM_201628)(SEQ ID NO:2) SIRNA | Locus Stability | 3327-3345 BP Expression inhibition ratio | Growth inhibition ratio |
|---|---|---|---|---|
| 22-1 | Unmodified siRNA<br>5-AGCCACAUUUCUAGGAGAUTT<br>TTUCGGUGUAAAGAUCCUCUA-5 | + | 39% | 0% |
| 22-2 | Random modification (2'-O-methyl)<br>5-**A**G**C**C**A**C**A**U**U**U**C**U**A**G**G**A**G**AUTT<br>TTUCGGUGUAAAGAUCCUCUA-5 | ++ | 19% | 52% |
| 22-3A | Specific modification (2'-O-methyl)<br>5-AGC**C**A**C**AUUUCUAGGAGAUTT<br>TTUCGG**U**G**U**AAAGA**U**CCUCUA-5 | ++ | 26% | 16% |
| 22-3B | Specific modification (2'-O-methyl)<br>5-AGC**C**ACAUUUCU**A**GGAGAUTT<br>TTUCGG**U**GUAAAGAUCCUCUA-5 | ++ | 29% | 4% |

Table 23

| gene | Human KAZRIN(NM_201628)(SEQ ID NO:2) SIRNA | Locus Stability | 3449-3467 BP Expression inhibition ratio | Growth inhibition ratio |
|---|---|---|---|---|
| 23-1 | Unmodified siRNA<br>5-GAUUCCAAACCCCACUAUCTT<br>TTCUAAGGUUUGGGGUGAUAG-5 | + | 73% | 0% |
| 23-2 | Random modification (2'-O-methyl)<br>5-**G**A**U**U**C**C**A**A**A**C**C**C**C**A**C**U**A**U**C**TT<br>TTCUAAGGUUUGGGGUGAUAG-5 | ++ | 54% | 57% |
| 23-3A | Specific modification (2'-O-methyl)<br>5-GAUUC**C**AAACCC**C**AC**U**AUCTT<br>TTCUAAGG**U**UUGGGGUGAUAG-5 | ++ | 67% | 13% |
| 23-3B | Specific modification (2'-O-methyl)<br>5-GAUUCCAAACCCCAC**U**AUCTT<br>TTCUAAGGUUUGGGGUG**A**UAG-5 | ++ | 69% | 5% |

Table 24

| gene | human KAZRIN(NM_201628)(SEQ ID NO:2) SIRNA | Locus Stability | 3751-3769 BP Expression inhibition ratio | Growth inhibition ratio |
|---|---|---|---|---|
| 24-1 | Unmodified siRNA<br>5-GAUACUUCUGGUUCCCUCCTT<br>TTCUAUGAAGACCAAGGGAGG-5 | + | 53% | 0% |
| 24-2 | Random modification (2'-O-methyl)<br>5-**G**A**U**A**C**U**U**C**U**G**G**U**U**C**C**C**U**C**C**TT<br>TTCUAUGAAGACCAAGGGAGG-5 | ++ | 45% | 60% |
| 24-3A | Specific modification (2'-O-methyl)<br>5-GA**U**ACUUC**U**GGUUCCCUCCTT<br>TTCUA**U**GAAGA**C**CAAGGGAGG-5 | ++ | 45% | 10% |
| 24-3B | Specific modification (2'-O-methyl)<br>5-GA**U**ACUUCUGGUUCCCUCCTT<br>TTCUA**U**GAAGACCAAGGGAGG-5 | ++ | 46% | 5% |

Table 25

| gene | Human KAZRIN(NM_201628)(SEQ ID NO:2) SIRNA | Locus Stability | 5508-5526 BP Expression inhibition ratio | Growth inhibition ratio |
|---|---|---|---|---|
| 25-1 | Unmodified siRNA<br>5-AGAGAAGGGCGUCUCUACATT<br>TTUCUCUUCCCGCAGAGAUGU-5 | + | 83% | 0% |
| 25-2 | Random modification (2'-deoxy-2'-fluoro)<br>5-**A**GA**G**A**A**G**GG**CG**U**CUC**U**A**C**ATT<br>TTUCUCUUCCCGCAGAGAUGU-5 | ++ | 75% | 49% |
| 25-3A | Specific modification (2'-deoxy-2'-fluoro)<br>5-AGAGAAGGG**C**GUCUC**U**A**C**ATT<br>TTUCUCUUCCCGCAGAGA**U**G**U**-5 | ++ | 71% | 16% |
| 25-3B | Specific modification (2'-deoxy-2'-fluoro)<br>5-AGAGAAGGGCGUCUCU**A**CATT<br>TTUCUCUUCCCGCAGAGA**U**GU-5 | ++ | 69% | 4% |

Table 26

| gene | Human KAZRIN(NM_201628)(SEQ ID NO:2) SIRNA | Locus Stability | 5939-5957 BP Expression inhibition ratio | Growth inhibition ratio |
|---|---|---|---|---|
| 26-1 | Unmodified siRNA<br>5-AGUUUCUAGGGCCGUGGCCTT<br>TTUCAAAGAUCCCGGCACCGG-5 | + | 90% | 0% |
| 26-2 | Random modification (2'-deoxy-2'-fluoro)<br>5-**A**G**U**U**U**C**U**A**G**G**GC**C**G**UG**G**CCTT<br>TTUCAAAGAUCCCGGCACCGG-5 | ++ | 75% | 61% |
| 26-3A | Specific modification (2'-deoxy-2'-fluoro)<br>5-AGUUUC**U**AGGGCCG**U**GGCCTT<br>TTUCAAAGA**U**CCC**G**GCA**C**CGG-5 | ++ | 85% | 13% |
| 26-3B | Specific modification (2'-deoxy-2'-fluoro)<br>5-AGUUUC**UA**GGGCCGUGGCCTT<br>TTUCAAAGA**U**CCCGGCACCGG-5 | ++ | 82% | 7% |

Table 27

| gene | Human CDKN1B(NM_004064)(SEQ ID NO:3) SIRNA | Locus Stability | 587-605 BP Expression inhibition ratio | Growth inhibition ratio |
|---|---|---|---|---|
| 27-1 | Unmodified siRNA<br>5-GAAGAGUUAACCCGGGACUTT<br>TTCUUCUCAAUUGGGCCCUGA-5 | + | 62% | 0% |
| 27-2 | Random modification (2'-O-methyl)<br>5-**G**A**A**G**A**G**U**U**A**A**C**C**C**G**G**G**A**CUTT<br>TTCUUCUCAAUUGGGCCCUGA-5 | ++ | 17% | 61% |
| 27-3A | Specific modification (2'-O-methyl)<br>5-GAAGAGU**U**AACCC**G**GG**A**CUTT<br>TTCUUCUCAA**U**UGGGCCCUGA-5 | ++ | 47% | 13% |
| 27-3B | Specific modification (2'-O-methyl)<br>5-GAAGAGU**U**AACCCGGGACUTT<br>TTCUUCUCAA**U**UGGGCCCUGA-5 | ++ | 51% | 6% |

Table 28

| gene | Human CDKN1B(NM_004064)(SEQ ID NO:3) SIRNA | Locus Stability | 808-826 BP Expression inhibition ratio | Growth inhibition ratio |
|---|---|---|---|---|
| 28-1 | Unmodified siRNA<br>5-CCGCCCGGCGGCGCCUUUATT<br>TTGGCGGGCCGCCGCGGAAAU-5 | + | 95% | 0% |
| 28-2 | Random modification (2'-O-methyl)<br>5-**C**C**G**C**C**C**G**G**C**G**G**C**G**C**C**U**U**U**A**TT<br>TTGGCGGGCCGCCGCGGAAAU-5 | ++ | 49% | 52% |
| 28-3A | Specific modification (2'-O-methyl)<br>5-CCGCCCGGCGGC**G**CCUU**UA**TT<br>TTGGCGGGCCGCCGCGGAA**AU**-5 | ++ | 85% | 11% |
| 28-3B | Specific modification (2'-O-methyl)<br>5-CCGCCCGGCGGCGCCUUU**A**TT<br>TTGGCGGGCCGCCGCGGAA**AU**-5 | ++ | 82% | 4% |

Table 29

| gene | Human CDKN1B(NM_004064)(SEQ ID NO:3) SIRNA | Locus Stability | 941-959 BP Expression inhibition ratio | Growth inhibition ratio |
|---|---|---|---|---|
| 29-1 | Unmodified siRNA<br>5-ACCGACGAUUCUUCUACUCTT<br>TTUGGCUGCUAAGAAGAUGAG-5 | + | 73% | 0% |
| 29-2 | Random modification (2'-O-methyl)<br>5-**A**C**C**G**A**C**G**A**U**U**C**U**U**C**U**A**C**U**C**TT<br>TTUGGCUGCUAAGAAGAUGAG-5 | ++ | 42% | 47% |
| 29-3A | Specific modification (2'-O-methyl)<br>5-ACCGACGAUUCUUCUACUCTT<br>TTUGGCUGCU**AA**GAAGA**U**GAG-5 | ++ | 67% | 9% |
| 29-3B | Specific modification (2'-O-methyl)<br>5-ACCGACGAUUCUUCUACUCTT<br>TTUGGCUGCUAAGAAGA**U**GAG-5 | ++ | 68% | 4% |

Table 30

| gene | Human CDKN1B(NM_004064)(SEQ ID NO:3) SIRNA | Locus Stability | 2262-2280 BP Expression inhibition ratio | Growth inhibition ratio |
|---|---|---|---|---|
| 30-1 | Unmodified siRNA<br>5-GGUAGAAUAGGUUUUCCCCTT<br>TTCCAUCUUAUCCAAAAGGGG-5 | + | 54% | 0% |
| 30-2 | Random modification (2'-O-methyl)<br>5-**G**G**U**A**G**A**A**U**A**G**G**U**U**U**U**C**C**C**C**TT<br>TTCCAUCUUAUCCAAAAGGGG-5 | ++ | 11% | 71% |
| 30-3A | Specific modification (2'-O-methyl)<br>5-GG**UA**GAA**UA**GGUUUUCCCCTT<br>TTCCA**U**CUUA**U**CCAAAAGGGG-5 | ++ | 45% | 10% |
| 30-3B | Specific modification (2'-O-methyl)<br>5-GG**U**AGAAUAGGUUUUCCCCTT<br>TTCCAUCUUA**U**CCAAAAGGGG-5 | ++ | 82% | 3% |

Table 31

| gene | Human CDKN1B(NM_004064)(SEQ ID NO:3) SIRNA | Locus Stability | 15-33 BP Expression inhibition ratio | Growth inhibition ratio |
|---|---|---|---|---|
| 31-1 | Unmodified siRNA<br>5-UCCCUUCCACCGCCAUAUUTT<br>TTAGGGAAGGUGGCGGUAUAA-5 | + | 58% | 0% |
| 31-2 | Random modification (2'-O-methyl)<br>5-**U**C**C**C**U**U**C**C**A**C**C**G**C**C**A**U**A**U**U**TT<br>TTAGGGAAGGUGGCGGUAUAA-5 | ++ | 11% | 59% |
| 31-3A | Specific modification (2'-O-methyl)<br>5-UCCCUUC**C**ACCGC**C**A**U**AUUTT<br>TTAGGGAAGG**U**GGCGG**U**A**U**AA-5 | ++ | 47% | 13% |
| 31-3B | Specific modification (2'-O-methyl)<br>5-UCCCUUCCACCGCCA**U**AUUTT<br>TTAGGGAAGGUGGCGGUA**U**AA-5 | ++ | 49% | 2% |

Table 32

| gene | Human CDKN1B(NM_004064)(SEQ ID NO:3) SIRNA | Locus Stability | 485-503 BP Expression inhibition ratio | Growth inhibition ratio |
|---|---|---|---|---|
| 32-1 | Unmodified siRNA<br>5-CGAGUGUCUAACGGGAGCCTT<br>TTGCUCACAGAUUGCCCUCGG-5 | + | 70% | 0% |
| 32-2 | Random modification (2'-O-methyl)<br>5-**C**G**A**G**U**G**U**C**U**A**A**C**G**G**G**A**G**C**C**TT<br>TTGCUCACAGAUUGCCCUCGG-5 | ++ | 25% | 56% |
| 32-3A | Specific modification (2'-O-methyl)<br>5-CGAG**U**GUC**U**AACGGGAGCCTT<br>TTGCUCA**C**AGA**U**UGCCCUCGG-5 | ++ | 65% | 9% |
| 32-3B | Specific modification (2'-O-methyl)<br>5-CGAGUGUCUAACGGGAGCCTT<br>TTGCUCACAGA**U**UGCCCUCGG-5 | ++ | 68% | 2% |

Table 33

| gene | Human CDKN1B(NM_004064)(SEQ ID NO:3) SIRNA | Locus Stability | 581-599 BP Expression inhibition ratio | Growth inhibition ratio |
|---|---|---|---|---|
| 33-1 | Unmodified siRNA<br>5-GACCACGAAGAGUUAACCCTT<br>TTCUGGUGCUUCUCAAUUGGG-5 | + | 91% | 0% |
| 33-2 | Random modification (2'-deoxy-2'-fluoro)<br>5-**G**A**C**C**A**C**G**A**A**G**A**G**U**U**A**C**C**C**C**TT<br>TTCUGGUGCUUCUCAAUUGGG-5 | ++ | 51% | 59% |
| 33-3A | Specific modification (2'-deoxy-2'-fluoro)<br>5-GAC**C**ACGAAGAGU**U**AACCCTT<br>TTCUGG**U**GCUUCUCAA**U**UGGG-5 | ++ | 85% | 13% |
| 33-3B | Specific modification (2'-deoxy-2'-fluoro)<br>5-GACCACGAAGAGU**U**AACCCTT<br>TTCUGGUGCUUCUCAA**U**UGGG-5 | ++ | 82% | 7% |

Table 34

| gene | Human CDKN1B(NM_004064)(SEQ ID NO:3) SIRNA | Locus Stability | 591-609 BP Expression inhibition ratio | Growth inhibition ratio |
|---|---|---|---|---|
| 34-1 | Unmodified siRNA<br>5-AGUUAACCCGGGACUUGGATT<br>TTUCAAUUGGGCCCUGAACCU-5 | + | 97% | 0% |
| 34-2 | Random modification (2'-deoxy-2'-fluoro)<br>5-**A**G**U**U**A**A**C**C**C**G**G**G**A**C**U**U**G**G**A**TT<br>TTUCAAUUGGGCCCUGAACCU-5 | ++ | 65% | 57% |
| 34-3A | Specific modification (2'-deoxy-2'-fluoro)<br>5-AGU**U**AACCCGGGACU**U**GGATT<br>TTUCAA**U**UGGGCCCUGAA**C**CU-5 | ++ | 85% | 9% |
| 34-3B | Specific modification (2'-deoxy-2'-fluoro)<br>5-AGU**U**AACCCGGGACU**U**GGATT<br>TTUCAA**U**UGGGCCCUGAA**C**CU-5 | ++ | 86% | 4% |

Table 35

| gene | Human CDKN1B(NM_004064)(SEQ ID NO:3) SIRNA | Locus Stability | 669-687 BP Expression inhibition ratio | Growth inhibition ratio |
|---|---|---|---|---|
| 35-1 | Unmodified siRNA<br>5-AUCACAAACCCCUAGAGGGTT<br>TTUAGUGUUUGGGGAUCUCCC-5 | + | 35% | 0% |
| 35-2 | Random modification (2'-O-methyl)<br>5-**A**U**C**A**C**A**A**A**C**C**C**C**U**A**G**A**G**G**G**TT<br>TTUAGUGUUUGGGGAUCUCCC-5 | ++ | 9% | 51% |
| 35-3A | Specific modification (2'-O-methyl)<br>5-AU**C**A**C**AAACCCC**U**AGAGGGTT<br>TTUAG**U**G**U**UUGGGGA**U**CUCCC-5 | ++ | 27% | 10% |
| 35-3B | Specific modification (2'-O-methyl)<br>5-AUCACAAACCCC**U**AGAGGGTT<br>TTUAGUGUUUGGGGA**U**CUCCC-5 | ++ | 29% | 4% |

Table 36

| gene | Human CDKN1B(NM_004064)(SEQ ID NO:3) SIRNA | Locus Stability | 674-692 BP Expression inhibition ratio | Growth inhibition ratio |
|---|---|---|---|---|
| 36-1 | Unmodified siRNA<br>5-AAACCCCUAGAGGGCAAGUTT<br>TTUUUGGGGAUCUCCCGUUCA-5 | + | 81% | 0% |
| 36-2 | Random modification (2'-O-methyl)<br>5-**A**A**A**C**C**C**C**U**A**G**A**G**G**G**C**A**A**G**U**TT<br>TTUUUGGGGAUCUCCCGUUCA-5 | ++ | 62% | 54% |
| 36-3A | Specific modification (2'-O-methyl)<br>5-AAACCCC**U**AGAGGG**C**AAGUTT<br>TTUUUGGGGA**U**CUCCCG**U**UCA-5 | ++ | 78% | 11% |
| 36-3B | Specific modification (2'-O-methyl)<br>5-AAACCCC**U**AGAGGGCAAGUTT<br>TTUUUGGGGA**U**CUCCCGUUCA-5 | ++ | 75% | 5% |

Table 37

| gene | Human CDKN1B(NM_004064)(SEQ ID NO:3) SIRNA | Locus Stability | 835-853 BP Expression inhibition ratio | Growth inhibition ratio |
|---|---|---|---|---|
| 37-1 | Unmodified siRNA<br>5-UCCGGCUAACUCUGAGGACTT<br>TTAGGCCGAUUGAGACUCCUG-5 | + | 95% | 0% |
| 37-2 | Random modification (2'-deoxy-2'-fluoro)<br>5-**U**C**C**G**G**C**U**A**A**C**U**C**U**G**A**G**G**A**C**TT<br>TTAGGCCGAUUGAGACUCCUG-5 | ++ | 41% | 61% |
| 37-3A | Specific modification (2'-deoxy-2'-fluoro)<br>5-UCCGGC**U**AACUC**U**GAGGACTT<br>TTAGGCCGA**U**UGAGA**C**UCCUG-5 | ++ | 85% | 9% |
| 37-3B | Specific modification (2'-deoxy-2'-fluoro)<br>5-UCCGGC**U**AACUCUGAGGACTT<br>TTAGGCCGA**U**UGAGACUCCUG-5 | ++ | 82% | 3% |

Table 38

| gene | Human CDKN1B(NM_004064)(SEQ ID NO:3) SIRNA | Locus Stability | 943-961 BP Expression inhibition ratio | Growth inhibition ratio |
|---|---|---|---|---|
| 38-1 | Unmodified siRNA<br>5-CGACGAUUCUUCUACUCAATT<br>TTGCUGCUAAGAAGAUGAGUU-5 | + | 72% | 0% |
| 38-2 | Random modification (2'-deoxy-2'-fluoro)<br>5-**C**G**A**C**G**A**U**U**C**U**U**C**U**A**C**U**C**A**A**TT<br>TTGCUGCUAAGAAGAUGAGUU-5 | ++ | 56% | 34% |
| 38-3A | Specific modification (2'-deoxy-2'-fluoro)<br>5-CGAC**G**AUUCUUC**U**ACUCAATT<br>TTGCUGCUAAGAAGA**U**GAG**U**U-5 | ++ | 62% | 9% |
| 38-3B | Specific modification (2'-deoxy-2'-fluoro)<br>5-CGACGAUUCUUC**U**ACUCAATT<br>TTGCUGCUAAGAAGA**U**GAGUU-5 | ++ | 67% | 4% |

Table 39

| gene | Human CDKN1B(NM_004064)(SEQ ID NO:3) SIRNA | Locus Stability | 1353-1371 BP Expression inhibition ratio | Growth inhibition ratio |
|---|---|---|---|---|
| 39-1 | Unmodified siRNA<br>5-UAAACUUUGGGGAAGGGAGTT<br>TTAUUUGAAACCCCUUCCCUC-5 | + | 73% | 0% |
| 39-2 | Random modification (2'-O-methyl)<br>5-**U**A**A**A**C**U**U**U**G**G**G**G**A**A**G**G**G**A**G**TT<br>TTAUUUGAAACCCCUUCCCUC-5 | ++ | 34% | 48% |
| 39-3A | Specific modification (2'-O-methyl)<br>5-**U**AAACUU**U**GGGGAAGGGAGTT<br>TTA**U**UUGAAA**C**CCCUUCCCUC-5 | ++ | 67% | 11% |
| 39-3B | Specific modification (2'-O-methyl)<br>5-**U**AAACUUUGGGGAAGGGAGTT<br>TTA**U**UUGAAACCCCUUCCCUC-5 | ++ | 72% | 3% |

Table 40

| gene | Human CDKN1B(NM_004064)(SEQ ID NO:3) | Locus | 1401-1419 BP | |
|---|---|---|---|---|
| | SIRNA | Stability | Expression inhibition ratio | Growth inhibition ratio |
| 40-1 | Unmodified siRNA<br>5-AGCGGGGUAUGAAGAGCUUTT<br>TTUCGCCCCAUACUUCUCGAA-5 | + | 95% | 0% |
| 40-2 | Random modification (2'-O-methyl)<br>5-**A**G**C**G**G**G**G**U**A**U**G**A**A**G**A**G**C**U**U**TT<br>TTUCGCCCCAUACUUCUCGAA-5 | ++ | 75% | 38% |
| 40-3A | Specific modification (2'-O-methyl)<br>5-AGCGGGG**U**A**U**GAAG**A**GCUUTT<br>TTUCGCCCCA**U**A**C**UUCUCGAA-5 | ++ | 85% | 13% |
| 40-3B | Specific modification (2'-O-methyl)<br>5-AGCGGGG**U**AUGAAGAGCUUTT<br>TTUCGCCCCA**U**ACUUCUCGAA-5 | ++ | 82% | 6% |

Table 41

| gene | Human CDKN1B(NM_004064)(SEQ ID NO:3) | Locus | 1711-1729 BP | |
|---|---|---|---|---|
| | SIRNA | Stability | Expression inhibition ratio | Growth inhibition ratio |
| 41-1 | Unmodified siRNA<br>5-CUCCCCAGUCUCUCUUAAATT<br>TTGAGGGGUCAGAGAGAAUUU-5 | + | 72% | 0% |
| 41-2 | Random modification (2'-O-methyl)<br>5-**C**U**C**C**C**C**A**G**U**C**U**C**U**C**U**U**A**A**A**TT<br>TTGAGGGGUCAGAGAGAAUUU-5 | ++ | 51% | 46% |
| 41-3A | Specific modification (2'-O-methyl)<br>5-CUC**C**C**C**AGU**C**UCUCU**U**AAATT<br>TTGAGGGG**U**CAGAGAGAA**U**UU-5 | ++ | 68% | 15% |
| 41-3B | Specific modification (2'-O-methyl)<br>5-CUCCCCAGUCUCUCUU**A**AATT<br>TTGAGGGGUCAGAGAGA**A**UUU-5 | ++ | 69% | 7% |

Table 42

| gene | chimpanzee SOD2(NM_001009022)(SEQ ID NO:4) | Locus | 108-126 BP | |
|---|---|---|---|---|
| | SIRNA | Stability | Expression inhibition ratio | Growth inhibition ratio |
| 42-1 | Unmodified siRNA<br>5-GAAUAACCUGAACGUCACCTT<br>TTCUUAUUGGACUUGCAGUGG-5 | + | 59% | 0% |
| 42-2 | Random modification (2'-O-methyl)<br>5-**G**A**A**U**A**A**C**C**U**G**A**A**C**G**U**C**A**C**C**TT<br>TTCUUAUUGGACUUGCAGUGG-5 | ++ | 17% | 49% |
| 42-3A | Specific modification (2'-O-methyl)<br>5-GAA**U**AACC**U**GAACGU**C**ACCTT<br>TTCUUA**U**UGGA**C**UUGCAG**U**GG-5 | ++ | 51% | 12% |
| 42-3B | Specific modification (2'-O-methyl)<br>5-GAA**U**AACCUGAACGUCACCTT<br>TTCUUA**U**UGGACUUGCAGUGG-5 | ++ | 55% | 4% |

Table 43

| gene | Chimpanzee SOD2(NM_001009022)(SEQ ID NO:4) | Locus | 119-137 BP | |
|---|---|---|---|---|
| | SIRNA | Stability | Expression inhibition ratio | Growth inhibition ratio |
| 43-1 | Unmodified siRNA<br>5-ACGUCACCGAGGAGAAGUATT<br>TTUGCAGUGGCUCCUCUUCAU-5 | + | 65% | 0% |
| 43-2 | Random modification (2'-O-methyl)<br>5-**A**C**G**U**C**A**C**C**G**A**G**G**A**G**A**A**G**U**A**TT<br>TTUGCAGUGGCUCCUCUUCAU-5 | ++ | 36% | 46% |
| 43-3A | Specific modification (2'-O-methyl)<br>5-ACGU**C**ACCGAGGAGAAG**U**ATT<br>TTUGCAG**U**GGCUCCUCUUCAU-5 | ++ | 48% | 8% |
| 43-3B | Specific modification (2'-O-methyl)<br>5-ACGUCACCGAGGAGAAG**U**ATT<br>TTUGCAGUGGCUCCUCUUCAU-5 | ++ | 69% | 2% |

Table 44

| gene | Chimpanzee SOD2(NM_001009022)(SEQ ID NO:4) | Locus | 131-149 BP | |
|---|---|---|---|---|
| | SIRNA | Stability | Expression inhibition ratio | Growth inhibition ratio |
| 44-1 | Unmodified siRNA<br>5-AGAAGUACCAGGAGGCGUUTT<br>TTUCUUCAUGGUCCUCCGCAA-5 | + | 75% | 0% |
| 44-2 | Random modification (2'-O-methyl)<br>5-**A**G**A**A**G**U**A**C**C**A**G**G**A**G**G**C**G**U**U**TT<br>TTUCUUCAUGGUCCUCCGCAA-5 | ++ | 24% | 39% |
| 44-3A | Specific modification (2'-O-methyl)<br>5-AGAAG**U**AC**C**AGGAG**G**CGUUTT<br>TTUCUUCA**U**GG**U**CCUCCGCAA-5 | ++ | 68% | 9% |
| 44-3B | Specific modification (2'-O-methyl)<br>5-AGAAG**U**ACCAGGAGGCGUUTT<br>TTUCUUCA**U**GGUCCUCCGCAA-5 | ++ | 69% | 3% |

Table 45

| gene | Chimpanzee SOD2(NM_001009022)(SEQ ID NO:4) | Locus | 168-186 BP | |
|---|---|---|---|---|
| | SIRNA | Stability | Expression inhibition ratio | Growth inhibition ratio |
| 45-1 | Unmodified siRNA<br>5-AGCCCAGAUAGCUCUUCAGTT<br>TTUCGGGUCUAUCGAGAAGUC-5 | + | 72% | 0% |
| 45-2 | Random modification (2'-deoxy-2'-fluoro)<br>5-**A**G**C**C**C**A**G**A**U**A**G**C**U**C**U**U**C**A**G**TT<br>TTUCGGGUCUAUCGAGAAGUC-5 | ++ | 35% | 46% |
| 45-3A | Specific modification (2'-deoxy-2'-fluoro)<br>5-AGCC**C**AGA**U**AGCUCUU**C**AGTT<br>TTUCGGG**U**CUA**U**CGAGAAG**U**C-5 | ++ | 61% | 12% |
| 45-3B | Specific modification (2'-deoxy-2'-fluoro)<br>5-AGCCCAGA**U**AGCUCUUCAGTT<br>TTUCGGGUCUA**U**CGAGAAGUC-5 | ++ | 69% | 5% |

Table 46

| gene | Chimpanzee SOD2(NM_001009022)(SEQ ID NO:4) | Locus | 240-258 BP | |
|---|---|---|---|---|
| | SIRNA | Stability | Expression inhibition ratio | Growth inhibition ratio |
| 46-1 | Unmodified siRNA<br>5-AAACCUCAGCCCUAACGGUTT<br>TTUUUGGAGUCGGGAUUGCCA-5 | + | 94% | 0% |
| 46-2 | Random modification (2'-O-methyl)<br>5-**A**A**A**C**C**U**C**A**G**C**C**C**U**A**A**C**G**G**U**TT<br>TTUUUGGAGUCGGGAUUGCCA-5 | ++ | 51% | 62% |
| 46-3A | Specific modification (2'-O-methyl)<br>5-AAAC**C**U**C**AGCCC**U**AACGGUTT<br>TTUUUGGAG**U**CGGGA**U**UGCCA-5 | ++ | 79% | 10% |
| 46-3B | Specific modification (2'-O-methyl)<br>5-AAACCUCAGCCC**UA**ACGGUTT<br>TTUUUGGAGUCGGGA**U**UGCCA-5 | ++ | 69% | 6% |

Table 47

| gene | Chimpanzee SOD2(NM_001009022)(SEQ ID NO:4) | Locus | 323-341 BP | |
|---|---|---|---|---|
| | SIRNA | Stability | Expression inhibition ratio | Growth inhibition ratio |
| 47-1 | Unmodified siRNA<br>5-UUAAGGAGAAGCUGACGGCTT<br>TTAAUUCCUCUUCGACUGCCG-5 | + | 73% | 0% |
| 47-2 | Random modification (2'-O-methyl)<br>5-**U**U**A**A**G**G**A**G**A**A**G**C**U**G**A**C**G**G**C**TT<br>TTAAUUCCUCUUCGACUGCCG-5 | ++ | 24% | 35% |
| 47-3A | Specific modification (2'-O-methyl)<br>5-U**U**AAGGAGAAGC**U**GACGGCTT<br>TTAA**U**UCCUCUUCGA**C**UGCCG-5 | ++ | 67% | 12% |
| 47-3B | Specific modification (2'-O-methyl)<br>5-U**U**AAGGAGAAGCUGACGGCTT<br>TTAA**U**UCCUCUUCGACUGCCG-5 | ++ | 69% | 3% |

Table 48

| gene | Chimpanzee SOD2(NM_001009022)(SEQ ID NO:4) SIRNA | Locus Stability | 391-409 BP Expression inhibition ratio | Growth inhibition ratio |
|---|---|---|---|---|
| 48-1 | Unmodified siRNA<br>5-AAGGAACGGGGACACUUACTT<br>TTUUCCUUGCCCCUGUGAAUG-5 | + | 44% | 0% |
| 48-2 | Random modification (2'-O-methyl)<br>5-**A**A**G**G**A**A**C**G**G**G**G**A**C**A**C**U**U**A**C**TT<br>TTUUCCUUGCCCCUGUGAAUG-5 | ++ | 12% | 46% |
| 48-3A | Specific modification (2'-O-methyl)<br>5-AAGGAACGGGGA**C**ACU**U**ACTT<br>TTUUCCUUGCCCCUG**U**GAA**U**G-5 | ++ | 35% | 15% |
| 48-3B | Specific modification (2'-O-methyl)<br>5-AAGGAACGGGGACACU**U**ACTT<br>TTUUCCUUGCCCCUGUGAA**U**G-5 | ++ | 38% | 6% |

Table 49

| gene | Chimpanzee SOD2(NM_001009022)(SEQ ID NO:4) SIRNA | Locus Stability | 486-504 BP Expression inhibition ratio | Growth inhibition ratio |
|---|---|---|---|---|
| 49-1 | Unmodified siRNA<br>5-GGAGCACGCUUACUACCUUTT<br>TTCCUCGUGCGAAUGAUGGAA-5 | + | 77% | 0% |
| 49-2 | Random modification (2'-O-methyl)<br>5-**G**G**A**G**C**A**C**G**C**U**U**A**C**U**A**C**C**U**U**TT<br>TTCCUCGUGCGAAUGAUGGAA-5 | ++ | 51% | 38% |
| 49-3A | Specific modification (2'-O-methyl)<br>5-GGAG**C**ACGCU**U**AC**U**ACCUUTT<br>TTCCUCG**U**GCGAA**U**GA**U**GGAA-5 | ++ | 68% | 13% |
| 49-3B | Specific modification (2'-O-methyl)<br>5-GGAGCACGCU**U**AC**U**ACCUUTT<br>TTCCUCGUGCGAA**U**GA**U**GGAA-5 | ++ | 69% | 7% |

Table 50

| gene | Rhesus monkey RECEPTOR(XM_001111972) (SEQ ID NO: 5) | Locus | 168-186 BP | |
|---|---|---|---|---|
| | SIRNA | Stability | Expression inhibition ratio | Growth inhibition ratio |
| 50-1 | Unmodified siRNA<br>5-GGCGGGCUCUUUCCUAUUCTT<br>TTCCGCCCGAGAAAGGAUAAG-5 | + | 77% | 0% |
| 50-2 | Random modification (2'-O-methyl)<br>5-**G**G**C**G**G**G**C**U**C**U**U**U**C**C**U**A**U**U**C**TT<br>TTCCGCCCGAGAAAGGAUAAG-5 | ++ | 51% | 43% |
| 50-3A | Specific modification (2'-O-methyl)<br>5-GGCGG**G**C**U**CUUUCC**U**AUUCTT<br>TTCCGCCCGAGAAAGGA**U**AAG-5 | ++ | 68% | 8% |
| 50-3B | Specific modification (2'-O-methyl)<br>5-GGCGGGCUCUUUCC**U**AUUCTT<br>TTCCGCCCGAGAAAGGA**U**AAG-5 | ++ | 69% | 5% |

Table 51

| gene | Rhesus monkey RECEPTOR(XM_001111972) (SEQ ID NO:5) | Locus | 2006-2024 BP | |
|---|---|---|---|---|
| | SIRNA | Stability | Expression inhibition ratio | Growth inhibition ratio |
| 51-1 | Unmodified siRNA<br>5-CCGAGAGCUCUCCUACCUUTT<br>TTGGCUCUCGAGAGGAUGGAA-5 | + | 80% | 0% |
| 51-2 | Random modification (2'-O-methyl)<br>5-**C**C**G**A**G**A**G**C**U**C**U**C**C**U**A**C**C**U**U**TT<br>TTGGCUCUCGAGAGGAUGGAA-5 | ++ | 34% | 43% |
| 51-3A | Specific modification (2'-O-methyl)<br>5-CCGAGAG**C**UC**U**CC**U**ACCUUTT<br>TTGGCUCUCGAGAGGA**U**GGAA-5 | ++ | 72% | 12% |
| 51-3B | Specific modification (2'-O-methyl)<br>5-CCGAGAGCUCUCC**U**ACCUUTT<br>TTGGCUCUCGAGAGGA**U**GGAA-5 | ++ | 75% | 5% |

Table 52

| gene | Rhesus monkey RECEPTOR(XM_001111972) (SEQ ID NO:5) | Locus | 2013-2031 BP | |
|---|---|---|---|---|
| | SIRNA | Stability | Expression inhibition ratio | Growth inhibition ratio |
| 52-1 | Unmodified siRNA<br>5-CUCUCCUACCUUCUCCUCUTT<br>TTGAGAGGAUGGAAGAGGAGA-5 | + | 83% | 0% |
| 52-2 | Random modification (2'-deoxy-2'-fluoro)<br>5-**C**U**C**U**C**C**U**A**C**C**U**U**C**U**C**C**U**C**U**TT<br>TTGAGAGGAUGGAAGAGGAGA-5 | ++ | 69% | 37% |
| 52-3A | Specific modification (2'-deoxy-2'-fluoro)<br>5-CUCUCC**U**ACCU**U**CUCC**U**CUTT<br>TTGAGAGGA**U**GGAAGAGGAGA-5 | ++ | 73% | 6% |
| 52-3B | Specific modification (2'-deoxy-2'-fluoro)<br>5-CUCUCCU**A**CCUUCUCCUCUTT<br>TTGAGAGGAUGGAAGAGGAGA-5 | ++ | 78% | 1% |

Table 53

| gene | Rhesus monkey RECEPTOR(XM_001111972) (SEQ ID NO:5) | Locus | 2018-2036 BP | |
|---|---|---|---|---|
| | SIRNA | Stability | Expression inhibition ratio | Growth inhibition ratio |
| 53-1 | Unmodified siRNA<br>5-CUACCUUCUCCUCUUCUCCTT<br>TTGAUGGAAGAGGAGAAGAGG-5 | + | 75% | 0% |
| 53-2 | Random modification (2'-O-methyl)<br>5-**C**U**A**C**C**U**U**C**U**C**C**U**C**U**U**C**U**C**C**TT<br>TTGAUGGAAGAGGAGAAGAGG-5 | ++ | 27% | 38% |
| 53-3A | Specific modification (2'-O-methyl)<br>5-C**UA**CCUUCUCCUCUUCUCCTT<br>TTG**AU**GGAAGAGGAGAAGAGG-5 | ++ | 68% | 5% |
| 53-3B | Specific modification (2'-O-methyl)<br>5-C**U**ACCUUCUCCUCUUCUCCTT<br>TTGA**U**GGAAGAGGAGAAGAGG-5 | ++ | 69% | 2% |

Table 54

| gene | Rhesus monkey RECEPTOR(XM_001111972) (SEQ ID NO: 5) | Locus | 13-31 BP | |
|---|---|---|---|---|
| | SIRNA | Stability | Expression inhibition ratio | Growth inhibition ratio |
| 54-1 | Unmodified siRNA<br>5-AGCCUCCAAACUCCUAGCUTT<br>TTUCGGAGGUUUGAGGAUCGA-5 | + | 43% | 0% |
| 54-2 | Random modification (2'-deoxy-2'-fluoro)<br>5-**A**G**C**C**U**C**C**A**A**A**C**U**C**C**U**A**G**C**U**TT<br>TTUCGGAGGUUUGAGGAUCGA-5 | ++ | 16% | 34% |
| 54-3A | Specific modification (2'-deoxy-2'-fluoro)<br>5-AGCCUC**C**AAACUCC**U**AGCUTT<br>TTUCGGAGG**U**UUGAGGA**U**CGA-5 | ++ | 32% | 9% |
| 54-3B | Specific modification (2'-deoxy-2'-fluoro)<br>5-AGCCUCCAAACUCC**U**AGCUTT<br>TTUCGGAGGUUUGAGGA**U**CGA-5 | ++ | 69% | 3% |

Table 55

| gene | Rhesus monkey RECEPTOR(XM_001111972) (SEQ ID NO:5) | Locus | 24-42 BP | |
|---|---|---|---|---|
| | SIRNA | Stability | Expression inhibition ratio | Growth inhibition ratio |
| 55-1 | Unmodified siRNA<br>5-UCCUAGCUGUCUUGUCCCUTT<br>TTAGGAUCGACAGAACAGGGA-5 | + | 75% | 0% |
| 55-2 | Random modification (2'-O-methyl)<br>5-**U**C**C**U**A**G**C**U**G**U**C**U**U**G**U**C**C**C**U**TT<br>TTAGGAUCGACAGAACAGGGA-5 | ++ | 27% | 33% |
| 55-3A | Specific modification (2'-O-methyl)<br>5-UCC**U**AGC**U**GUCU**U**GUCCCUTT<br>TTAGGA**U**CGA**C**AGAA**C**AGGGA-5 | ++ | 68% | 8% |
| 55-3B | Specific modification (2'-O-methyl) | | | |

(continued)

| gene | Rhesus monkey RECEPTOR(XM_001111972) (SEQ ID NO:5) | Locus | 24-42 BP | |
|---|---|---|---|---|
| | SIRNA | Stability | Expression inhibition ratio | Growth inhibition ratio |
| | 5-UCC**U**AGCUGUCUUGUCCCUTT<br>TTAGGA**U**CGACAGAACAGGGA-5 | ++ | 71% | 1% |

Table 56

| gene | Rhesus monkey RECEPTOR(XM_001111972) (SEQ ID NO:5) | Locus | 162-180 BP | |
|---|---|---|---|---|
| | SIRNA | Stability | Expression inhibition ratio | Growth inhibition ratio |
| 56-1 | Unmodified siRNA<br>5-AUACUUGGCGGGCUCUUUCTT<br>TTUAUGAACCGCCCGAGAAAG-5 | + | 53% | 0% |
| 56-2 | Random modification (2'-O-methyl)<br>5-**A**U**A**C**U**U**G**G**C**G**G**G**C**U**C**U**U**U**C**TT<br>TTUAUGAACCGCCCGAGAAAG-5 | ++ | 19% | 35% |
| 56-3A | Specific modification (2'-O-methyl)<br>5-A**U**ACU**U**GGC**G**GGCUCUUUCTT<br>TTUA**U**GAA**C**CGCCCGAGAAAG-5 | ++ | 42% | 6% |
| 56-3B | Specific modification (2'-O-methyl)<br>5-A**U**ACUUGGCGGGCUCUUUCTT<br>TTUAUGAACCGCCCGAGAAAG-5 | ++ | 48% | 1% |

Table 57

| gene | Rhesus monkey RECEPTOR(XM_001111972) (SEQ ID NO:5) | Locus | 536-554 BP | |
|---|---|---|---|---|
| | SIRNA | Stability | Expression inhibition ratio | Growth inhibition ratio |
| 57-1 | Unmodified siRNA<br>5-GGGGCUCUUCUACAUUCCCTT<br>TTCCCCGAGAAGAUGUAAGGG-5 | + | 77% | 0% |
| 57-2 | Random modification (2'-O-methyl)<br>5-**G**G**G**G**C**U**C**U**U**C**U**A**C**A**U**U**C**C**C**TT<br>TTCCCCGAGAAGAUGUAAGGG-5 | ++ | 51% | 34% |

(continued)

| gene | Rhesus monkey RECEPTOR(XM_001111972) (SEQ ID NO:5) | Locus | 536-554 BP | |
|---|---|---|---|---|
| | SIRNA | Stability | Expression inhibition ratio | Growth inhibition ratio |
| 57-3A | Specific modification (2'-O-methyl)<br>5-GGGGCUCUUC**U**ACA**U**UCCCTT<br>TTCCCCGAGAAGA**U**G**U**AAGGG-5 | ++ | 68% | 5% |
| 57-3B | Specific modification (2'-O-methyl)<br>5-GGGGCUCUUCU**A**CAUUCCCTT<br>TTCCCCGAGAAGA**U**GUAAGGG-5 | ++ | 72% | 2% |

Table 58

| gene | Rhesus monkey RECEPTOR(XM_001111972) (SEQ ID NO:5) | Locus | 669-687 BP | |
|---|---|---|---|---|
| | SIRNA | Stability | Expression inhibition ratio | Growth inhibition ratio |
| 58-1 | Unmodified siRNA<br>5-GAGUAUUUCCGCUGGAACUTT<br>TTCUCAUAAAGGCGACCUUGA-5 | + | 87% | 0% |
| 58-2 | Random modification (2'-deoxy-2'-fluoro)<br>5-**G**A**G**U**A**U**U**U**C**C**G**C**U**G**G**A**A**C**U**TT<br>TTCUCAUAAAGGCGACCUUGA-5 | ++ | 21% | 37% |
| 58-3A | Specific modification (2'-deoxy-2'-fluoro)<br>5-GAG**U**AUUUCCGC**U**GGAACUTT<br>TTCUCA**U**AAAGGCGA**C**CUUGA-5 | ++ | 73% | 6% |
| 58-3B | Specific modification (2'-deoxy-2'-fluoro)<br>5-GAG**U**AUUUCCGCUGGAACUTT<br>TTCUCA**U**AAAGGCGACCUUGA-5 | ++ | 75% | 1% |

Table 59

| gene | Rhesus monkey RECEPTOR(XM_001111972) (SEQ ID NO:5) | Locus | 753-771 BP | |
|---|---|---|---|---|
| | SIRNA | Stability | Expression inhibition ratio | Growth inhibition ratio |
| 59-1 | Unmodified siRNA | | | |

(continued)

| gene | Rhesus monkey RECEPTOR(XM_001111972) (SEQ ID NO:5) | Locus | 753-771 BP | |
|---|---|---|---|---|
| | SIRNA | Stability | Expression inhibition ratio | Growth inhibition ratio |
| | 5-GCUGAGGAAAGGGACAUCUTT<br>TTCGACUCCUUUCCCUGUAGA-5 | + | 85% | 0% |
| 59-2 | Random modification (2'-O-methyl) | | | |
| | 5-**G**C**U**G**A**G**G**A**A**A**G**G**G**A**C**A**U**C**U**TT<br>TTCGACUCCUUUCCCUGUAGA-5 | ++ | 61% | 31% |
| 59-3A | Specific modification (2'-O-methyl) | | | |
| | 5-GC**U**GAGGAAAGG**G**A**C**AUCUTT<br>TTCGA**C**UCCUUUCCCUG**U**AGA-5 | ++ | 76% | 7% |
| 59-3B | Specific modification (2'-O-methyl) | | | |
| | 5-GCUGAGGAAAGGGACAUCUTT<br>TTCGACUCCUUUCCCUG**UA**GA-5 | ++ | 78% | 3% |

Table 60

| gene | Rhesus monkey RECEPTOR(XM_001111972) (SEQ ID NO:5) | Locus | 800-818 BP | |
|---|---|---|---|---|
| | SIRNA | Stability | Expression inhibition ratio | Growth inhibition ratio |
| 60-1 | Unmodified siRNA | | | |
| | 5-GUACUCUGAUGAGGAAGAGTT<br>TTCAUGAGACUACUCCUUCUC-5 | + | 75% | 0% |
| 60-2 | Random modification (2'-O-methyl) | | | |
| | 5-**G**U**A**C**U**C**U**G**A**U**G**A**G**G**A**A**G**A**G**TT<br>TTCAUGAGACUACUCCUUCUC-5 | ++ | 18% | 37% |
| 60-3A | Specific modification (2'-O-methyl) | | | |
| | 5-G**U**ACUC**U**GA**U**GAGGAAGAGTT<br>TTCA**U**GAGA**C**UA**C**UCCUUCUC-5 | ++ | 68% | 8% |
| 60-3B | Specific modification (2'-O-methyl) | | | |
| | 5-G**U**ACUCUGAUGAGGAAGAGTT<br>TTCAUGAGACUACUCCUUCUC-5 | ++ | 69% | 1% |

Table 61

| gene | Rhesus monkey RECEPTOR(XM_001111972) (SEQ ID NO:5) | Locus | 854-872 BP | |
|---|---|---|---|---|
| | SIRNA | Stability | Expression inhibition ratio | Growth inhibition ratio |
| 61-1 | Unmodified siRNA<br>5-GGCCAAAGUAAUCGUGGUUTT<br>TTCCGGUUUCAUUAGCACCAA-5 | + | 93% | 0% |
| 61-2 | Random modification (2'-O-methyl)<br>5-**G**G**C**C**A**A**A**G**U**A**A**U**C**G**U**G**G**U**U**TT<br>TTCCGGUUUCAUUAGCACCAA-5 | ++ | 37% | 31% |
| 61-3A | Specific modification (2'-O-methyl)<br>5-GGC**C**AAAG**U**AAUCG**U**GGUUTT<br>TTCCGG**U**UUCA**U**UAGCA**C**CAA-5 | ++ | 86% | 10% |
| 61-3B | Specific modification (2'-O-methyl)<br>5-GGCCAAAG**U**AAUCGUGGUUTT<br>TTCCGGUUUCA**U**UAGCACCAA-5 | ++ | 85% | 5% |

Table 62

| gene | Rhesus monkey RECEPTOR(XM_001111972) (SEQ ID NO:5) | Locus | 1602-1620 BP | |
|---|---|---|---|---|
| | SIRNA | Stability | Expression inhibition ratio | Growth inhibition ratio |
| 62-1 | Unmodified siRNA<br>5-GUCUAUGCCAAGAAGGGAGTT<br>TTCAGAUACGGUUCUUCCCUC-5 | + | 81% | 0% |
| 62-2 | Random modification (2'-O-methyl)<br>5-**G**U**C**U**A**U**G**C**C**A**A**G**A**A**G**G**G**A**G**TT<br>TTCAGAUACGGUUCUUCCCUC-5 | ++ | 24% | 31% |
| 62-3A | Specific modification (2'-O-methyl)<br>5-GUC**U**A**U**GC**C**AAGAAGGGAGTT<br>TTCAGA**U**A**C**GG**U**UCUUCCCUC-5 | ++ | 72% | 7% |
| 62-3B | Specific modification (2'-O-methyl)<br>5-GUC**U**AUGCCAAGAAGGGAGTT<br>TTCAGA**U**ACGGUUCUUCCCUC-5 | ++ | 76% | 2% |

Table 63

| gene | Rhesus monkey RECEPTOR(XM_001111972) (SEQ ID NO:5) | Locus | 2293-2311 BP | |
|---|---|---|---|---|
| | SIRNA | Stability | Expression inhibition ratio | Growth inhibition ratio |
| 63-1 | Unmodified siRNA<br>5-UCUACACCGCGCCCCCUCTT<br>TTAGAUGUGGCGCGGGGGAG-5 | + | 84% | 0% |
| 63-2 | Random modification (2'-O-methyl)<br>5-**U**C**U**A**C**A**C**C**G**C**G**C**C**C**C**CU**C**TT<br>TTAGAUGUGGCGCGGGGGAG-5 | ++ | 39% | 33% |
| 63-3A | Specific modification (2'-O-methyl)<br>5-UC**U**A**C**ACCG**C**GC**C**CCCUCTT<br>TTAGA**U**G**U**GGCGCGGGGGAG-5 | ++ | 73% | 6% |
| 63-3B | Specific modification (2'-O-methyl)<br>5-UC**U**ACACCGCGCCCCCCUCTT<br>TTAGA**U**GUGGCGCGGGGGAG-5 | ++ | 79% | 3% |

Table 64

| gene | Rhesus monkey RECEPTOR(XM_001111972) (SEQ ID NO:5) | Locus | 2302-2320 BP | |
|---|---|---|---|---|
| | SIRNA | Stability | Expression inhibition ratio | Growth inhibition ratio |
| 64-1 | Unmodified siRNA<br>5-CGCCCCCCUCAAGCUACCGTT<br>TTGCGGGGGGAGUUCGAUGGC-5 | + | 81% | 0% |
| 64-2 | Random modification (2'-O-methyl)<br>5-**C**G**C**C**C**C**C**C**U**C**A**A**G**C**U**A**C**C**G**TT<br>TTGCGGGGGGAGUUCGAUGGC-5 | ++ | 44% | 48% |
| 64-3A | Specific modification (2'-O-methyl)<br>5-CG**C**CC**C**CCU**C**AAGC**U**ACCGTT<br>TTGCGGGGGGAG**U**UCGA**U**GGC-5 | ++ | 72% | 9% |
| 64-3B | Specific modification (2'-O-methyl)<br>5-CGCCCCCCUCAAGC**U**ACCGTT<br>TTGCGGGGGGAGUUCGA**U**GGC-5 | ++ | 76% | 3% |

Table 65

| gene | Rhesus monkey RECEPTOR(XM_001111972) (SEQ ID NO: 5) | Locus | 2314-2332 BP | |
|---|---|---|---|---|
| | SIRNA | Stability | Expression inhibition ratio | Growth inhibition ratio |
| 65-1 | Unmodified siRNA<br>5-GCUACCGCAACCACGAGCUTT<br>TTCGAUGGCGUUGGUGCUCGA-5 | + | 47% | 0% |
| 65-2 | Random modification (2'-deoxy-2'-fluoro)<br>5-**G**C**U**A**C**C**G**C**A**A**C**C**A**C**G**A**G**C**U**TT<br>TTCGAUGGCGUUGGUGCUCGA-5 | ++ | 21% | 37% |
| 65-3A | Specific modification (2'-deoxy-2'-fluoro)<br>5-GC**U**ACCG**C**AAC**C**ACGAGCUTT<br>TTCGA**U**GGCG**U**UGG**U**GCUCGA-5 | ++ | 42% | 7% |
| 65-3B | Specific modification (2'-deoxy-2'-fluoro)<br>5-GC**U**ACCGCAACCACGAGCUTT<br>TTCGA**U**GGCGUUGGUGCUCGA-5 | ++ | 45% | 3% |

Table 66

| gene | Rhesus monkey RECEPTOR(XM_001111972) (SEQ ID NO:5) SIRNA | Locus Stability | 2522-2540 BP Expression inhibition ratio | Growth inhibition ratio |
|---|---|---|---|---|
| 66-1 | Unmodified siRNA<br>5-CUCCUUCAUUCCAGCCUAUTT<br>TTGAGGAAGUAAGGUCGGAUA-5 | + | 84% | 0% |
| 66-2 | Random modification (2'-O-methyl)<br>5-**C**U**C**C**U**U**C**A**U**U**C**C**A**G**C**C**U**A**U**TT<br>TTGAGGAAGUAAGGUCGGAUA-5 | ++ | 51% | 31% |
| 66-3A | Specific modification (2'-O-methyl)<br>5-CUCCUU**C**AUUC**C**AGCC**U**AUTT<br>TTGAGGAAG**U**AAGG**U**CGGA**U**A-5 | ++ | 78% | 8% |
| 66-3B | Specific modification (2'-O-methyl)<br>5-CUCCUUCAUUCCAGCC**U**AUTT<br>TTGAGGAAGUAAGGUCGGA**U**A-5 | ++ | 81% | 4% |

Table 67

| gene | Rhesus monkey RECEPTOR(XM_001111972) (SEQ ID NO:5) | Locus | 2998-3016 BP | |
|---|---|---|---|---|
| | SIRNA | Stability | Expression inhibition ratio | Growth inhibition ratio |
| 67-1 | Unmodified siRNA<br>5-AGAAAAGCAGCGAUACGCUTT<br>TTCUUUUCGUCGCUAUGCGA-5 | + | 78% | 0% |
| 67-2 | Random modification (2'-O-methyl)<br>5-**A**G**A**A**A**A**G**C**A**G**C**G**A**U**A**C**G**C**U**TT<br>TTCUUUUCGUCGCUAUGCGA-5 | ++ | 34% | 37% |
| 67-3A | Specific modification (2'-O-methyl)<br>5-AGAAAAG**C**AGCGA**U**ACGCUTT<br>TTCUUUUCG**U**CGCUAUGCGA-5 | ++ | 68% | 8% |
| 67-3B | Specific modification (2'-O-methyl)<br>5-AGAAAAGCAGCGA**U**ACGCUTT<br>TTCUUUUCGUCGCUAUGCGA-5 | ++ | 69% | 1% |

Table 68

| gene | Dog B cell lymphoma(NM_001003016) (SEQ ID NO: 6) | Locus | 480-498 BP | |
|---|---|---|---|---|
| | SIRNA | Stability | Expression inhibition ratio | Growth inhibition ratio |
| 68-1 | Unmodified siRNA<br>5-GGAGCUAGACGGGUACGAGTT<br>TTCCUCGAUCUGCCCAUGCUC-5 | + | 93% | 0% |
| 68-2 | Random modification (2'-O-methyl)<br>5-**G**G**A**G**C**U**A**G**A**C**G**G**G**U**A**C**G**AGTT<br>TTCCUCGAUCUGCCCAUGCUC-5 | ++ | 75% | 51% |
| 68-3A | Specific modification (2'-O-methyl)<br>5-GGAGC**U**AGACGGG**U**ACGAGTT<br>TTCCUCGA**U**CUGCCCA**U**GCUC-5 | ++ | 82% | 9% |
| 68-3B | Specific modification (2'-O-methyl)<br>5-GGAGC**UA**GACGGG**U**ACGAGTT<br>TTCCUCGA**U**CUGCCCAUGCUC-5 | ++ | 81% | 10% |

Table 69

| gene | dog B cell lymphoma(NM_001003016) (SEQ ID NO:6) SIRNA | Locus Stability | 489-507 BP Expression inhibition ratio | Growth inhibition ratio |
|---|---|---|---|---|
| 69-1 | Unmodified siRNA<br>5-CGGGUACGAGCCGGAACCUTT<br>TTGCCCAUGCUCGGCCUUGGA-5 | ++ | 70% | 0% |
| 69-2 | Random modification (2'-deoxy-2'-fluoro)<br>5-**C**G**G**G**U**A**C**G**A**G**C**C**G**G**A**A**C**C**U**TT<br>TTGCCCAUGCUCGGCCUUGGA-5 | +++ | 61% | 43% |
| 69-3A | Specific modification (2'-deoxy-2'-fluoro)<br>5-CGGG**UA**CGAGCCGGAACCUTT<br>TTGCCCA**U**GCUCGGCCUUGGA-5 | +++ | 68% | 8% |
| 69-3B | Specific modification (2'-deoxy-2'-fluoro)<br>5-CGGGUACGAGCCGGAACCUTT<br>TTGCCCA**U**GCUCGGCCUUGGA-5 | +++ | 69% | 1% |

Table 70

| gene | dog B cell lymphoma(NM_001003016) (SEQ ID NO:6) SIRNA | Locus Stability | 578-596 BP Expression inhibition ratio | Growth inhibition ratio |
|---|---|---|---|---|
| 70-1 | Unmodified siRNA<br>5-ACGGCUCGCUACCCUCGACTT<br>TTUGCCGAGCGAUGGGAGCUG-5 | + | 87% | 0% |
| 70-2 | Random modification (2'-deoxy-2'-fluoro)<br>5-**A**C**G**G**C**U**C**G**C**U**A**C**C**C**U**C**G**ACTT<br>TTUGCCGAGCGAUGGGAGCUG-5 | +++ | 72% | 37% |
| 70-3A | Specific modification (2'-deoxy-2'-fluoro)<br>5-ACGGCUCGC**U**ACCCUCGACTT<br>TTUGCCGAGCGA**U**GGGAGCUG-5 | +++ | 78% | 4% |
| 70-3B | Specific modification (2'-deoxy-2'-fluoro)<br>5-ACGGCUCGCU**A**CCCUCGACTT<br>TTUGCCGAGCGAUGGGAGCUG-5 | +++ | 83% | 1% |

Table 71

| gene | dog B cell lymphoma(NM_001003016) (SEQ ID NO:6) SIRNA | Locus Stability | 1071-1089 BP Expression inhibition ratio | Growth inhibition ratio |
|---|---|---|---|---|
| 71-1 | Unmodified siRNA<br>5-AGAGGACCUAGAAGGCGGCTT<br>TTUCUCCUGGAUCUUCCGCCG-5 | + | 97% | 0% |
| 71-2 | Random modification (2'-O-methyl)<br>5-AGAGGACC**U**AGAAGGCGGCTT<br>TT**U**C**U**CC**U**GGA**U**C**UU**CCGCCG-5 | ++ | 72% | 34% |
| 71-3A | Specific modification (2'-O-methyl)<br>5-AGAGGACC**U**AGAAGGCGGCTT<br>TTUCUCCUGGA**U**CUUCCGCCG-5 | ++ | 89% | 4% |
| 71-3B | Specific modification (2'-O-methyl)<br>5-AGAGGACCU**A**GAAGGCGGCTT<br>TTUCUCCUGGAUCUUCCGCCG-5 | ++ | 92% | 1% |

Table 72

| gene | dog B cell lymphoma(NM_001003016) (SEQ ID NO:6) SIRNA | Locus Stability | 21-39 BP Expression inhibition ratio | Growth inhibition ratio |
|---|---|---|---|---|
| 72-1 | Unmodified siRNA<br>5-AACUCUACUGUGGAGUCGGTT<br>TTUUGAGAUGACACCUCAGCC-5 | + | 77% | 0% |
| 72-2 | Random modification (2'-O-methyl)<br>5-AA**C**U**C**UA**C**UGUGGAGU**C**GGTT<br>TTUUGAGAUGA**C**A**CC**U**C**AG**CC**-5 | +++ | 57% | 32% |
| 72-3A | Specific modification (2'-O-methyl)<br>5-AACUC**U**AC**U**G**U**GGAGUCGGTT<br>TTUUGAGA**U**GACA**C**CUCAGCC-5 | +++ | 68% | 8% |
| 72-3B | Specific modification (2'-O-methyl)<br>5-AACUC**U**ACUGUGGAGUCGGTT<br>TTUUGAGA**U**GACACCUCAGCC-5 | +++ | 69% | 1% |

Table 73

| gene | dog B cell lymphoma(NM_001003016) (SEQ ID NO:6) SIRNA | Locus Stability | 124-142 BP Expression inhibition ratio | Growth inhibition ratio |
|---|---|---|---|---|
| 73-1 | Unmodified siRNA<br>5-AGAAACGCAGUAAUCCGGATT<br>TTUCUUUGCGUCAUUAGGCCU-5 | + | 86% | 0% |
| 73-2 | Random modification (2'-O-methyl)<br>5-AGAAA**C**G**C**AG**U**AA**UCC**GGATT<br>TT**UCUUU**G**C**G**UC**A**UU**AGG**CCU**-5 | +++ | 80% | 43% |
| 73-3A | Specific modification (2'-O-methyl)<br>5-AGAAACG**C**AG**U**AAUCCGGATT<br>TTUCUUUGCG**U**CA**U**UAGGCCU-5 | ++ | 81% | 6% |
| 73-3B | Specific modification (2'-O-methyl)<br>5-AGAAACGCAG**UA**AUCCGGATT<br>TTUCUUUGCGUCA**U**UAGGCCU-5 | ++ | 85% | 1% |

Table 74

| gene | dogB cell lymphoma(NM_001003016) (SEQ ID NO:6) SIRNA | Locus Stability | 132-150 BP Expression inhibition ratio | Growth inhibition ratio |
|---|---|---|---|---|
| 74-1 | Unmodified siRNA<br>5-AGUAAUCCGGACUCAACUCTT<br>TTUCAUUAGGCCUGAGUUGAG-5 | + | 72% | 0% |
| 74-2 | Random modification (2'-deoxy-2'-fluoro)<br>5-AG**U**AA**UCC**GGA**CUC**AA**CUC**TT<br>TTUCAUUAGGCCUGAGUUGAG-5 | ++ | 59% | 17% |
| 74-3A | Specific modification (2'-deoxy-2'-fluoro)<br>5-AG**U**AAUCCGGACU**C**AACUCTT<br>TTUCA**U**UAGGCCUGAG**U**UGAG-5 | ++ | 68% | 4% |
| 74-3B | Specific modification (2'-deoxy-2'-fluoro)<br>5-AG**U**AAUCCGGACUCAACUCTT<br>TTUCA**U**UAGGCCUGAGUUGAG-5 | ++ | 69% | 1% |

Table 75

| gene | dog B cell lymphoma(NM_001003016) (SEQ ID NO:6) SIRNA | Locus Stability | 616-634 BP Expression inhibition ratio | Growth inhibition ratio |
|---|---|---|---|---|
| 75-1 | Unmodified siRNA<br>5-GAGGAAGAUGAGUUGUACCTT<br>TTCUCCUUCUACUCAACAUGG-5 | + | 81% | 0% |
| 75-2 | Random modification (2'-O-methyl)<br>5-**GAGGAAGA**U**GAG**UU**G**U**A**CCTT<br>TTCUCCUUCUACUCAACAUGG-5 | ++ | 70% | 29% |
| 75-3A | Specific modification (2'-O-methyl)<br>5-GAGGAAGA**U**GAGU**U**G**U**ACCTT<br>TTCUCCUUCUA**C**UCAA**C**AUGG-5 | +++ | 76% | 7% |
| 75-3B | Specific modification (2'-O-methyl)<br>5-GAGGAAGA**U**GAGUUGUACCTT<br>TTCUCCUUCUA**C**UCAA**C**AUGG-5 | +++ | 77% | 2% |

Table 76

| gene | dog B cell lymphoma(NM_001003016) (SEQ ID NO:6) SIRNA | Locus Stability | 993-1011 BP Expression inhibition ratio | Growth inhibition ratio |
|---|---|---|---|---|
| 76-1 | Unmodified siRNA<br>5-AGAUGUUCUCGUAAGGACGTT<br>TTUCUACAAGAGCAUUCCUGC-5 | + | 62% | 0% |
| 76-2 | Random modification (2'-O-methyl)<br>5-A**G**A**U**G**UUCUC**G**U**AA**G**GA**C**GTT<br>TTUCUACAAGAGCAUUCCUGC-5 | +++ | 53% | 17% |
| 76-3A | Specific modification (2'-O-methyl)<br>5-AGA**U**GUUCUCG**U**AAGGACGTT<br>TTUCUA**C**AAGAGCA**U**UCCUGC-5 | +++ | 60% | 6% |
| 76-3B | Specific modification (2'-O-methyl) | | | |

(continued)

| gene | dog B cell lymphoma(NM_001003016) (SEQ ID NO:6) | Locus | 993-1011 BP | |
|---|---|---|---|---|
| | SIRNA | Stability | Expression inhibition ratio | Growth inhibition ratio |
| | 5-AGAUGUUCUCGUAAGGACGTT<br>TTUCUACAAGAGCA**U**UCCUGC-5 | +++ | 69% | 1% |

Table 77

| gene | dog B cell lymphoma(NM_001003016) (SEQ ID NO:6) | Locus | 999-1017 BP | |
|---|---|---|---|---|
| | SIRNA | Stability | Expression inhibition ratio | Growth inhibition ratio |
| 77-1 | Unmodified siRNA<br>5-UCUCGUAAGGACGAAACGATT<br>TTAGAGCAUUCCUGCUUUGCU-5 | + | 58% | 0% |
| 77-2 | Random modification (2'-O-methyl)<br>5-**U**C**U**C**G**U**A**A**G**G**A**C**G**A**A**A**C**GATT<br>TTAGAGCAUUCCUGCUUUGCU-5 | ++ | 34% | 21% |
| 77-3A | Specific modification (2'-O-methyl)<br>5-UCUCG**UA**AGGACGA**A**A**C**GATT<br>TTAGAGCA**U**UCCUGCUUUGCU-5 | ++ | 52% | 6% |
| 77-3B | Specific modification (2'-O-methyl)<br>5-UCUCGUAAGGACGAAACGATT<br>TTAGAGCA**U**UCCUGCUUUGCU-5 | ++ | 55% | 0% |

Table 78

| gene | dog B cell lymphoma(NM_001003016) (SEQ ID NO:6) | Locus | 1268-1286 BP | |
|---|---|---|---|---|
| | SIRNA | Stability | Expression inhibition ratio | Growth inhibition ratio |
| 78-1 | Unmodified siRNA<br>5-GCUGUAACCUCGGAGAGUUTT<br>TTCGACAUUGGAGCCUCUCAA-5 | ++ | 71% | 0% |
| 78-2 | Random modification (2'-deoxy-2'-fluoro)<br>5-**G**C**U**G**U**A**A**C**C**U**C**G**G**A**G**A**G**UUTT<br>TTCGACAUUGGAGCCUCUCAA-5 | +++ | 51% | 23% |
| 78-3A | Specific modification (2'-deoxy-2'-fluoro) | | | |

(continued)

| gene | dog B cell lymphoma(NM_001003016) (SEQ ID NO:6) | Locus | 1268-1286 BP | |
|---|---|---|---|---|
| | SIRNA | Stability | Expression inhibition ratio | Growth inhibition ratio |
| | 5-GC**U**G**U**AACCUCGGAGAGUUTT<br>TTCGA**C**A**U**UGGAGCCUCUCAA-5 | +++ | 62% | 4% |
| 78-3B | Specific modification (2'-deoxy-2'-fluoro) | | | |
| | 5-GCUG**U**AACCUCGGAGAGUUTT<br>TTCGACA**U**UGGAGCCUCUCAA-5 | +++ | 69% | 1% |

Table 79

| gene | dog B cell lymphoma(NM_001003016) (SEQ ID NO: 6) | Locus | 1275-1293 BP | |
|---|---|---|---|---|
| | SIRNA | Stability | Expression inhibition ratio | Growth inhibition ratio |
| 79-1 | Unmodified siRNA | | | |
| | 5-CCUCGGAGAGUUCUACUCUTT<br>TTGGAGCCUCUCAAGAUGAGA-5 | + | 87% | 0% |
| 79-2 | Random modification (2'-O-methyl) | | | |
| | 5-**C**C**U**C**G**G**A**G**A**G**U**U**C**U**A**C**U**CUTT<br>TTGGAGCCUCUCAAGAUGAGA-5 | ++ | 34% | 27% |
| 79-3A | Specific modification (2'-O-methyl) | | | |
| | 5-CCUCGGAGAGUUC**U**ACUCUTT<br>TTGGAGCCUCUCAAGA**U**GAGA-5 | ++ | 85% | 3% |
| 79-3B | Specific modification (2'-O-methyl) | | | |
| | 5-CCUCGGAGAGUUCUACUCUTT<br>TTGGAGCCUCUCAAGA**U**GAGA-5 | ++ | 85% | 0% |

Table 80

| gene | dog B cell lymphoma(NM_001003016) (SEQ ID NO:6) | Locus | 1308-1326 BP | |
|---|---|---|---|---|
| | SIRNA | Stability | Expression inhibition ratio | Growth inhibition ratio |
| 80-1 | Unmodified siRNA | | | |
| | 5-GCAAGUGGCAAGAGGAUUATT<br>TTCGUUCACCGUUCUCCUAAU-5 | + | 71% | 0% |
| 80-2 | Random modification (2'-O-methyl) | | | |

(continued)

| gene | dog B cell lymphoma(NM_001003016) (SEQ ID NO:6) | Locus | 1308-1326 BP | |
|---|---|---|---|---|
| | SIRNA | Stability | Expression inhibition ratio | Growth inhibition ratio |
| | 5-**G**C**A**A**G**U**G**G**C**A**A**G**A**G**G**A**U**UATT<br>TTCGUUCACCGUUCUCCUAAU-5 | ++ | 55% | 36% |
| 80-3A | Specific modification (2'-O-methyl)<br>5-G**C**AAGUGGCAAGAGGAU**U**ATT<br>TTCG**U**UCA**C**CG**U**UCUCCUAA**U**-5 | ++ | 68% | 8% |
| 80-3B | Specific modification (2'-O-methyl)<br>5-GCAAGUGGCAAGAGGAU**U**ATT<br>TTCGUUCACCGUUCUCCUAA**U**-5 | ++ | 69% | 1% |

Table 81

| gene | dog B cell lymphoma(NM_001003016) (SEQ ID NO:6) | Locus | 1347-1365 BP | |
|---|---|---|---|---|
| | SIRNA | Stability | Expression inhibition ratio | Growth inhibition ratio |
| 81-1 | Unmodified siRNA<br>5-AUGGGAGAAGUAGUCCCCCTT<br>TTUACCCUCUUCAUCAGGGGG-5 | + | 86% | 0% |
| 81-2 | Random modification (2'-O-methyl)<br>5-**A**U**G**G**G**A**G**A**A**G**U**A**G**U**C**C**C**CCTT<br>TTUACCCUCUUCAUCAGGGGG-5 | +++ | 71% | 21% |
| 81-3A | Specific modification (2'-O-methyl)<br>5-A**U**GGGAGAAG**U**AGUCCCCCTT<br>TTUA**C**CCUCUUCA**U**CAGGGGG-5 | +++ | 82% | 4% |
| 81-3B | Specific modification (2'-O-methyl)<br>5-AUGGGAGAAG**U**AGUCCCCCTT<br>TTUACCCUCUUCA**U**CAGGGGG-5 | +++ | 84% | 1% |

Table 82

| gene | dog B cell lymphoma(NM_001003016) (SEQ ID NO:6) | Locus | 1354-1372 BP | |
|---|---|---|---|---|
| | SIRNA | Stability | Expression inhibition ratio | Growth inhibition ratio |
| 82-1 | Unmodified siRNA | | | |

(continued)

| gene | dog B cell lymphoma(NM_001003016) (SEQ ID NO:6) SIRNA | Locus Stability | 1354-1372 BP Expression inhibition ratio | Growth inhibition ratio |
|---|---|---|---|---|
| | 5-AAGUAGUCCCCCUUGAAGATT<br>TTUUCAUCAGGGGGAACUUCU-5 | + | 75% | 0% |
| 82-2 | Random modification (2'-deoxy-2'-fluoro)<br>5-**A**A**G**U**A**G**U**C**C**C**C**C**U**U**G**A**A**GATT<br>TTUUCAUCAGGGGGAACUUCU-5 | ++ | 53% | 27% |
| 82-3A | Specific modification (2'-deoxy-2'-fluoro)<br>5-AAG**U**AGUCCC**C**CU**U**GAAGATT<br>TTUUCA**U**CAGGGGGAA**C**UUCU-5 | ++ | 64% | 6% |
| 82-3B | Specific modification (2'-deoxy-2'-fluoro)<br>5-AAGU**A**GUCCCCCUUGAAGATT<br>TTUUCA**U**CAGGGGGAACUUCU-5 | ++ | 69% | 1% |

Table 83

| gene | dog B cell lymphoma(NM_001003016) (SEQ ID NO:6) SIRNA | Locus Stability | 1461-1479 BP Expression inhibition ratio | Growth inhibition ratio |
|---|---|---|---|---|
| 83-1 | Unmodified siRNA<br>5-GGUAGGGUUGAAGAGACUUTT<br>TTCCAUCCCAACUUCUCUGAA-5 | + | 97% | 0% |
| 83-2 | Random modification (2'-O-methyl)<br>5-**G**G**U**A**G**G**G**U**U**G**A**A**G**A**G**A**C**UUTT<br>TTCC**A**UCC**C**AACUUCUCUGAA-5 | +++ | 58% | 27% |
| 83-3A | Specific modification (2'-O-methyl)<br>5-GG**U**AGGGU**U**GAAGAGACUUTT<br>TTCCA**U**CCCAA**C**UUCUCUGAA-5 | +++ | 94% | 3% |
| 83-3B | Specific modification (2'-O-methyl)<br>5-GGUAGGGUUGAAGAGACUUTT<br>TTCCA**U**CCCAACUUCUCUGAA-5 | +++ | 95% | 0% |

Table 84

| gene | rat(NM_001014070.1)(SEQ ID NO:7) | Locus | 623-641 BP | |
|---|---|---|---|---|
| | SIRNA | Stability | Expression inhibition ratio | Growth inhibition ratio |
| 84-1 | Unmodified siRNA<br>5-AGCGCUUAAAGGGCGAGAAGATT<br>TTUCGCGAAUUUCCCGCUCUUCU-5 | ++ | 86% | 0% |
| 84-2 | Random modification (2'-O-methyl)<br>5-**A**G**C**G**C**U**U**A**A**A**G**G**G**C**G**A**G**A**A**G**A**TT<br>TTUCGCGAAUUUCCCGCUCUUCU-5 | +++ | 49% | 25% |
| 84-3A | Specific modification (2'-O-methyl)<br>5-AGCGCU**U**AAAGGGC**G**AGA**A**GATT<br>TTUCGCGAA**U**UUCCCGCUCUUCU-5 | +++ | 85% | 7% |
| 84-3B | Specific modification (2'-O-methyl)<br>5-AGCGCU**U**AAAGGGCGAGAAGATT<br>TTUCGCGAA**U**UUCCCGCUCUUCU-5 | +++ | 85% | 1% |

Table 85

| gene | rat(NM_001014070.1)(SEQ ID NO:7) | Locus | 2541-2559 BP | |
|---|---|---|---|---|
| | SIRNA | Stability | Expression inhibition ratio | Growth inhibition ratio |
| 85-1 | Unmodified siRNA<br>5-GGCCUUUCUCUCUAGAUAUTT<br>TTCCGGAAAGAGAGAUCUAUA-5 | + | 91% | 0% |
| 85-2 | Random modification (2'-O-methyl)<br>5-**G**G**C**C**U**U**U**C**U**C**U**C**U**A**G**A**U**A**U**TT<br>TTCCG**G**AAAGAGAGAUCUAUA-5 | +++ | 80% | 31% |
| 85-3A | Specific modification (2'-O-methyl)<br>5-GGCCUUUCUCUC**U**AGA**U**AUTT<br>TTCCGGAAAGAGAGA**U**CUA**U**A-5 | +++ | 89% | 5% |
| 85-3B | Specific modification (2'-O-methyl)<br>5-GGCCUUUCUCUCUAGAUAUTT<br>TTCCGGAAAGAGAGA**U**CUA**U**A-5 | +++ | 90% | 1% |

Table 86

| gene | rat(NM_001014070.1)(SEQ ID NO:7) | Locus | 2785-2803 BP | |
|---|---|---|---|---|
| | SIRNA | Stability | Expression inhibition ratio | Growth inhibition ratio |
| 86-1 | Unmodified siRNA<br>5-GCCUCUCUAUCCUUAGCUUTT<br>TTCGGAGAGAUAGGAAUCGAA-5 | + | 49% | 0% |
| 86-2 | Random modification (2'-deoxy-2'-fluoro)<br>5-**G**C**C**U**C**U**C**U**A**U**C**C**U**U**A**G**C**UUTT<br>TTCGGAGAG**A**UAGG**A**AUCGAA-5 | +++ | 34% | 37% |
| 86-3A | Specific modification (2'-deoxy-2'-fluoro)<br>5-GCCU**C**UC**U**AUCCU**U**AGCUUTT<br>TTCGGAGAGA**U**AGGAA**U**CGAA-5 | +++ | 47% | 6% |
| 86-3B | Specific modification (2'-deoxy-2'-fluoro)<br>5-GCCUCUCUAUCCUUAGCUUTT<br>TTCGGAGAG**A**UAGGAA**U**CGAA-5 | +++ | 48% | 1% |

Table 87

| gene | rat(NM_001014070.1)(SEQ ID NO:7) | Locus | 487-505 BP | |
|---|---|---|---|---|
| | SIRNA | Stability | Expression inhibition ratio | Growth inhibition ratio |
| 87-1 | Unmodified siRNA<br>5-AAGGACCUAGAGGAGUCACTT<br>TTUUCCUGGAUCUCCUCAGUG-5 | + | 90% | 0% |
| 87-2 | Random modification (2'-deoxy-2'-fluoro)<br>5-**A**A**G**G**A**C**C**U**A**G**A**G**G**A**G**U**C**ACTT<br>TTUUCCUGGAUCUCCUCAGUG-5 | ++ | 31% | 25% |
| 87-3A | Specific modification (2'-deoxy-2'-fluoro)<br>5-AAGGACC**U**AGAGGAGU**C**ACTT<br>TTUUCCUGGA**U**CUCCUCAG**U**G-5 | ++ | 87% | 4% |
| 87-3B | Specific modification (2'-deoxy-2'-fluoro)<br>5-AAGGACC**U**AGAGGAGUCACTT<br>TTUUCCUGGA**U**CUCCUCAGUG-5 | ++ | 88% | 1% |

Table 88

| gene | rat(NM_001014070.1)(SEQ ID NO:7) SIRNA | Locus Stability | 614-632 BP Expression inhibition ratio | Growth inhibition ratio |
|---|---|---|---|---|
| 88-1 | Unmodified siRNA<br>5-CUGACCGGAAGCGCUUAAATT<br>TTGACUGGCCUUCGCGAAUUU-5 | + | 87% | 0% |
| 88-2 | Random modification (2'-O-methyl)<br>5-**C**U**G**A**C**C**G**G**A**A**G**C**G**C**U**U**A**AATT<br>TTGA**C**UGG**C**CUU**C**GCGAAUUU-5 | +++ | 27% | 37% |
| 88-3A | Specific modification (2'-O-methyl)<br>5-C**U**GACCGGAAGCGCU**U**AAATT<br>TTGA**C**UGGCCUUCGCGAA**U**UU-5 | +++ | 85% | 5% |
| 88-3B | Specific modification (2'-O-methyl)<br>5-CUGACCGGAAGCGCU**U**AAATT<br>TTGACUGGCCUUCGCGAA**U**UU-5 | +++ | 86% | 1% |

Table 89

| gene | rat(NM_001014070.1)(SEQ ID NO:7) SIRNA | Locus Stability | 628-646 BP Expression inhibition ratio | Growth inhibition ratio |
|---|---|---|---|---|
| 89-1 | Unmodified siRNA<br>5-UUAAAGGGCGAGAAGACCGTT<br>TTAAUUUCCCGCUCUUCUGGC-5 | ++ | 89% | 0% |
| 89-2 | Random modification (2'-O-methyl)<br>5-**U**U**A**A**A**G**G**G**C**G**A**G**A**A**G**A**C**C**G**TT<br>TTAAUUUCCCGCUCUUCUGGC-5 | +++ | 67% | 47% |
| 89-3A | Specific modification (2'-O-methyl)<br>5-U**UA**AAG**G**GCGAGAAGACCGTT<br>TTAA**U**UUCCCGCUCUUCUGGC-5 | +++ | 85% | 3% |
| 89-3B | Specific modification (2'-O-methyl)<br>5-U**UA**AAGGGCGAGAAGACCGTT<br>TTAA**U**UUCCCGCUCUUCUGGC-5 | +++ | 87% | 0% |

Table 90

| gene | rat(NM_001014070.1)(SEQ ID NO:7) SIRNA | Locus Stability | 1257-1275 BP Expression inhibition ratio | Growth inhibition ratio |
|---|---|---|---|---|
| 90-1 | Unmodified siRNA<br>5-GAAGAGCCUACACAACCCUTT<br>TTCUUCUCGGAUGUGUUGGGA-5 | + | 77% | 0% |
| 90-2 | Random modification (2'-O-methyl)<br>5-**G**A**A**G**A**G**C**C**U**A**C**A**C**A**A**C**C**C**U**TT<br>TT**C**U**U**C**U**C**G**G**A**U**G**UGUUGGGA-5 | ++ | 50% | 27% |
| 90-3A | Specific modification (2'-O-methyl)<br>5-GAAGAGCC**U**A**C**A**C**AACCCUTT<br>TTCUUCUCGGA**U**G**U**G**U**UGGGA-5 | ++ | 75% | 5% |
| 90-3B | Specific modification (2'-O-methyl)<br>5-GAAGAGCC**U**A**C**ACAACCCUTT<br>TTCUUCUCGGA**U**G**U**GUUGGGA-5 | ++ | 76% | 1% |

Table 91

| gene | rat(NM_001014070.1)(SEQ ID NO:7) SIRNA | Locus Stability | 1288-1306 BP Expression inhibition ratio | Growth inhibition ratio |
|---|---|---|---|---|
| 91-1 | Unmodified siRNA<br>5-CUAGAGGAUCUUGAAGACCTT<br>TTGAUCUCCUAGAACUUCUGG-5 | + | 83% | 0% |
| 91-2 | Random modification (2'-deoxy-2'-fluoro)<br>5-**C**U**A**G**A**G**G**A**U**C**U**U**G**A**A**G**A**CCTT<br>TTGAUCUCCUAGAACUUCUGG-5 | ++ | 61% | 25% |
| 91-3A | Specific modification (2'-deoxy-2'-fluoro)<br>5-C**U**AGAGG**A**UCU**U**GAAGACCTT<br>TTGA**U**CUCCUAGAACUUCUGG-5 | ++ | 78% | 6% |
| 91-3B | Specific modification (2'-deoxy-2'-fluoro)<br>5-C**U**AGAGGAUCUUGAAGACCTT<br>TTGAUCUCCUAGAACUUCUGG-5 | ++ | 81% | 1% |

Table 92

| gene | rat(NM_001014070.1)(SEQ ID NO:7) | Locus | 1427-1445 BP | |
|---|---|---|---|---|
| | SIRNA | Stability | Expression inhibition ratio | Growth inhibition ratio |
| 92-1 | Unmodified siRNA<br>5-ACAUAGAGGAAGACGCGGATT<br>TTUGUAUCUCCUUCUGCGCCU-5 | + | 91% | 0% |
| 92-2 | Random modification (2'-deoxy-2'-fluoro)<br>5-**A**C**A**U**A**G**A**G**G**A**A**GACGCGGATT<br>TTUG**U**A**U**C**U**C**C**U**U**C**U**G**C**G**C**C**U**-5 | +++ | 57% | 34% |
| 92-3A | Specific modification (2'-deoxy-2'-fluoro)<br>5-A**C**A**U**AG**A**GGAAGACGCGGATT<br>TTUG**U**A**U**CUCCUUCUGCGCCU-5 | +++ | 86% | 6% |
| 92-3B | Specific modification (2'-deoxy-2'-fluoro)<br>5-ACAUAGAGGAAGACGCGGATT<br>TTUGUA**U**CUCCUUCUGCGCCU-5 | +++ | 89% | 0% |

Table 93

| gene | rat(NM_001014070.1)(SEQ ID NO:7) | Locus | 2091-2109 BP | |
|---|---|---|---|---|
| | SIRNA | Stability | Expression inhibition ratio | Growth inhibition ratio |
| 93-1 | Unmodified siRNA<br>5-AUCGUCGUCAGGGUAAGGUTT<br>TTUAGCAGCAGUCCCAUUCCA-5 | + | 50% | 0% |
| 93-2 | Random modification (2'-O-methyl)<br>5-**A**U**C**G**U**C**G**U**C**A**G**G**G**U**A**A**G**G**U**TT<br>TT**U**A**G**C**A**GCAGUCCCAUUCCA-5 | ++ | 34% | 37% |
| 93-3A | Specific modification (2'-O-methyl)<br>5-AUCGUCGU**C**AGGG**U**AAGGUTT<br>TTUAGCAGCAG**U**CCCA**U**UCCA-5 | ++ | 47% | 4% |
| 93-3B | Specific modification (2'-O-methyl)<br>5-AUCGUCGUCAGGG**U**AAGGUTT<br>TTUAGCAGCAGUCCCA**U**UCCA-5 | ++ | 49% | 1% |

Table 94

| gene | rat(NM_001014070.1)(SEQ ID NO:7) SIRNA | Locus Stability | 2537-2555 BP Expression inhibition ratio | Growth inhibition ratio |
|---|---|---|---|---|
| 94-1 | Unmodified siRNA<br>5-CUCUGGCCUUUCUCUCUAGTT<br>TTGAGACCGGAAAGAGAGAUC-5 | + | 48% | 0% |
| 94-2 | Random modification (2'-O-methyl)<br>5-**C**U**C**U**G**G**C**C**U**U**U**C**U**C**U**C**U**A**G**TT<br>TTGAGACCGGAAAGAGAGAUC-5 | ++ | 24% | 22% |
| 94-3A | Specific modification (2'-O-methyl)<br>5-CUC**U**GGCCUUUCUCUC<u>**U**A</u>GTT<br>TTGAGA**C**CGGAAAGAGAG<u>AU</u>C-5 | ++ | 45% | 5% |
| 94-3B | Specific modification (2'-O-methyl)<br>5-CUCUGGCCUUUCUCUC<u>**U**A</u>GTT<br>TTGAGACCGGAAAGAGAG<u>AU</u>C-5 | ++ | 47% | 1% |

Table 95

| gene | rat(NM_001014070.1)(SEQ ID NO:7) SIRNA | Locus Stability | 2688-2706 BP Expression inhibition ratio | Growth inhibition ratio |
|---|---|---|---|---|
| 95-1 | Unmodified siRNA<br>5-GACAAGGGAAGGUUAGCCUTT<br>TTCUGUUCCCUUCCAAUCGGA-5 | + | 73% | 0% |
| 95-2 | Random modification (2'-O-methyl)<br>5-**G**A**C**A**A**G**G**G**A**A**G**G**U**U**A**G**C**C**U**TT<br>TTCUGUUCCCUUCCAAUCGGA-5 | +++ | 34% | 29% |
| 95-3A | Specific modification (2'-O-methyl)<br>5-GA**C**AAGGGAAGGU<u>**U**A</u>GCCUTT<br>TTCUG**U**UCCCUUCCA<u>A**U**</u>CGGA-5 | +++ | 68% | 6% |
| 95-3B | Specific modification (2'-O-methyl)<br>5-GACAAGGGAAGGU<u>**U**A</u>GCCUTT<br>TTCUGUUCCCUUCCA<u>A**U**</u>CGGA-5 | +++ | 72% | 1% |

Table 96

| gene | rat(NM_001014070.1)(SEQ ID NO:7) | Locus | 2988-2006 BP | |
|---|---|---|---|---|
| | SIRNA | Stability | Expression inhibition ratio | Growth inhibition ratio |
| 96-1 | Unmodified siRNA<br>5-AGACUAAGCCCAAAAGGGCTT<br>TTUCUGAUUCGGGUUUUCCCG-5 | + | 48% | 0% |
| 96-2 | Random modification (2'-deoxy-2'-fluoro)<br>5-**A**G**A**C**U**A**A**G**C**C**C**A**A**A**A**G**G**G**C**TT<br>TTUCUGAUUCGGGUUUUCCCG-5 | +++ | 26% | 21% |
| 96-3A | Specific modification (2'-deoxy-2'-fluoro)<br>5-AGAC**U**AAGCC**C**AAAAGGGCTT<br>TTUCUGA**U**UCGGG**U**UUUCCCG-5 | +++ | 45% | 5% |
| 96-3B | Specific modification (2'-deoxy-2'-fluoro)<br>5-AGAC**U**AAGCCCAAAAGGGCTT<br>TTUCUGA**U**UCGGGUUUUCCCG-5 | +++ | 47% | 1% |

Table 97

| gene | rat(NM_001014070.1)(SEQ ID NO:7) | Locus | 3132-3150 BP | |
|---|---|---|---|---|
| | SIRNA | Stability | Expression inhibition ratio | Growth inhibition ratio |
| 97-1 | Unmodified siRNA<br>5-CCUCCCCAUCCCCUUUUAATT<br>TTGGAGGGGUAGGGGAAAAUU-5 | + | 81% | 0% |
| 97-2 | Random modification (2'-O-methyl)<br>5-**C**C**U**C**C**C**C**A**U**C**C**C**C**U**U**U**U**A**A**TT<br>TT**G**G**A**G**G**G**G**UAGGGGAAAAUU-5 | ++ | 48% | 44% |
| 97-3A | Specific modification (2'-O-methyl)<br>5-CCUCCC**C**AUCCCCUUU**U**AATT<br>TTGGAGGGG**U**AGGGGAAA**A**UU-5 | ++ | 80% | 4% |
| 97-3B | Specific modification (2'-O-methyl)<br>5-CCUCCCCAUCCCCUUUUAATT<br>TTGGAGGGGUAGGGGAAA**A**UU-5 | ++ | 80% | 0% |

Table 98

| gene | rat(NM_001014070.1)(SEQ ID NO:7) SIRNA | Locus Stability | 3471-3489 BP Expression inhibition ratio | Growth inhibition ratio |
|---|---|---|---|---|
| 98-1 | Unmodified siRNA<br>5-AUCAGAGGGAGGUCUUAGATT<br>TTUAGUCUCCCUCCAGAAUCU-5 | + | 73% | 0% |
| 98-2 | Random modification (2'-O-methyl)<br>5-**A**U**C**A**G**A**G**G**G**A**G**G**U**C**U**U**A**G**A**TT<br>TT**U**A**G**U**C**UCCCUCCA**G**AAUCU-5 | ++ | 19% | 32% |
| 98-3A | Specific modification (2'-O-methyl)<br>5-AU**C**AGAGGGAGGUCU**U**AGATT<br>TTUAG**U**CUCCCUCCAGAA**U**CU-5 | ++ | 68% | 6% |
| 98-3B | Specific modification (2'-O-methyl)<br>5-AUCAGAGGGAGGUCU**U**AGATT<br>TTUAGUCUCCCUCCAGAA**U**CU-5 | ++ | 69% | 1% |

Table 99

| gene | rat(NM_001014070.1)(SEQ ID NO:7) SIRNA | Locus Stability | 3477-3495 BP Expression inhibition ratio | Growth inhibition ratio |
|---|---|---|---|---|
| 99-1 | Unmodified siRNA<br>5-GGGAGGUCUUAGAAAACACTT<br>TTCCCUCCAGAAUCUUUUGUG-5 | + | 84% | 0% |
| 99-2 | Random modification (2'-O-methyl)<br>5-**G**G**G**A**G**G**U**C**U**U**A**G**A**A**A**A**C**A**C**TT<br>TTCCCUCCAGAAUCUUUUGUG-5 | ++ | 17% | 31% |
| 99-3A | Specific modification (2'-O-methyl)<br>5-GGGAGGUCU**U**AGAAAA**C**ACTT<br>TTCCCUCCAGAA**U**CUUUUGUG-5 | ++ | 84% | 3% |
| 99-3B | Specific modification (2'-O-methyl)<br>5-GGGAGGUCUUAGAAAACACTT<br>TTCCCUCCAGAA**U**CUUUUGUG-5 | ++ | 82% | 0% |

Table 100

| gene | rat(NM_001014070.1)(SEQ ID NO:7) SIRNA | Locus Stability | 3613-3631 BP Expression inhibition ratio | Growth inhibition ratio |
|---|---|---|---|---|
| 100-1 | Unmodified siRNA<br>5-GGCUUAGUCACUCCUCUUCTT<br>TTCCGAAUCAGUGAGGAGAAG-5 | + | 71% | 0% |
| 100-2 | Random modification (2'-deoxy-2'-fluoro)<br>5-**G**G**C**U**U**A**G**U**C**A**C**U**C**C**U**C**U**U**C**TT<br>TTCCGAAUCAGUGAGGAGAAG-5 | ++ | 23% | 14% |
| 100-3A | Specific modification (2'-deoxy-2'-fluoro)<br>5-GGCU**U**AGU**C**ACUCCUCUUCTT<br>TTCCGAA**U**CAG**U**GAGGAGAAG-5 | ++ | 68% | 4% |
| 100-3B | Specific modification (2'-deoxy-2'-fluoro)<br>5-GGCU**U**AGUCACUCCUCUUCTT<br>TTCCGAA**U**CAGUGAGGAGAAG-5 | ++ | 69% | 1% |

Table 101

| gene | Rat BIRC5(NM_022274)(SEQ ID NO:8) SIRNA | Locus Stability | 42-60 BP Expression inhibition ratio | Growth inhibition ratio |
|---|---|---|---|---|
| 101-1 | Unmodified siRNA<br>5-ACCUUAAGGACCACCGGAUTT<br>TTUGGAAUUCCUGGUGGCCUA-5 | + | 77% | 0% |
| 101-2 | Random modification (2'-O-methyl)<br>5-**A**C**C**U**U**A**A**G**G**A**C**C**A**C**C**G**G**A**U**TT<br>TT**U**G**G**A**A**U**U**CCUGGUGG**C**CUA-5 | ++ | 52% | 24% |
| 101-3A | Specific modification (2'-O-methyl)<br>5-ACCU**U**AAGGAC**C**ACCGGAUTT<br>TTUGGAA**U**UCCUGG**U**GGCCUA-5 | ++ | 72% | 6% |
| 101-3B | Specific modification (2'-O-methyl)<br>5-ACCU**U**AAGGACCACCGGAUTT<br>TTUGGAA**U**UCCUGGUGGCCUA-5 | ++ | 74% | 1% |

Table 102

| gene | Rat BIRC5(NM_022274)(SEQ ID NO:8) SIRNA | Locus Stability | 56-74 BP Expression inhibition ratio | Growth inhibition ratio |
|---|---|---|---|---|
| 102-1 | Unmodified siRNA<br>5-CGGAUCUACACCUUCAAGATT<br>TTGCCUAGAUGUGGAAGUUCU-5 | + | 73% | 0% |
| 102-2 | Random modification (2'-O-methyl)<br>5-**C**G**G**A**U**C**U**A**C**A**C**C**U**U**C**A**A**G**A**TT<br>TT**G**C**C**U**A**G**A**UGUGGAAGUUCU-5 | ++ | 46% | 19% |
| 102-3A | Specific modification (2'-O-methyl)<br>5-CGGAUC**U**A**C**ACCUU**C**AAGATT<br>TTGCCUAGA**U**G**U**GGAAG**U**UCU-5 | ++ | 68% | 5% |
| 102-3B | Specific modification (2'-O-methyl)<br>5-CGGAUC**U**ACACCUUCAAGATT<br>TTGCCUAGA**U**GUGGAAGUUCU-5 | ++ | 69% | 1% |

Table 103

| gene | Rat BIRC5(NM_022274)(SEQ ID NO:8) SIRNA | Locus Stability | 145-163 BP Expression inhibition ratio | Growth inhibition ratio |
|---|---|---|---|---|
| 103-1 | Unmodified siRNA<br>5-UACCGAGAAUGAGCCUGAUTT<br>TTAUGGCUCUUACUCGGACUA-5 | + | 57% | 0% |
| 103-2 | Random modification (2'-O-methyl)<br>5-UACCGAGAAUG**A**G**C**C**U**G**A**UTT<br>TT**A**U**G**G**C**U**C**U**U**A**C**U**C**G**G**A**C**U**A**-5 | +++ | 23% | 27% |
| 103-3A | Specific modification (2'-O-methyl)<br>5-**U**ACCGAGAA**U**GAGCC**U**GAUTT<br>TTA**U**GGCUCUUA**C**UCGGA**C**UA-5 | +++ | 54% | 6% |
| 103-3B | Specific modification (2'-O-methyl)<br>5-**U**ACCGAGAAUGAGCCUGAUTT<br>TTA**U**GGCUCUUACUCGGACUA-5 | +++ | 55% | 1% |

Table 104

| gene | Rat BIRC5(NM_022274)(SEQ ID NO:8) SIRNA | Locus Stability | 183-201 BP Expression inhibition ratio | Growth inhibition ratio |
|---|---|---|---|---|
| 104-1 | Unmodified siRNA<br>5-GCUUUAAGGAACUGGAAGGTT<br>TTCGAAAUUCCUUGACCUUCC-5 | + | 38% | 0% |
| 104-2 | Random modification (2'-O-methyl)<br>5-**G**C**U**U**U**A**A**G**G**A**A**C**U**G**G**A**A**G**G**TT<br>TTCGAAAUUCC**U**U**G**A**C**C**U**U**C**C-5 | ++ | 25% | 19% |
| 104-3A | Specific modification (2'-O-methyl)<br>5-GCUU**U**AAGGAAC**U**GGAAGGTT<br>TTCGAAA**U**UCCUUGA**C**CUUCC-5 | ++ | 37% | 3% |
| 104-3B | Specific modification (2'-O-methyl)<br>5-GCUU**U**AAGGAACUGGAAGGTT<br>TTCGAAA**U**UCCUUGACCUUCC-5 | ++ | 37% | 0% |

Table 105

| gene | mice(NM_008960.2)(SEQ ID NO:9) SIRNA | Locus Stability | 247-265 BP Expression inhibition ratio | Growth inhibition ratio |
|---|---|---|---|---|
| 105-1 | Unmodified siRNA<br>5-UCCUCGUCGUCUGUUCUAATT<br>TTAGGAGCAGCAGACAAGAUU-5 | + | 86% | 0% |
| 105-2 | Random modification (2'-O-methyl)<br>5-**U**C**C**U**C**G**U**C**G**U**C**U**G**U**U**C**U**A**A**TT<br>TT**A**G**G**AGCAGCAGACA**A**G**A**UU-5 | +++ | 63% | 35% |
| 105-3A | Specific modification (2'-O-methyl)<br>5-UCCUCGU**C**GUC**U**GUUC**U**AATT<br>TTAGGAGCAGCAGA**C**AAGA**U**U-5 | +++ | 82% | 4% |
| 105-3B | Specific modification (2'-O-methyl)<br>5-UCCUCGUCGUCUGUUC**U**AATT<br>TTAGGAGCAGCAGACAAGA**U**U-5 | +++ | 84% | 1% |

Table 106

| gene | mice(NM_008960.2)(SEQ ID NO:9) SIRNA | Locus Stability | 260-278 BP Expression inhibition ratio | Growth inhibition ratio |
|---|---|---|---|---|
| 106-1 | Unmodified siRNA<br>5-UUCUAACCGGGCAGCUUCUTT<br>TTAAGAUUGGCCCGUCGAAGA-5 | ++ | 73% | 0% |
| 106-2 | Random modification (2'-deoxy-2'-fluoro)<br>5-**U**U**C**U**A**A**C**C**G**G**G**C**A**G**C**U**U**C**U**TT<br>TTAAGAUUGGCCCGUCGAAGA-5 | +++ | 61% | 37% |
| 106-3A | Specific modification (2'-deoxy-2'-fluoro)<br>5-UUC**U**AACCGGG**C**AGCUUCUTT<br>TTAAGA**U**UGGCCCG**U**CGAAGA-5 | +++ | 71% | 5% |
| 106-3B | Specific modification (2'-deoxy-2'-fluoro)<br>5-UUC**U**AACCGGGCAGCUUCUTT<br>TTAAGA**U**UGGCCCGUCGAAGA-5 | +++ | 72% | 1% |

Table 107

| gene | mice(NM_008960.2)(SEQ ID NO:9) SIRNA | Locus Stability | 920-938 BP Expression inhibition ratio | TGrowth inhibition ratio |
|---|---|---|---|---|
| 107-1 | Unmodified siRNA<br>5-GAGGAUGGAUUCGACUUAGTT<br>TTCUCCUACCUAAGCUGAAUC-5 | + | 95% | 0% |
| 107-2 | Random modification (2'-O-methyl)<br>5-**G**A**G**G**A**U**G**G**A**U**U**C**G**A**C**U**U**AGTT<br>TTCUCCUACC**U**AAGC**U**GAA**U**C-5 | ++ | 23% | 22% |
| 107-3A | Specific modification (2'-O-methyl)<br>5-GAGGA**U**GGAUUCG**A**CU**U**AGTT<br>TTCUCCUA**C**CUAAGCU**G**AA**U**C-5 | ++ | 91% | 6% |
| 107-3B | Specific modification (2'-O-methyl)<br>5-GAGGAUGGAUUCGACUUAGTT<br>TTCUCCUACCUAAGCUGAA**U**C-5 | ++ | 94% | 0% |

Table 108

| gene | mice(NM_008960.2)(SEQ ID NO:9) SIRNA | Locus Stability | 1240-1258 BP Expression inhibition ratio | Growth inhibition ratio |
|---|---|---|---|---|
| 108-1 | Unmodified siRNA<br>5-UAAAGCUGGAAAGGGACGGTT<br>TTAUUUCGACCUUUCCCUGCC-5 | + | 67% | 0% |
| 108-2 | Random modification (2'-O-methyl)<br>5-**U**A**A**A**G**C**U**G**G**A**A**A**G**G**G**A**C**G**G**TT<br>TT**A**UUUCG**A**CCUUUCCCUGCC-5 | ++ | 29% | 28% |
| 108-3A | Specific modification (2'-O-methyl)<br>5-**U**AAAGC**U**GGAAAGGGACGGTT<br>TTA**U**UUCGA**C**CUUUCCCUGCC-5 | ++ | 65% | 4% |
| 108-3B | Specific modification (2'-O-methyl)<br>5-**U**AAAGCUGGAAAGGGACGGTT<br>TTA**U**UUCGACCUUUCCCUGCC-5 | ++ | 67% | 1% |

Table 109

| gene | mice(NM_008960.2)(SEQ ID NO:9) SIRNA | Locus Stability | 1336-1354 BP Expression inhibition ratio | Growth inhibition ratio |
|---|---|---|---|---|
| 109-1 | Unmodified siRNA<br>5-GGAAGUAAGGACCAGAGACTT<br>TTCCUUCAUUCCUGGUCUCUG-5 | + | 45% | 0% |
| 109-2 | Random modification (2'-O-methyl)<br>5-**G**G**A**A**G**U**A**A**G**G**A**C**C**A**G**A**G**A**C**TT<br>TTCCUUCAUUCCU**GG**UCUCU**G**-5 | ++ | 23% | 27% |
| 109-3A | Specific modification (2'-O-methyl)<br>5-GGAAG**U**AAGGAC**C**AGAGACTT<br>TTCCUUCA**U**UCCUGG**U**CUCUG-5 | ++ | 41% | 4% |
| 109-3B | Specific modification (2'-O-methyl)<br>5-GGAAG**U**AAGGACCAGAGACTT<br>TTCCUUCA**U**UCCUGGUCUCUG-5 | ++ | 44% | 1% |

Table 110

| gene | mice(NM_008960.2)(SEQ ID NO:9) | Locus | 1862-1880 BP | |
|---|---|---|---|---|
| | SIRNA | Stability | Expression inhibition ratio | Growth inhibition ratio |
| 110-1 | Unmodified siRNA<br>5-AAGGCCAACCGAUACUUCUTT<br>TTUUCCGGUUGGCUAUGAAGA-5 | ++ | 70% | 0% |
| 110-2 | Random modification (2'-deoxy-2'-fluoro)<br>5-**A**A**G**G**C**C**A**A**C**C**G**A**U**A**C**U**U**C**U**TT<br>TT**UU**CCGG**UU**GGC**U**A**U**GAAGA-5 | +++ | 46% | 24% |
| 110-3A | Specific modification (2'-deoxy-2'-fluoro)<br>5-A**A**GGC**C**AACCGA**U**ACUUCUTT<br>TTUUCCGG**U**UGGCUA**U**GAAGA-5 | +++ | 68% | 5% |
| 110-3B | Specific modification (2'-deoxy-2'-fluoro)<br>5-AAGGCCAACCGA**U**ACUUCUTT<br>TTUUCCGGUUGGCUA**U**GAAGA-5 | +++ | 69% | 1% |

Table 111

| gene | mice(NM_008960.2)(SEQ ID NO:9) | Locus | 2489-2507 BP | |
|---|---|---|---|---|
| | SIRNA | Stability | Expression inhibition ratio | Growth inhibition ratio |
| 111-1 | Unmodified siRNA<br>5-GUUCACGUCCUACCCCUUUTT<br>TTCAAGUGCAGGAUGGGGAAA-5 | + | 67% | 0% |
| 111-2 | Random modification (2'-deoxy-2'-fluoro)<br>5-**G**U**U**C**A**C**G**U**C**C**U**A**C**C**C**C**U**U**U**TT<br>TT**C**AAGUG**C**AGGAUGGGGAAA-5 | ++ | 23% | 27% |
| 111-3A | Specific modification (2'-deoxy-2'-fluoro)<br>5-GUU**C**ACGUCC**U**ACCCCUUUTT<br>TTCAAG**U**GCAGGA**U**GGGGAAA-5 | ++ | 65% | 5% |
| 111-3B | Specific modification (2'-deoxy-2'-fluoro)<br>5-GUUCACGUCCUACCCCUUUTT<br>TTCAAGUGCAGGA**U**GGGGAAA-5 | ++ | 69% | 0% |

Table 112

| gene | mice(NM_008960.2)(SEQ ID NO:9) SIRNA | Locus Stability | 3572-3590 BP Expression inhibition ratio | Growth inhibition ratio |
|---|---|---|---|---|
| 112-1 | Unmodified siRNA<br>5-AGAGUUGGGAUUAGGGCUUTT<br>TTUCUCAACCCUAAUCCCGAA-5 | + | 71% | 0% |
| 112-2 | Random modification (2'-O-methyl)<br>5-AGAGUUGGGAUUAGGG**C**UUTT<br>TT**U**C**U**C**A**A**C**C**C**U**A**A**U**C**C**C**G**A**A**-5 | ++ | 45% | 19% |
| 112-3A | Specific modification (2'-O-methyl)<br>5-AGAGU**U**GGGAU**U**AGGGCUUTT<br>TTUCUCAA**C**CCUAA**U**CCCGAA-5 | ++ | 68% | 4% |
| 112-3B | Specific modification (2'-O-methyl)<br>5-AGAGUUGGGAUUAGGGCUUTT<br>TTUCUCAACCCUAA**U**CCCGAA-5 | ++ | 69% | 0% |

Table 113

| gene | mice(NM_008960.2)(SEQ ID NO:9) SIRNA | Locus Stability | 3694-3712 BP Expression inhibition ratio | Growth inhibition ratio |
|---|---|---|---|---|
| 113-1 | Unmodified siRNA<br>5-UUACAAAGGAUCUCCUCCCTT<br>TTAAUGUUUCCUAGAGGAGGG-5 | + | 81% | 0% |
| 113-2 | Random modification (2'-O-methyl)<br>5-**U**U**A**C**A**A**A**G**G**A**U**C**U**C**C**U**C**C**C**TT<br>TTAAUGUUU**CC**UAGAGGAGGG-5 | ++ | 57% | 31% |
| 113-3A | Specific modification (2'-O-methyl)<br>5-U**U**A**C**AAAGG**A**UCUCCUCCCTT<br>TTAA**U**G**U**UUCCU**A**GAGGAGGG-5 | ++ | 78% | 5% |
| 113-3B | Specific modification (2'-O-methyl)<br>5-U**U**ACAAAGGAUCUCCUCCCTT<br>TTAA**U**GUUUCCUAGAGGAGGG-5 | ++ | 69% | 1% |

Table 114

| gene | mice(NM_008960.2)(SEQ ID NO:9) SIRNA | Locus Stability | 5159-5177 BP Expression inhibition ratio | Growth inhibition ratio |
|---|---|---|---|---|
| 114-1 | Unmodified siRNA<br>5-AACAGCCUUACCCCGAUUCTT<br>TTUUGUCGGAAUGGGGCUAAG-5 | + | 70% | 0% |
| 114-2 | Random modification (2'-O-methyl)<br>5-**A**A**C**A**G**C**C**U**U**A**C**C**C**C**G**A**U**U**C**TT<br>TTUUGUCGG**AA**UGGGGCU**AA**G-5 | ++ | 32% | 24% |
| 114-3A | Specific modification (2'-O-methyl)<br>5-AA**C**AGCCU**U**ACCCCGAUUCTT<br>TTUUG**U**CGGAA**U**GGGGCUAAG-5 | ++ | 68% | 4% |
| 114-3B | Specific modification (2'-O-methyl)<br>5-AACAGCCUUACCCCGAUUCTT<br>TTUUGUCGGAA**U**GGGGCUAAG-5 | ++ | 69% | 0% |

Table 115

| gene | mice(NM_008960.2)(SEQ ID NO:9) SIRNA | Locus Stability | 5166-5184 BP Expression inhibition ratio | Growth inhibition ratio |
|---|---|---|---|---|
| 115-1 | Unmodified siRNA<br>5-UUACCCCGAUUCAGCCUCUTT<br>TTAAUGGGGCUAAGUCGGAGA-5 | + | 84% | 0% |
| 115-2 | Random modification (2'-deoxy-2'-fluoro)<br>5-**U**U**A**C**C**C**C**G**A**U**U**C**A**G**C**C**U**C**U**TT<br>TTAAUGGGGCUAAGUCGGAGA-5 | ++ | 67% | 24% |
| 115-3A | Specific modification (2'-deoxy-2'-fluoro)<br>5-U**U**ACCCCGAUU**C**AGCCUCUTT<br>TTAA**U**GGGGCUAAG**U**CGGAGA-5 | ++ | 82% | 3% |
| 115-3B | Specific modification (2'-deoxy-2'-fluoro)<br>5-U**U**ACCCCGAUUCAGCCUCUTT<br>TTA**A**UGGGGCUAAGUCGGAGA-5 | ++ | 69% | 1% |

Table 116

| gene | mice(NM_008960.2)(SEQ ID NO:9) SIRNA | Locus Stability | 5695-5713 BP Expression inhibition ratio | Growth inhibition ratio |
|---|---|---|---|---|
| 116-1 | Unmodified siRNA<br>5-UAACCCCAAGAACGGGCUUTT<br>TTAUUGGGGUUCUUGCCCGAA-5 | + | 75% | 0% |
| 116-2 | Random modification (2'-deoxy-2'-fluoro)<br>5-**U**A**A**C**C**C**C**A**A**G**A**A**C**G**G**G**C**U**U**TT<br>TT**A**UUGGGGUUCUUGCCCG**AA**-5 | ++ | 47% | 25% |
| 116-3A | Specific modification (2'-deoxy-2'-fluoro)<br>5-<u>**U**A</u>ACCC**C**AAGAA**C**GGGCUUTT<br>TT<u>A**U**</u>UGGGG**U**UCUU**G**CCCGAA-5 | ++ | 72% | 6% |
| 116-3B | Specific modification (2'-deoxy-2'-fluoro)<br>5-<u>**U**A</u>ACCCCAAGAACGGGCUUTT<br>TT<u>A**U**</u>UGGGGUUCUUGCCCGAA-5 | ++ | 73% | 1% |

Table 117

| gene | aspergillusAF293protein(XM_748400) (SEQ ID NO:10) SIRNA | Locus Stability | 499-517 BP Expression inhibition ratio | Growth inhibition ratio |
|---|---|---|---|---|
| 117-1 | Unmodified siRNA<br>5-CUAGUCGAAAGAAAACGGGTT<br>TTGAUCAGCUUUCUUUUGCCC-5 | + | 57% | 0% |
| 117-2 | Random modification (2'-O-methyl)<br>5-**C**U**A**G**U**C**G**A**A**A**G**A**A**A**A**C**G**G**G**TT<br>TTG**A**UC**A**GCUUUCUUUUGCCC-5 | ++ | 23% | 19% |
| 117-3A | Specific modification (2'-O-methyl)<br>5-C<u>**U**A</u>GUCGA**A**AGA**AA**ACGGGTT<br>TTG<u>A**U**</u>CAGCUUUCUUUUGCCC-5 | ++ | 55% | 6% |
| 117-3B | Specific modification (2'-O-methyl)<br>5-C<u>**U**A</u>GUCGAAAGAAAACGGGTT<br>TTG<u>A**U**</u>CAGCUUUCUUUUGCCC-5 | ++ | 56% | 1% |

Table 118

| gene | aspergillusAF293protein(XM_748400) (SEQ ID NO:10) | Locus | 1214-1232 BP | |
|---|---|---|---|---|
| | SIRNA | Stability | Expression inhibition ratio | Growth inhibition ratio |
| 118-1 | Unmodified siRNA<br>5-AGGACGGUACUUUCGAAAGTT<br>TTUCCUGCCAUGAAAGCUUUC-5 | + | 46% | 0% |
| 118-2 | Random modification (2'-O-methyl)<br>5-**A**G**G**A**C**G**G**U**A**C**U**U**U**C**G**A**A**A**G**TT<br>TT**U**CC**U**GCCA**U**GAAAGC**UUU**C-5 | ++ | 17% | 51% |
| 118-3A | Specific modification (2'-O-methyl)<br>5-AGGACGG**UA**CUU**U**CG**A**AAGTT<br>TTUCCUGCCA**U**GAAAGCUUUC-5 | ++ | 44% | 6% |
| 118-3B | Specific modification (2'-O-methyl)<br>5-AGGACGGUACUUUCGAAAGTT<br>TTUCCUGCC**A**UGAAAGCUUUC-5 | ++ | 46% | 0% |

Table 119

| gene | Aspergillus AF293 protein XM_748400) (SEQ ID NO:10) | Locus | 1234-1252 BP | |
|---|---|---|---|---|
| | SIRNA | Stability | Expression inhibition ratio | Growth inhibition ratio |
| 119-1 | Unmodified siRNA<br>5-GUAUCCGAAGACGAAGCUATT<br>TTCAUAGGCUUCUGCUUCGAU-5 | + | 97% | 0% |
| 119-2 | Random modification (2'-O-methyl)<br>5-**G**U**A**U**C**C**G**A**A**G**A**C**G**A**A**G**C**U**A**TT<br>TTC**A**U**A**GGCUUCUGCUUCG**A**U-5 | +++ | 30% | 31% |
| 119-3A | Specific modification (2'-O-methyl)<br>5-G**UA**UCCGAA**GA**CGAAGC**UA**TT<br>TTCA**U**AGGCUUCUGCUUCGA**U**-5 | +++ | 92% | 8% |
| 119-3B | Specific modification (2'-O-methyl)<br>5-GUAUCCGAAGACGAAGCUATT<br>TTCAUAGGCUUCUGCUUCGAU-5 | +++ | 95% | 1% |

Table 120

| gene | Aspergillus AF293 protein(XM_748400) (SEQ ID NO: 10) | Locus | 14-32 BP | |
|---|---|---|---|---|
| | SIRNA | Stability | Expression inhibition ratio | Growth inhibition ratio |
| 120-1 | Unmodified siRNA<br>5-AGAGAGGUGGAGGUAUCUCTT<br>TTUCUCUCCACCUCCAUAGAG-5 | + | 91% | 0% |
| 120-2 | Random modification (2'-O-methyl)<br>5-**A**G**A**G**A**G**G**U**G**G**A**G**G**U**A**U**C**U**C**TT<br>TTU**C**U**C**UCC**A**CCUCCAUAGAG-5 | ++ | 67% | 24% |
| 120-3A | Specific modification (2'-O-methyl)<br>5-AG**A**GAGG**U**GGAGG**U**AUCUCTT<br>TTUCU**C**UCCA**C**CUCCA**U**AGAG-5 | ++ | 87% | 8% |
| 120-3B | Specific modification (2'-O-methyl)<br>5-AGAGAGGUGGAGG**U**AUCUCTT<br>TTUCUCUCCACCUCCA**U**AGAG-5 | ++ | 90% | 1% |

Table 121

| gene | Aspergillus AF293 protein XM_748400) (SEQ ID NO:10) | Locus | 103-121 BP | |
|---|---|---|---|---|
| | SIRNA | Stability | Expression inhibition ratio | Growth inhibition ratio |
| 121-1 | Unmodified siRNA<br>5-GAAAACGCUUGUAAAGCCGTT<br>TTCUUUUGCGAACAUUUCGGC-5 | + | 58% | 0% |
| 121-2 | Random modification (2'-O-methyl)<br>5-**G**A**A**A**A**C**G**C**U**U**G**U**A**A**A**G**C**C**G**TT<br>TTCUUUUGCG**AA**C**A**UUUCGGC-5 | ++ | 23% | 24% |
| 121-3A | Specific modification (2'-O-methyl)<br>5-GAAAACGCU**U**G**U**AAAGCCGTT<br>TTCUUUUGCGAA**C**A**U**UUCGGC-5 | ++ | 54% | 5% |
| 121-3B | Specific modification (2'-O-methyl)<br>5-GAAAACGCUUGUAAAGCCGTT<br>TTCUUUUGCGAACA**U**UUCGGC-5 | ++ | 56% | 0% |

Table 122

| gene | Aspergillus AF293protein(XM_748400) (SEQ ID NO:10) SIRNA | Locus Stability | 493-511 BP Expression inhibition ratio | Growth inhibition ratio |
|---|---|---|---|---|
| 122-1 | Unmodified siRNA<br>5-GCUGAACUAGUCGAAAGAATT<br>TTCGACUUGAUCAGCUUUCUU-5 | + | 81% | 0% |
| 122-2 | Random modification (2'-deoxy-2'-fluoro)<br>5-**G**C**U**G**A**A**C**U**A**G**U**C**G**A**A**A**G**A**A**TT<br>TTCG**A**CUUG**A**UC**A**GCUUUCUU-5 | ++ | 49% | 29% |
| 122-3A | Specific modification (2'-deoxy-2'-fluoro)<br>5-GC**U**GAAC**U**AGUCGAAAGAATT<br>TTCGA**C**UUGA**U**CAGCUUUCUU-5 | ++ | 78% | 3% |
| 122-3B | Specific modification (2'-deoxy-2'-fluoro)<br>5-GCUGAAC**U**AGUCGAAAGAATT<br>TTCGACUUGA**U**CAGCUUUCUU-5 | ++ | 79% | 1% |

Table 123

| gene | Aspergillus AF293 protein(XM_748400) (SEQ ID NO:10) SIRNA | Locus Stability | 537-555 BP Expression inhibition ratio | Growth inhibition ratio |
|---|---|---|---|---|
| 123-1 | Unmodified siRNA<br>5-GGAAGAAGAUAGAGCAUCCTT<br>TTCCUUCUUCUAUCUCGUAGG-5 | + | 38% | 0% |
| 123-2 | Random modification (2'-deoxy-2'-fluoro)<br>5-**G**G**A**A**G**A**A**G**A**U**A**G**A**G**C**A**U**C**C**TT<br>TTCCUUCUUCU**A**UCUCGU**A**GG-5 | ++ | 13% | 19 |
| 123-3A | Specific modification (2'-deoxy-2'-fluoro)<br>5-GGAAGAAGA**U**AGAG**C**AUCCTT<br>TTCCUUCUUCUA**U**CUCG**U**AGG-5 | ++ | 37% | 4% |
| 123-3B | Specific modification (2'-deoxy-2'-fluoro)<br>5-GGAAGAAGAUAGAGCAUCCTT<br>TTCCUUCUUCUA**U**CUCGUAGG-5 | ++ | 37% | 0% |

Table 124

| gene | Aspergillus AF293 protein XM_748400) (SEQ ID NO: 10) | Locus | 623-641 BP | |
|---|---|---|---|---|
| | SIRNA | Stability | Expression inhibition ratio | Growth inhibition ratio |
| 124-1 | Unmodified siRNA<br>5-AUCUACCCAGCUCUCCCUUTT<br>TTUAGAUGGGUCGAGAGGGAA-5 | + | 79% | 0% |
| 124-2 | Random modification (2'-O-methyl)<br>5-**A**U**C**U**A**C**C**C**A**G**C**U**C**U**C**C**C**U**U**TT<br>TT**U**AGA**U**GGG**U**CGAGAGGGAA-5 | ++ | 43% | 21% |
| 124-3A | Specific modification (2'-O-methyl)<br>5-AUC**U**ACC**C**AGCUC**U**CCCUUTT<br>TTUAGA**U**GGG**U**CGAGAGGGAA-5 | ++ | 75% | 6% |
| 124-3B | Specific modification (2'-O-methyl)<br>5-AUC**U**ACCCAGCUCUCCCUUTT<br>TTUAGA**U**GGGUCGAGAGGGAA-5 | ++ | 77% | 1% |

Table 125

| gene | Aspergillus AF293protein(XM_748400) (SEQ ID NO:10) | Locus | 1096-1114 BP | |
|---|---|---|---|---|
| | SIRNA | Stability | Expression inhibition ratio | Growth inhibition ratio |
| 125-1 | Unmodified siRNA<br>5-AGCGAUCCCGAACAAGUAUTT<br>TTUCGCUAGGGCUUGUUCAUA-5 | + | 87% | 0% |
| 125-2 | Random modification (2'-O-methyl)<br>5-**A**G**C**G**A**U**C**C**C**G**A**A**C**A**A**G**U**A**U**TT<br>TTU**C**G**C**UAGGG**C**UUGUU**C**AUA-5 | ++ | 34% | 25% |
| 125-3A | Specific modification (2'-O-methyl)<br>5-AGCGAUCCC**G**AA**C**AAG**U**AUTT<br>TTUCGCUAGGGCUUG**U**UCA**U**A-5 | ++ | 84% | 4% |
| 125-3B | Specific modification (2'-O-methyl) | | | |

(continued)

| gene | Aspergillus AF293protein(XM_748400) (SEQ ID NO:10) | Locus | 1096-1114 BP | |
|---|---|---|---|---|
| | SIRNA | Stability | Expression inhibition ratio | Growth inhibition ratio |
| | 5-AGCGAUCCCGAACAAGUAUTT<br>TTUCGCUAGGGCUUGUUCA**U**A-5 | ++ | 85% | 0% |

Table 126

| gene | Aspergillus AF293 protein(XM_748400) (SEQ ID NO:10) | Locus | 1227-1245 BP | |
|---|---|---|---|---|
| | SIRNA | Stability | Expression inhibition ratio | Growth inhibition ratio |
| 126-1 | Unmodified siRNA<br>5-CGAAAGUGUAUCCGAAGACTT<br>TTGCUUUCACAUAGGCUUCUG-5 | + | 77% | 0% |
| 126-2 | Random modification (2'-deoxy-2'-fluoro)<br>5-**C**G**A**A**A**G**U**G**U**A**U**C**C**G**A**A**G**A**C**TT<br>TT**G**CUUUCACAUA**GG**CUUCU**G**-5 | ++ | 41% | 22% |
| 126-3A | Specific modification (2'-deoxy-2'-fluoro)<br>5-CGAAAG**U**G**U**AUCCGAAGACTT<br>TTGCUUUCA**C**A**U**AGGCUUCUG-5 | ++ | 75% | 3% |
| 126-3B | Specific modification (2'-deoxy-2'-fluoro)<br>5-CGAAAGUGUAUCCGAAGACTT<br>TTGCUUUCACA**U**AGGCUUCUG-5 | ++ | 69% | 0% |

Table 127

| gene | Aspergillus AF293 protein(XM_748400) (SEQ ID NO:10) | Locus | 1238-1256 BP | |
|---|---|---|---|---|
| | SIRNA | Stability | Expression inhibition ratio | Growth inhibition ratio |
| 127-1 | Unmodified siRNA<br>5-CCGAAGACGAAGCUAUGAATT<br>TTGGCUUCUGCUUCGAUACUU-5 | + | 48% | 0% |
| 127-2 | Random modification (2'-deoxy-2'-fluoro)<br>5-**C**C**G**A**A**G**A**C**G**A**A**G**C**U**A**U**G**A**A**TT<br>TT**GG**CUUCU**G**CUUC**G**AUACUU-5 | ++ | 23% | 19% |
| 127-3A | Specific modification (2'-deoxy-2'-fluoro) | | | |

(continued)

| gene | Aspergillus AF293 protein(XM_748400) (SEQ ID NO:10) SIRNA | Locus Stability | 1238-1256 BP Expression inhibition ratio | Growth inhibition ratio |
|---|---|---|---|---|
| | 5-CCGAAGACGAAGC**U**A**U**GAATT<br>TTGGCUUCUGCUUCGA**U**A**C**UU-5 | ++ | 46% | 2% |
| 127-3B | Specific modification (2'-deoxy-2'-fluoro)<br>5-CCGAAGACGAAGCUAUGAATT<br>TTGGCUUCUGCUUCGA**U**ACUU-5 | ++ | 47% | 0% |

Table 128

| gene | Human BIC(NR 001458.3)(SEQ ID NO:1) SIRNA | Locus Stability | 170-188 BP Expression inhibition ratio | Growth inhibition ratio |
|---|---|---|---|---|
| 128-1 | Unmodified siRNA<br>5-UCUCCCACCCAAUGGAGAU<br>TTAGAGGGUGGGUUACCUCUA-5 | + | 83% | 0% |
| 128-2 | Random modification (2'-O-methyl)<br>5-**U**C**U**C**C**C**A**C**C**C**A**A**U**G**G**A**G**AU<br>TT**A**G**A**GGGUGGGUU**A**CCUCU**A**-5 | ++ | 49% | 24% |
| 128-3A | Specific modification (2'-O-methyl)<br>5-UCUCC**C**ACC**C**A**A**U**G**GAGAU<br>TTAGAGGG**U**GGGUUACCUCUA-5 | ++ | 78% | 9% |
| 128-3B | Specific modification (2'-O-methyl)<br>5-UCUCC**C**ACCC**A**AUGGAGAU<br>TTAGAGGGUGGGUUACCUCUA-5 | ++ | 81% | 4% |

Table 129

| gene | Human BIC(NR 001458.3)(SEQ ID NO:1) SIRNA | Locus Stability | 206-224 BP Expression inhibition ratio | Growth inhibition ratio |
|---|---|---|---|---|
| 129-1 | Unmodified siRNA<br>5-AAACCAGGAAGGGGAAAUCTT<br>TTUUUGGUCCUUCCCCUUUAG-5 | + | 69% | 0% |
| 129-2 | Random modification (2'-deoxy-2'-fluoro) | | | |

(continued)

| gene | Human BIC(NR 001458.3)(SEQ ID NO:1) SIRNA | Locus Stability | 206-224 BP Expression inhibition ratio | Growth inhibition ratio |
|---|---|---|---|---|
| | 5-**A**A**A**C**C**A**G**G**A**A**G**G**G**G**A**A**A**U**C**TT<br>TTUUUGGUCCUUCCCCUUU**A**G-5 | ++ | 42% | 41% |
| 129-3A | Specific modification (2'-deoxy-2'-fluoro)<br>5-AAAC**CA**GG**A**AG**G**GGAAAUCTT<br>TTUUUG**GU**CCUUCCCCUUUAG-5 | ++ | 61% | 9% |
| 129-3B | Specific modification (2'-deoxy-2'-fluoro)<br>5-AAAC**CA**GGAAGGGGAAAUCTT<br>TTUUUGGUCCUUCCCCUUUAG-5 | ++ | 64% | 3% |

Table 130

| gene | Human BIC(NR 001458.3)(SEQ ID NO:1) SIRNA | Locus Stability | 213-231 BP Expression inhibition ratio | Growth inhibition ratio |
|---|---|---|---|---|
| 130-1 | Unmodified siRNA<br>5-GAAGGGGAAAUCUGUGGUUTT<br>TTCUUCCCCUUUAGACACCAA-5 | + | 66% | 0% |
| 130-2 | Random modification (2'-O-methyl)<br>5-**G**A**A**G**G**G**G**A**A**A**U**C**U**G**U**G**G**U**U**TT<br>TTCUUCCCCUUUAGACACCAA-5 | +++ | 41% | 24% |
| 130-3A | Specific modification (2'-O-methyl)<br>5-GAAGGGGAAAUC**UGUG**GUUTT<br>TTCUUCCCCUUUAG**ACAC**CAA-5 | +++ | 61% | 6% |
| 130-3B | Specific modification (2'-O-methyl)<br>5-GAAGGGGAAAUC**U**GUGGUUTT<br>TTCUUCCCCUUUAGACA**C**CAA-5 | +++ | 65% | 2% |

Table 131

| gene | Human BIC(NR 001458.3)(SEQ ID NO:1) SIRNA | Locus Stability | 398-416 BP Expression inhibition ratio | Growth inhibition ratio |
|---|---|---|---|---|
| 131-1 | Unmodified siRNA | | | |

(continued)

| gene | Human BIC(NR 001458.3)(SEQ ID NO:1) SIRNA | Locus Stability | 398-416 BP Expression inhibition ratio | Growth inhibition ratio |
|---|---|---|---|---|
| | 5-GGGAGGAUGACAAAGAAGCTT<br>TTCCCUCCUACUGUUUCUUCG-5 | + | 86% | 0% |
| 131-2 | Random modification (2'-deoxy-2'-fluoro)<br>5-GGGAGGAUGACAAAGAAGCTT<br>TT**CCC**U**CC**UA**C**UGUUU**C**UU**C**G-5 | ++ | 37% | 19% |
| 131-3A | Specific modification (2'-deoxy-2'-fluoro)<br>5-GGGAGGA**UG**A**C**AAAGAAGCTT<br>TTCCCUCCUA**C**UG**U**UUCUUCG-5 | ++ | 78% | 9% |
| 131-3B | Specific modification (2'-deoxy-2'-fluoro)<br>5-GGGAGGA**U**GA**C**AAAGAAGCTT<br>TTCCCUCCUACUG**U**UUCUUCG-5 | ++ | 84% | 2% |

Table 132

| gene | Human BIC(NR 001458.3)(SEQ ID NO:1) SIRNA | Locus Stability | 170-188 BP Expression inhibition ratio | Growth inhibition ratio |
|---|---|---|---|---|
| 132-1 | Unmodified siRNA<br>5-GAAACUGGUACUUUCCCCCTT<br>TTCUUUGACCAUGAAAGGGGG-5 | + | 90% | 0% |
| 132-2 | Random modification (2'-O-methyl)<br>5-**G**A**A**A**C**U**G**G**U**A**C**U**U**U**C**C**C**C**C**TT<br>TTCUUUGACCAUGAAAGGGGG-5 | ++ | 57% | 24% |
| 132-3A | Specific modification (2'-O-methyl)<br>5-GAAAC**UG**G**U**AC**U**UUCCCCCTT<br>TTCUUUG**AC**CA**U**GAAAGGGGG-5 | ++ | 85% | 7% |
| 132-3 B | Specific modification (2'-O-methyl)<br>5-GAAAC**UG**G**U**ACUUUCCCCCTT<br>TTCUUUGACCA**U**GAAAGGGGG-5 | ++ | 88% | 3% |

Table 133

| gene | Human KAZRIN(NM_201628)(SEQ ID NO:2) SIRNA | Locus Stability | 13-31 BP Expression inhibition ratio | 13-31 BP Growth inhibition ratio |
|---|---|---|---|---|
| 133-1 | Unmodified siRNA<br>5-AUCGCUGCUCCUCCUCCUCTT<br>TTUAGCGACGAGGAGGAGGAG-5 | + | 72% | 0% |
| 133-2 | Random modification (2'-deoxy-2'-fluoro)<br>5-**A**U**C**G**C**U**G**C**U**C**C**U**C**C**U**C**C**U**C**TT<br>TT**U**AGCGACGAGGAGGAGGAG-5 | ++ | 39% | 24% |
| 133-3A | Specific modification (2'-deoxy-2'-fluoro)<br>5-AUCGC**U**GC**U**CC**U**CCUCCUCTT<br>TTUAGCGA**C**GAGGAGGAGGAG-5 | ++ | 69% | 6% |
| 133-3B | Specific modification (2'-deoxy-2'-fluoro)<br>5-AUCGC**U**GCUCCUCCUCCUCTT<br>TTUAGCGA**C**GAGGAGGAGGAG-5 | ++ | 71% | 1% |

Table 134

| gene | Human KAZRIN(NM_201628)(SEQ ID NO:2) SIRNA | Locus Stability | 239-257 BP Expression inhibition ratio | 239-257 BP Growth inhibition ratio |
|---|---|---|---|---|
| 134-1 | Unmodified siRNA<br>5-AGCUCUUUGUCACCUCUCUTT<br>TTUCGAGAAACAGUGGAGAGA-5 | + | 39% | 0% |
| 134-2 | Random modification (2'-O-methyl)<br>5-**A**GCUCUUUGUC**A**CCUCUCUTT<br>TT**U**C**G**A**G**A**A**A**C**A**G**U**G**G**A**G**A**G**A**-5 | ++ | 19% | 24% |
| 134-3A | Specific modification (2'-O-methyl)<br>5-AGCUCUU**U**GU**C**ACC**U**C**U**CUTT<br>TTUCGAGAAA**C**AG**U**GGAGAGA-5 | ++ | 34% | 9% |
| 134-3B | Specific modification (2'-O-methyl)<br>5-AGCUCUUUGUC**A**CCUCUCUTT<br>TTUCGAGAA**AC**AGUGGAGAGA-5 | ++ | 37% | 3% |

Table 135

| gene | Human KAZRIN(NM_201628)(SEQ ID NO:2) SIRNA | Locus Stability | 294-312 BP Expression inhibition ratio | Growth inhibition ratio |
|---|---|---|---|---|
| 135-1 | Unmodified siRNA<br>5-AAUAAGCAGCUCGCGCUCCTT<br>TTUUAUUCGUCGAGCGCGAGG-5 | + | 58% | 0% |
| 135-2 | Random modification (2'-O-methyl)<br>5-**A**A**U**A**A**G**C**A**G**C**U**C**G**C**G**C**U**C**C**TT<br>TTUU**A**UUCGUCG**A**GCGCG**A**GG-5 | ++ | 32% | 24% |
| 135-3A | Specific modification (2'-O-methyl)<br>5-AA**U**AAG**C**AGCUCG**C**GCUCCTT<br>TTUUA**U**UCG**U**CGAGCGCGAGG-5 | ++ | 51% | 9% |
| 135-3B | Specific modification (2'-O-methyl)<br>5-AA**U**AAG**C**AGCUCGCGCUCCTT<br>TTUUA**U**UCGUCGAGCGCGAGG-5 | ++ | 54% | 2% |

Table 136

| gene | Human KAZRIN(NM_201628)(SEQ ID NO:2) SIRNA | Locus Stability | 1383-1401 BP Expression inhibition ratio | Growth inhibition ratio |
|---|---|---|---|---|
| 136-1 | Unmodified siRNA<br>5-CUAGAGGAUCUUGAAGACCTT<br>TTGAUCUCCUAGAACUUCUGG-5 | + | 97% | 0% |
| 136-2 | Random modification (2'-deoxy-2'-fluoro)<br>5-**C**U**A**G**A**G**G**A**U**C**U**U**G**A**A**G**A**C**C**TT<br>TT**G**AUCUCCUA**G**AACUUCU**GG**-5 | ++ | 38% | 29% |
| 136-3A | Specific modification (2'-deoxy-2'-fluoro)<br>5-C**U**AGAGGAUCU**U**GAAGACCTT<br>TTGA**U**CUCCUAGAA**C**UUCUGG-5 | ++ | 91% | 5% |
| 136-3B | Specific modification (2'-deoxy-2'-fluoro)<br>5-CUAGAGGAUCUUGAAGACCTT<br>TTGA**U**CUCCUAGAA**C**UUCUGG-5 | ++ | 95% | 1% |

Table 137

| gene | Human KAZRIN(NM_201628)(SEQ ID NO:2) | Locus | 2151-2169 BP | |
|---|---|---|---|---|
| | SIRNA | Stability | Expression inhibition ratio | Growth inhibition ratio |
| 137-1 | Unmodified siRNA<br>5-AUCGACCUGAAGGAGUACGTT<br>TTUAGCUGGACUUCCUCAUGC-5 | + | 32% | 0% |
| 137-2 | Random modification (2'-O-methyl)<br>5-A**UC**GA**CCU**GAAGGAG**U**A**C**GTT<br>TT**U**AG**CU**GGA**CUUCCUC**A**U**G**C**-5 | ++ | 19% | 24% |
| 137-3A | Specific modification (2'-O-methyl)<br>5-AUC**G**ACC**U**GAAGGAG**U**ACGTT<br>TTUAGCUGGA**C**UUCCUCA**U**GC-5 | ++ | 29% | 6% |
| 137-3B | Specific modification (2'-O-methyl)<br>5-AUCGACC**U**GAAGGAG**U**ACGTT<br>TTUAGCUGGACUUCCUCA**U**GC-5 | ++ | 30% | 1% |

Table 138

| gene | Human CDKN1B(NM_004064)(SEQ ID NO:3) | Locus | 80-98 BP | |
|---|---|---|---|---|
| | SIRNA | Stability | Expression inhibition ratio | Growth inhibition ratio |
| 138-1 | Unmodified siRNA<br>5-CCCUCCCCUGUCCCCGCUUTT<br>TTGGGAGGGGACAGGGGCGAA-5 | + | 75% | 0% |
| 138-2 | Random modification (2'-deoxy-2'-fluoro)<br>5-**C**C**C**U**C**C**C**C**U**G**U**C**C**C**C**G**C**U**U**TT<br>TTGGG**A**GGGG**A**C**A**GGGGCG**AA**-5 | ++ | 41% | 34% |
| 138-3A | Specific modification (2'-deoxy-2'-fluoro)<br>5-CCCUCCCC**U**GUCCCC**G**CUUTT<br>TTGGGAGGGGA**C**AGGGGCGAA-5 | ++ | 72% | 3% |
| 138-3B | Specific modification (2'-deoxy-2'-fluoro)<br>5-CCCUCCCC**U**GUCCCCGCUUTT<br>TTGGGAGGGGACAGGGGCGAA-5 | ++ | 73% | 1% |

Table 139

| gene | Human CDKN1B(NM_004064)(SEQ ID NO:3) SIRNA | Locus Stability | 431-449 BP Expression inhibition ratio | Growth inhibition ratio |
|---|---|---|---|---|
| 139-1 | Unmodified siRNA<br>5-UUUGAUCAGCGGAGACUCGTT<br>TTAAACUAGUCGCCUCUGAGC-5 | + | 94% | 0% |
| 139-2 | Random modification (2'-O-methyl)<br>5-**U**U**U**G**A**U**C**A**G**C**G**G**A**G**A**C**U**C**G**TT<br>TTAAAC**U**AGUC**G**CCUCUGAGC-5 | ++ | 49% | 31% |
| 139-3A | Specific modification (2'-O-methyl)<br>5-UU**U**GAU**C**AGCGGAGACUCGTT<br>TTAAA**C**UAG**U**CGCCUCUGAGC-5 | ++ | 89% | 3% |
| 139-3B | Specific modification (2'-O-methyl)<br>5-UUU**G**AU**C**AGCGGAGACUCGTT<br>TTAAACUAGUCGCCUCUGAGC-5 | ++ | 82% | 0.5% |

Table 140

| gene | Human CDKN1B(NM_004064)(SEQ ID NO:3) SIRNA | Locus Stability | 591-609 BP Expression inhibition ratio | Growth inhibition ratio |
|---|---|---|---|---|
| 140-1 | Unmodified siRNA<br>5-AGUUAACCCGGGACUUGGATT<br>TTUCAAUUGGGCCCUGAACCU-5 | + | 93% | 0% |
| 140-2 | Random modification (2'-O-methyl)<br>5-**A**G**UU**A**A**C**C**C**G**G**G**A**C**U**U**G**G**ATT<br>TTUCAAUU**GGG**CCCU**G**AACCU-5 | ++ | 45% | 34% |
| 140-3A | Specific modification (2'-O-methyl)<br>5-AGU**U**AACCCGGGACU**U**GGATT<br>TTUCAA**U**UGGGCCCUGAA**C**CU-5 | ++ | 90% | 3% |
| 140-3B | Specific modification (2'-O-methyl)<br>5-AGU**U**AACCCGGGACUUGGATT<br>TTUCAAUUGGGCCCUGAA**C**CU-5 | ++ | 91% | 1% |

Table 141

| gene | Human CDKN1B(NM_004064)(SEQ ID NO:3) SIRNA | Locus Stability | 669-687 BP Expression inhibition ratio | Growth inhibition ratio |
|---|---|---|---|---|
| 141-1 | Unmodified siRNA<br>5-AUCACAAACCCCUAGAGGGTT<br>TTUAGUGUUUGGGGAUCUCCC-5 | + | 77% | 0% |
| 141-2 | Random modification (2'-O-methyl)<br>5-**A**U**C**A**C**A**A**A**C**C**C**C**U**A**G**A**G**G**G**TT<br>TTU**A**GUGUUUGGGG**A**UCUCCC-5 | ++ | 35% | 31% |
| 141-3A | Specific modification (2'-O-methyl)<br>5-AU**C**A**C**AAACCCC**U**AGAGGGTT<br>TTUAG**U**G**U**UUGGGGA**U**CUCCC-5 | ++ | 75% | 4% |
| 141-3B | Specific modification (2'-O-methyl)<br>5-AU**C**A**C**AAACCCCUAGAGGGTT<br>TTUAG**U**GUUUGGGGA**U**CUCCC-5 | ++ | 75% | 1% |

Table 142

| gene | Human CDKN1B(NM_004064)(SEQ ID NO:3) SIRNA | Locus Stability | 674-692 BP Expression inhibition ratio | Growth inhibition ratio |
|---|---|---|---|---|
| 142-1 | Unmodified siRNA<br>5-AAACCCCUAGAGGGCAAGUTT<br>TTUUUGGGGAUCUCCCGUUCA-5 | + | 84% | 0% |
| 142-2 | Random modification (2'-deoxy-2'-fluoro)<br>5-AAACCCCUAGAGGGCAAGUTT<br>TT**U**U**U**G**G**G**G**A**U**C**U**C**C**C**G**U**U**C**A**-5 | ++ | 37% | 29% |
| 142-3A | Specific modification (2'-deoxy-2'-fluoro)<br>5-AAACCCC**U**AGAGGG**C**AAG**U**TT<br>TTUUUGGGGA**U**CUCCCG**U**UCA-5 | ++ | 82% | 5% |
| 142-3B | Specific modification (2'-deoxy-2'-fluoro)<br>5-AAACCCC**U**AGAGGG**C**AAGUTT<br>TTUUUGGGGAUCUCCCGUUCA-5 | ++ | 83% | 1% |

Table 143

| gene | Chinpanzee SOD2(NM_001009022) (SEQ ID NO:4) SIRNA | Locus Stability | 45-63 BP | |
|---|---|---|---|---|
| | | | Expression inhibition ratio | Growth inhibition ratio |
| 143-1 | Unmodified siRNA<br>5-GGAACCUCACAUCAACGCGCATT<br>TTCCUUGGAGUGUAGUUGCGCGU-5 | + | 70% | 0% |
| 143-2 | Random modification (2'-deoxy-2'-fluoro)<br>5-**G**G**A**A**C**C**U**C**A**C**A**U**C**A**A**C**G**C**G**C**A**TT<br>TTCCUUGG**A**GUGU**A**GUUGCGCGU-5 | ++ | 21% | 43% |
| 143-3A | Specific modification (2'-deoxy-2'-fluoro)<br>5-GGAACCU**C**A**C**AU**C**AACGCG**C**ATT<br>TTCCUUGGAG**U**G**U**AG**U**UGCGCG**U**-5 | ++ | 52% | 11% |
| 143-3B | Specific modification (2'-deoxy-2'-fluoro)<br>5-GGAACCU**C**ACAUCAACGCGCATT<br>TTCCUUGGAGUG**U**AG**U**UGCGC**G**U-5 | ++ | 65% | 4% |

Table 144

| gene | Chimpanzee SOD2(NM_001009022) (SEQ ID NO:4) SIRNA | Locus Stability | 116-134 BP | |
|---|---|---|---|---|
| | | | Expression inhibition ratio | Growth inhibition ratio |
| 144-1 | Unmodified siRNA<br>5-UGAACGUCACCGAGGAGAATT<br>TTACUUGCAGUGGCUCCUCUU-5 | + | 87% | 0% |
| 144-2 | Random modification (2'-O-methyl)<br>5-**U**G**A**A**C**G**U**C**A**C**C**G**A**G**G**A**G**A**A**TT<br>TT**A**CUUGC**A**GUGGCUCCUCUU-5 | ++ | 65% | 24% |
| 144-3A | Specific modification (2'-O-methyl)<br>5-**U**GAACGU**C**ACCGAGGAGAATT<br>TTA**C**UUGCAG**U**GGCUCCUCUU-5 | ++ | 82% | 5% |
| 144-3B | Specific modification (2'-O-methyl)<br>5-UGAACGU**C**ACCGAGGAGAATT<br>TTA**C**UUGCAGUGGCUCCUCUU-5 | ++ | 85% | 1% |

Table 145

| gene | Chimpanzee SOD2(NM_001009022) (SEQ ID NO:4) SIRNA | Locus Stability | 266-284 BP Expression inhibition ratio | Growth inhibition ratio |
|---|---|---|---|---|
| 145-1 | Unmodified siRNA<br>5-AACCCAAAGGGGAGUUGCUTT<br>TTUUGGGUUUCCCCUCAACGA-5 | + | 97% | 0% |
| 145-2 | Random modification (2'-O-methyl)<br>5-AA**CCC**AAAGGGGAGUUG**C**UTT<br>TT**U**U**G**G**G**U**U**U**C**C**C**C**U**C**A**A**C**G**A**-5 | ++ | 37% | 49% |
| 145-3A | Specific modification (2'-O-methyl)<br>5-AACC**C**AAAGGG**G**AGU**U**GCUTT<br>TTUUGGG**U**UUCCCCUCAA**C**GA-5 | ++ | 92% | 6% |
| 145-3B | Specific modification (2'-O-methyl)<br>5-AACCCAAAGGGGAGU**U**GCUTT<br>TTUUGGG**U**UUCCCCUCAACGA-5 | ++ | 83% | 1% |

Table 146

| gene | Chimpanzee SOD2(NM_001009022) (SEQ ID NO:4) | Locus | | |
|---|---|---|---|---|
| | | | 274-292 BP | |
| | SIRNA | Stability | Expression inhibition ratio | Growth inhibition ratio |
| 146-1 | Unmodified siRNA<br>5-AAAGGGGAGUUGCUGGAAGTT<br>TTUUUCCCCUCAACGACCUUC-5 | + | 77% | 0% |
| 146-2 | Random modification (2'-O-methyl)<br>5-**A**A**A**G**G**G**G**A**G**U**U**G**C**U**G**G**A**A**G**TT<br>TTUUUCCCCUC**AA**CGACCUUC-5 | ++ | 47% | 31% |
| 146-3A | Specific modification (2'-O-methyl)<br>5-AAAGGGGAGU**U**GC**U**GGAAGTT<br>TTUUUCCCCUCAA**C**GA**C**CUUC-5 | ++ | 74% | 4% |
| 146-3B | Specific modification (2'-O-methyl)<br>5-AAAGGGGAGU**U**GCUGGAAGTT<br>TTUUUCCCCUCAACGA**C**CUUC-5 | ++ | 76% | 1% |

Table 147

| gene | Chimpanzee SOD2(NM_001009022) (SEQ ID NO:4) SIRNA | Locus Stability | 295-313 BP Expression inhibition ratio | Growth inhibition ratio |
|---|---|---|---|---|
| 147-1 | Unmodified siRNA<br>5-AAACGUGACUUUGGUUCCUTT<br>TTUUUGCACUGAAACCAAGGA-5 | + | 86% | 0% |
| 147-2 | Random modification (2'-O-methyl)<br>5-**AAA**C**G**U**G**A**C**U**U**U**G**G**U**U**C**C**U**TT<br>TTUUU**G**CACU**G**AAACCAA**GG**A-5 | ++ | 37% | 29% |
| 147-3A | Specific modification (2'-O-methyl)<br>5-AAACG**U**GACUU**U**GGUUC**C**UTT<br>TTUUUGCA**C**UGAAA**C**CAAGGA-5 | ++ | 82% | 5% |
| 147-3B | Specific modification (2'-O-methyl)<br>5-AAACGUGACUU**U**GGUUCCUTT<br>TTUUUGCA**C**UGAAACCAAGGA-5 | ++ | 83% | 1% |

Table 148

| gene | Chimpanzee SOD2(NM_001009022) (SEQ ID NO:4) SIRNA | Locus Stability | 108-126 BP Expression inhibition ratio | Growth inhibition ratio |
|---|---|---|---|---|
| 148-1 | Unmodified siRNA<br>5-GAAUAACCUGAACGUCACCTT<br>TTCUUAUUGGACUUGCAGUGG-5 | + | 72% | 0% |
| 148-2 | Random modification (2'-O-methyl)<br>5-**G**A**A**U**A**A**C**C**U**G**A**A**C**G**U**C**A**C**C**TT<br>TTCUU**A**UUGG**A**CUUGC**A**GUGG-5 | ++ | 21% | 19% |
| 148-3A | Specific modification (2'-O-methyl)<br>5-GAA**U**AACC**U**GAACGU**C**ACCTT<br>TTCUUA**U**UGGA**C**UUGCAG**U**GG-5 | ++ | 69% | 10% |
| 148-3B | Specific modification (2'-O-methyl)<br>5-GAA**U**AACCUGAACGU**C**ACCTT<br>TTCUUAUUGGA**C**UUGCAGUGG-5 | ++ | 71% | 3% |

Table 149

| gene | Chimpanzee SOD2(NM_001009022) (SEQ ID NO:4) SIRNA | Locus Stability | 119-137 BP | |
|---|---|---|---|---|
| | | | Expression inhibition ratio | Growth inhibition ratio |
| 149-1 | Unmodified siRNA<br>5-ACGUCACCGAGGAGAAGUATT<br>TTUGCAGUGGCUCCUCUUCAU-5 | + | 81% | 0% |
| 149-2 | Random modification (2'-O-methyl)<br>5-**A**C**G**U**C**A**C**C**G**A**G**G**A**G**A**A**G**U**A**TT<br>TTU**G**CA**G**U**GG**CUCCUCUUCAU-5 | ++ | 31% | 26% |
| 149-3A | Specific modification (2'-O-methyl)<br>5-ACGU**C**ACCGAGGAGAAG**UA**TT<br>TTUGCAG**U**GGCUCCUCUUC**AU**-5 | ++ | 75% | 6% |
| 149-3B | Specific modification (2'-O-methyl)<br>5-ACGU**C**ACCGAGGAGAAGUATT<br>TTUGCAG**U**GGCUCCUCUUCA**U**-5 | ++ | 80% | 1% |

Table 150

| gene | Chimpanzee SOD2(NM_001009022) (SEQ ID NO:4) SIRNA | Locus Stability | 131-149 BP | |
|---|---|---|---|---|
| | | | Expression inhibition ratio | Growth inhibition ratio |
| 150-1 | Unmodified siRNA<br>5-AGAAGUACCAGGAGGCGUUTT<br>TTUCUUCAUGGUCCUCCGCAA-5 | + | 62% | 0% |
| 150-2 | Random modification (2'-deoxy-2'-fluoro)<br>5-**A**G**A**A**G**U**A**C**C**A**G**G**A**G**G**C**G**U**U**TT<br>TTUCUUCAU**GG**UCCUCC**G**CAA-5 | ++ | 39% | 35% |
| 150-3A | Specific modification (2'-deoxy-2'-fluoro)<br>5-AGAAG**U**AC**C**AGGAGGCGUUTT<br>TTUCUUCA**U**GG**U**CCUCCGCAA-5 | ++ | 57% | 3% |
| 150-3B | Specific modification (2'-deoxy-2'-fluoro)<br>5-AGAAG**U**ACCAGGAGGCGUUTT<br>TTUCUUCAUGG**U**CCUCCGCAA-5 | ++ | 60% | 1% |

Table 151

| gene | Chimpanzee SOD2(NM_001009022) (SEQ ID NO:4) SIRNA | Locus Stability | 240-258 BP Expression inhibition ratio | Growth inhibition ratio |
|---|---|---|---|---|
| 151-1 | Unmodified siRNA<br>5-AAACCUCAGCCCUAACGGUTT<br>TTUUUGGAGUCGGGAUUGCCA-5 | + | 71% | 0% |
| 151-2 | Random modification (2'-O-methyl)<br>5-**A**A**A**C**C**U**C**A**G**C**C**C**U**A**A**C**G**G**U**TT<br>TTUUUGGAGU**C**GGGAUUG**CC**A-5 | ++ | 28% | 27% |
| 151-3A | Specific modification (2'-O-methyl)<br>5-AAACCU**C**AGCCC**U**AACGGUTT<br>TTUUUGGAG**U**CGGGA**U**UGCCA-5 | ++ | 70% | 3% |
| 151-3B | Specific modification (2'-O-methyl)<br>5-AAACCUC**A**GCCCUAACGGUTT<br>TTUUUGGAGUCGGGA**U**UGCCA-5 | ++ | 70% | 1% |

Table 152

| gene | chimpanzee SOD2(NM_001009022) (SEQ ID NO:4) SIRNA | Locus Stability | 323-341 BP Expression inhibition ratio | Growth inhibition ratio |
|---|---|---|---|---|
| 152-1 | Unmodified siRNA<br>5-UUAAGGAGAAGCUGACGGCTT<br>TTAAUUCCUCUUCGACUGCCG-5 | + | 87% | 0% |
| 152-2 | Random modification (2'-O-methyl)<br>5-**U**U**A**A**G**G**A**G**A**A**G**C**U**G**A**C**G**G**C**TT<br>TTAAUUCCUCUUC**G**ACU**G**CC**G**-5 | ++ | 45% | 24% |
| 152-3A | Specific modification (2'-O-methyl)<br>5-U**U**AAGGAGAAGC**UG**ACGGCTT<br>TTAA**U**UCCUCUUCGA**C**UGCCG-5 | ++ | 82% | 4% |
| 152-3B | Specific modification (2'-O-methyl)<br>5-U**U**AAGGAGAAGCUGACGGCTT<br>TTAA**U**UCCUCUUCGA**C**UGCCG-5 | ++ | 83% | 1% |

Table 153

| gene | rhesus monkey RECEPTOR(XM_001111972) (SEQ ID NO:5) | Locus | 3-21 BP | |
|---|---|---|---|---|
| | SIRNA | Stability | Expression inhibition ratio | Growth inhibition ratio |
| 153-1 | Unmodified siRNA<br>5-AACUUCUGGGAGCCUCCAATT<br>TTUUGAAGACCCUCGGAGGUU-5 | + | 75% | 0% |
| 153-2 | Random modification (2'-O-methyl)<br>5-**A**A**C**U**U**C**U**G**G**G**A**G**C**C**U**C**C**A**A**TT<br>TTUUG**AA**G**A**CCCUCGG**A**GGUU-5 | ++ | 42% | 27% |
| 153-3A | Specific modification (2'-O-methyl)<br>5-AACUUC**U**GGGAGCCUC**C**AATT<br>TTUUGAAGA**C**CCUCGGAGG**U**U-5 | ++ | 69% | 4% |
| 153-3B | Specific modification (2'-O-methyl)<br>5-AACUUCUGGGAGCCUC**C**AATT<br>TTUUGAAGA**C**CCUCGGAGGUU-5 | ++ | 73% | 1% |

Table 154

| gene | Rhesus monkey RECEPTOR(XM_001111972) (SEQ ID NO: 5) | Locus | 80-98 BP | |
|---|---|---|---|---|
| | SIRNA | Stability | Expression inhibition ratio | Growth inhibition ratio |
| 154-1 | Unmodified siRNA<br>5-CCAGCGAGCCCAAAAGAAATT<br>TTGGUCGCUCGGGUUUUCUUU-5 | + | 87% | 0% |
| 154-2 | Random modification (2'-deoxy-2'-fluoro)<br>5-CCA**G**C**G**A**G**CCCAAAA**G**AAATT<br>TT**G**G**U**C**G**C**U**C**G**G**G**U**U**U**U**C**U**U**U**-5 | ++ | 35% | 24% |
| 154-3A | Specific modification (2'-deoxy-2'-fluoro)<br>5-C**C**AGCGAGCC**C**AAAA**G**AAATT<br>TTG**G**U**U**CGCUCGG**G**U**U**UUCUUU-5 | ++ | 82% | 5% |
| 154-3B | Specific modification (2'-deoxy-2'-fluoro)<br>5-C**C**AGCGAGCC**C**AAAAGAAATT<br>TTGGUCGCUCGGGUUUUCUUU-5 | ++ | 85% | 1% |

Table 155

| gene | Rhesus monkey RECEPTOR(XM_001111972) (SEQ ID NO:5) | Locus | 216-234 BP | |
|---|---|---|---|---|
| | SIRNA | Stability | Expression inhibition ratio | Growth inhibition ratio |
| 155-1 | Unmodified siRNA<br>5-CUCAAAUCAAGGCCGGAGUTT<br>TTGAGUUUAGUUCCGGCCUCA-5 | + | 86% | 0% |
| 155-2 | Random modification (2'-O-methyl)<br>5-**C**U**C**A**A**A**U**C**A**A**G**G**C**C**G**G**A**G**U**TT<br>TT**G**A**G**UUUA**G**UUCC**GG**CCUCA-5 | ++ | 36% | 34% |
| 155-3A | Specific modification (2'-O-methyl)<br>5-CU**C**AAAU**C**AAGGCCGGAGUTT<br>TTGAG**U**UUAG**U**UCCGGCCUCA-5 | ++ | 82% | 4% |
| 155-3B | Specific modification (2'-O-methyl)<br>5-CUCAAAUCAAGGCCGGAGUTT<br>TTGAG**U**UUAG**U**UCCGGCCUCA-5 | ++ | 85% | 1% |

Table 156

| gene | Rhesus monkey RECEPTOR(XM_001111972) (SEQ ID NO:5) | Locus | 13-31 BP | |
|---|---|---|---|---|
| | SIRNA | Stability | Expression inhibition ratio | Growth inhibition ratio |
| 156-1 | Unmodified siRNA<br>5-AGCCUCCAAACUCCUAGCUTT<br>TTUCGGAGGUUUGAGGAUCGA-5 | + | 71% | 0% |
| 156-2 | Random modification (2'-O-methyl)<br>5-**A**G**C**C**U**C**C**A**A**A**C**U**C**C**U**A**G**C**U**TT<br>TTU**C**GGAGGUUUGAGGAU**C**GA-5 | ++ | 47% | 19% |
| 156-3A | Specific modification (2'-O-methyl)<br>5-AGCCUC**C**AAACUCC**U**AGCUTT<br>TTUCGGAGG**U**UUGAGGA**U**CGA-5 | ++ | 65% | 3% |
| 156-3B | Specific modification (2'-O-methyl)<br>5-AGCCUCCAAACUCCUAGCUTT<br>TTUCGGAGG**U**UUGAGGA**U**CGA-5 | ++ | 70% | 1% |

Table 157

| gene | Rhesus monkey RECEPTOR(XM_001111972) (SEQ ID NO:5) | Locus | 162-180 BP | |
|---|---|---|---|---|
| | SIRNA | Stability | Expression inhibition ratio | Growth inhibition ratio |
| 157-1 | Unmodified siRNA<br>5-AUACUUGGCGGGCUCUUUCTT<br>TTUAUGAACCGCCCGAGAAAG-5 | + | 73% | 0% |
| 157-2 | Random modification (2'-O-methyl)<br>5-**A**U**A**C**U**U**G**G**C**G**G**G**C**U**C**U**U**U**C**TT<br>TTUAU**G**AACC**G**CCC**G**A**G**AAA**G**-5 | ++ | 25% | 31% |
| 157-3A | Specific modification (2'-O-methyl)<br>5-A**UA**CU**UG**GCGGG**C**UCUUUCTT<br>TTUA**U**GAA**C**CGCCCGAGAAAG-5 | ++ | 70% | 4% |
| 157-3B | Specific modification (2'-O-methyl)<br>5-AU**A**CUUGGCGGGCUCUUUCTT<br>TTUAUGAA**C**CGCCCGAGAAAG-5 | ++ | 72% | 1% |

Table 158

| gene | Dog B cell lymphoma(NM_001003016) (SEQ ID NO:6) | Locus | 508-526 BP | |
|---|---|---|---|---|
| | SIRNA | Stability | Expression inhibition ratio | Growth inhibition ratio |
| 158-1 | Unmodified siRNA<br>5-UUGGGGAAGCGGCCGGCGGTT<br>TTAACCCCUUCGCCGGCCGCC-5 | + | 84% | 0% |
| 158-2 | Random modification (2'-O-methyl)<br>5-**U**U**G**G**G**G**A**A**G**C**G**G**C**C**G**G**C**G**G**TT<br>TT**AA**CCCCUUCGCCGGCCGCC-5 | ++ | 36% | 27% |
| 158-3A | Specific modification (2'-O-methyl)<br>5-U**UG**GGG**AA**GCGGCCGGCGGTT<br>TTAA**C**CCCUUCGCCGGCCGCC-5 | ++ | 78% | 5% |
| 158-3B | Specific modification (2'-O-methyl)<br>5-U**U**GGGGAAGCGGCCGGCGGTT<br>TTAA**C**CCCUUCGCCGGCCGCC-5 | ++ | 82% | 1% |

Table 159

| gene | Dog Bcell lymphoma(NM_001003016) (SEQ ID NO:6) | Locus | 794-812 BP | |
|---|---|---|---|---|
| | SIRNA | Stability | Expression inhibition ratio | Growth inhibition ratio |
| 159-1 | Unmodified siRNA<br>5-AAGGCAUGCUUCGGAAACUTT<br>TTUUCCGUACGAAGCCUUUGA-5 | + | 96% | 0% |
| 159-2 | Random modification (2'-O-methyl)<br>5-**A**A**G**G**C**A**U**G**C**U**U**C**G**G**A**A**A**C**U**TT<br>TTUUCC**G**UAC**G**AAGCCUUU**G**A-5 | ++ | 60% | 35% |
| 159-3A | Specific modification (2'-O-methyl)<br>5-AAGG**C**A**UG**CUUCGGAAACUTT<br>TTUUCCG**U**A**C**GAAGCCUUUGA-5 | ++ | 87% | 6% |
| 159-3B | Specific modification (2'-O-methyl)<br>5-AAGG**C**AUGCUUCGGAAACUTT<br>TTUUCCGUA**C**GAAGCCUUUGA-5 | ++ | 95% | 1% |

Table 160

| gene | Dog B cell lymphoma(NM_001003016) (SEQ ID NO:6) | Locus | 801-819 BP | |
|---|---|---|---|---|
| | SIRNA | Stability | Expression inhibition ratio | Growth inhibition ratio |
| 160-1 | Unmodified siRNA<br>5-GCUUCGGAAACUGGACAUCTT<br>TTCGAAGCCUUUGACCUGUAG-5 | + | 91% | 0% |
| 160-2 | Random modification (2'-deoxy-2'-fluoro)<br>5-**G**C**U**U**C**G**G**A**A**C**U**G**G**A**C**A**U**C**TT**<br>TTC**G**AA**G**CCUUU**G**ACCU**G**UA**G**-5 | ++ | 29% | 37% |
| 160-3A | Specific modification (2'-deoxy-2'-fluoro)<br>5-GCUUCGGAAAC**UG**GA**C**AUCTT<br>TTCGAAGCCUUUG**AC**CUG**U**AG-5 | ++ | 87% | 5% |
| 160-3B | Specific modification (2'-deoxy-2'-fluoro)<br>5-GCUUCGGAAACUGGA**C**AUCTT<br>TTCGAAGCCUUUGA**C**CUG**U**AG-5 | ++ | 90% | 1% |

Table 161

| gene | Dog Bcell lymphoma(NM_001003016) (SEQ ID NO:6) SIRNA | Locus Stability | 21-39 BP Expression inhibition ratio | Growth inhibition ratio |
|---|---|---|---|---|
| 161-1 | Unmodified siRNA<br>5-AACUCUACUGUGGAGUCGGTT<br>TTUUGAGAUGACACCUCAGCC-5 | + | 73% | 0% |
| 161-2 | Random modification (2'-deoxy-2'-fluoro)<br>5-**A**A**C**U**C**U**A**C**U**G**U**G**G**A**G**U**C**G**G**TT<br>TTUU**G**A**G**AU**G**ACACCUCA**G**CC-5 | ++ | 25% | 31% |
| 161-3A | Specific modification (2'-deoxy-2'-fluoro)<br>5-AACUC**U**AC**U**G**U**GGAGUCGGTT<br>TTUUGAGA**U**GA**C**A**C**CUCAGCC-5 | ++ | 65% | 8% |
| 161-3B | Specific modification (2'-deoxy-2'-fluoro)<br>5-AACUCUAC**U**GUGGAGUCGGTT<br>TTUUGAGA**U**GACA**C**CUCAGCC-5 | ++ | 72% | 2% |

Table 162

| gene | Dog Bcell lymphoma(NM_001003016) (SEQ ID NO:6) SIRNA | Locus Stability | 124-142 BP Expression inhibition ratio | Growth inhibition ratio |
|---|---|---|---|---|
| 162-1 | Unmodified siRNA<br>5-AGAAACGCAGUAAUCCGGATT<br>TTCUUUGCGUCAUUAGGCCU-5 | + | 87% | 0% |
| 162-2 | Random modification (2'-O-methyl)<br>5-**A**G**A**A**A**C**G**C**A**G**U**A**A**U**C**C**G**G**A**TT<br>TTCUUU**G**C**G**UCAUUA**GG**CCU-5 | ++ | 36% | 27% |
| 162-3A | Specific modification (2'-O-methyl)<br>5-AGAAACG**C**AG**UA**AUCCGGATT<br>TTCUUUGCG**U**C**AU**UAGGCCU-5 | ++ | 78% | 5% |
| 162-3B | Specific modification (2'-O-methyl)<br>5-AGAAACGCAG**U**AAUCCGGATT<br>TTCUUUGCG**U**CAUUAGGCCU-5 | ++ | 82% | 1% |

Table 163

| gene | rat(NM_001014070.1) (SEQ ID NO:7) | Locus | 447-465 BP | |
|---|---|---|---|---|
| | SIRNA | Stability | Expression inhibition ratio | Growth inhibition ratio |
| 163-1 | Unmodified siRNA<br>5-GGAAGUCCACCUUCUCCGATT<br>TTCCUUCAGGUGGAAGAGGCU-5 | + | 55% | 0% |
| 163-2 | Random modification (2'-O-methyl)<br>5-**G**G**A**A**G**U**C**C**A**C**C**U**U**C**U**C**C**G**A**TT<br>TTCC**UU**CAGG**U**GGAAGAGGC**U**-5 | ++ | 27% | 31% |
| 163-3A | Specific modification (2'-O-methyl)<br>5-GGAAGUC<u>**CA**</u>CC**UU**CUCCGATT<br>TTCCUUCAG<u>G**U**</u>GGAAGAGGCU-5 | ++ | 51% | 5% |
| 163-3B | Specific modification (2'-O-methyl)<br>5-GGAAGUC<u>C**A**</u>CCUUCUCCGATT<br>TTCCUUCAG<u>G**U**</u>GGAAGAGGCU-5 | ++ | 54% | 1% |

Table 164

| gene | rat(NM_001014070.1)(SEQ ID NO:7) | Locus | 460-478 BP | |
|---|---|---|---|---|
| | SIRNA | Stability | Expression inhibition ratio | Growth inhibition ratio |
| 164-1 | Unmodified siRNA<br>5-CUCCGACAGAUGAAGGAAATT<br>TTGAGGCUGUCUACUUCCUUU-5 | + | 73% | 0% |
| 164-2 | Random modification (2'-O-methyl)<br>5-**C**U**C**C**G**A**C**A**G**A**U**G**A**A**G**G**A**A**A**TT<br>TTG**A**GGCUGUCU**A**CUUCCUUU-5 | ++ | 46% | 21% |
| 164-3A | Specific modification (2'-O-methyl)<br>5-CUCCGA<u>**CA**</u>GA<u>**U**G</u>AAGGAAATT<br>TTGAGGCU<u>**GU**</u>CU<u>A**C**</u>UUCCUUU-5 | ++ | 70% | 6% |
| 164-3B | Specific modification (2'-O-methyl)<br>5-CUCCGA<u>CA</u>GA<u>**U**G</u>AAGGAAATT<br>TTGAGGCU<u>G**U**</u>CU<u>AC</u>UUCCUUU-5 | ++ | 72% | 1% |

Table 165

| gene | rat(NM_001014070.1)(SEQ ID NO:7) SIRNA | Locus Stability | 487-505 BP Expression inhibition ratio | Growth inhibition ratio |
|---|---|---|---|---|
| 165-1 | Unmodified siRNA<br>5-AAGGACCUAGAGGAGUCACTT<br>TTUUCCUGGAUCUCCUCAGUG-5 | + | 83% | 0% |
| 165-2 | Random modification (2'-O-methyl)<br>5-**A**A**G**G**A**C**C**U**A**G**A**G**G**A**G**U**C**A**C**TT<br>TTUUCCU**GG**AUCUCCUCA**G**U**G**-5 | ++ | 36% | 27% |
| 165-3A | Specific modification (2'-O-methyl)<br>5-AAGGACC**U**AGAGGAGU**CA**CTT<br>TTUUCCUGGA**U**CUCCUCAG**U**G-5 | ++ | 78% | 5% |
| 165-3B | Specific modification (2'-O-methyl)<br>5-AAGGACC**U**AGAGGAGUCACTT<br>TTUUCCUGGAUCUCCUCAG**U**G-5 | ++ | 80% | 1% |

Table 166

| gene | rat(NM_001014070.1)(SEQ ID NO:7) SIRNA | Locus Stability | 614-632 BP Expression inhibition ratio | Growth inhibition ratio |
|---|---|---|---|---|
| 166-1 | Unmodified siRNA<br>5-CUGACCGGAAGCGCUUAAATT<br>TTGACUGGCCUUCGCGAAUUU-5 | + | 90% | 0% |
| 166-2 | Random modification (2'-deoxy-2'-fluoro)<br>5-**C**U**G**A**C**C**G**G**A**A**G**C**G**C**U**U**A**A**A**TT<br>TTG**A**CUGGCCUUCGCG**AA**UUU-5 | ++ | 59% | 24% |
| 166-3A | Specific modification (2'-deoxy-2'-fluoro)<br>5-C**U**GACCGGAA**G**CGCU**U**AAATT<br>TTGA**C**UGGCCUUCGCGAA**U**UU-5 | ++ | 85% | 4% |
| 166-3B | Specific modification (2'-deoxy-2'-fluoro)<br>5-C**U**GACCGGAAGCGCUUAAATT<br>TTGACUGGCCUUCGCGAA**U**UU-5 | ++ | 87% | 1% |

Table 167

| gene | rat(NM_001014070.1)(SEQ ID NO:7) | Locus | 1257-1275 BP | |
|---|---|---|---|---|
| | SIRNA | Stability | Expression inhibition ratio | Growth inhibition ratio |
| 167-1 | Unmodified siRNA<br>5-GAAGAGCCUACACAACCCUTT<br>TTCUUCUCGGAUGUGUUGGGA-5 | + | 76% | 0% |
| 167-2 | Random modification (2'-O-methyl)<br>5-**G**A**A**G**A**G**C**C**U**A**C**A**C**A**A**C**C**C**U**TT<br>TTCUUCUC**GG**AU**G**U**G**UU**GGG**A-5 | ++ | 43% | 31% |
| 167-3A | Specific modification (2'-O-methyl)<br>5-GAAGAGCC**UAC**A**C**AACCCUTT<br>TTCUUCUCGGA**U**G**U**G**U**UGGGA-5 | ++ | 74% | 10% |
| 167-3B | Specific modification (2'-O-methyl)<br>5-GAAGAGCCUA**C**A**C**AACCCUTT<br>TTCUUCUCGGA**U**GUG**U**UGGGA-5 | ++ | 74% | 3% |

Table 168

| gene | Rat BIRC5(NM_022274)(SEQ ID NO:8) | Locus | 364-382 BP | |
|---|---|---|---|---|
| | SIRNA | Stability | Expression inhibition ratio | Growth inhibition ratio |
| 168-1 | Unmodified siRNA<br>5-ACAGAAAGAGUUCGAGGAGTT<br>TTUGUCUUUCUCAAGCUCCUC-5 | + | 65% | 0% |
| 168-2 | Random modification (2'-O-methyl)<br>5-**A**C**A**G**A**A**A**G**A**G**U**U**C**G**A**G**G**A**G**TT<br>TTU**G**UCUUUCUCAA**G**CUCCUC-5 | ++ | 37% | 28% |
| 168-3A | Specific modification (2'-O-methyl)<br>5-A**CA**GAAAG**A**GUUCGAGGAGTT<br>TTUG**U**CUUUCUCAAGCUCCUC-5 | ++ | 61% | 6% |
| 168-3B | Specific modification (2'-O-methyl)<br>5-A**C**AGAAAGAGUUCGAGGAGTT<br>TTUG**U**CUUUCUCAAGCUCCUC-5 | ++ | 64% | 1% |

Table 169

| gene | Rat BIRC5(NM_022274)(SEQ ID NO:8) SIRNA | Locus Stability | 527-545 BP Expression inhibition ratio | Growth inhibition ratio |
|---|---|---|---|---|
| 169-1 | Unmodified siRNA<br>5-AGGAGACCGUGACAUUUCGTT<br>TTUCCUCUGGCACUGUAAAGC-5 | + | 75% | 0% |
| 169-2 | Random modification (2'-deoxy-2'-fluoro)<br>5-**A**G**G**A**G**A**C**C**G**U**G**A**C**A**U**U**U**C**G**TT<br>TTUCCUCU**GG**CACU**G**UAAA**G**C-5 | +++ | 33% | 24% |
| 169-3A | Specific modification (2'-deoxy-2'-fluoro)<br>5-AGGAGACCG**U**GA**C**AUUUCGTT<br>TTUCCUCUGGC**AC**UG**U**AAAGC-5 | +++ | 69% | 6% |
| 169-3B | Specific modification (2'-deoxy-2'-fluoro)<br>5-AGGAGACCG**U**GA**C**AUUUCGTT<br>TTUCCUCUGGCACUG**U**AAAGC-5 | +++ | 72% | 3% |

Table 170

| gene | Rat BIRC5(NM_022274)(SEQ ID NO:8 SIRNA | Locus Stability | 42-60 BP Expression inhibition ratio | Growth inhibition ratio |
|---|---|---|---|---|
| 170-1 | Unmodified siRNA<br>5-ACCUUAAGGACCACCGGAUTT<br>TTUGGAAUUCCUGGUGGCCUA-5 | + | 73% | 0% |
| 170-2 | Random modification (2'-deoxy-2'-fluoro)<br>5-**A**C**C**U**U**A**A**G**G**A**C**C**A**C**C**G**G**A**U**TT<br>TTUGG**AA**UUCCUGGUGGCCU**A**-5 | ++ | 41% | 25% |
| 170-3A | Specific modification (2'-deoxy-2'-fluoro)<br>5-ACCU**U**AAGGAC**CA**CCGGAUTT<br>TTUGGA**AU**UCCUGG**U**GGCCUA-5 | ++ | 70% | 8% |
| 170-3B | Specific modification (2'-deoxy-2'-fluoro) | | | |

(continued)

| gene | Rat BIRC5(NM_022274)(SEQ ID NO:8 SIRNA | Locus Stability | 42-60 BP Expression inhibition ratio | Growth inhibition ratio |
|---|---|---|---|---|
| | 5-ACCU**U**AAGGACCACCGGAUTT<br>TTUGGAAUUCCUGG**U**GGCCUA-5 | ++ | 71% | 1% |

Table 171

| gene | Rat BIRC5(NM_022274)(SEQ ID NO:8) SIRNA | Locus Stability | 56-74 BP Expression inhibition ratio | Growth inhibition ratio |
|---|---|---|---|---|
| 171-1 | Unmodified siRNA | | | |
| | 5-CGGAUCUACACCUUCAAGATT<br>TTGCCUAGAUGUGGAAGUUCU-5 | + | 44% | 0% |
| 171-2 | Random modification (2'-O-methyl) | | | |
| | 5-**C**G**G**A**U**C**U**A**C**A**C**C**U**U**C**A**A**G**A**TT<br>TTGCC**U**AGA**U**G**U**GGAAG**UU**C**U**-5 | ++ | 29% | 27% |
| 171-3A | Specific modification (2'-O-methyl) | | | |
| | 5-CGGAUC**UAC**ACCUU**C**AAGATT<br>TTGCCUAGA**U**G**U**GGAAG**U**UCU-5 | ++ | 40% | 9% |
| 171-3B | Specific modification (2'-O-methyl) | | | |
| | 5-CGGAUC**U**ACACCUUC**A**AGATT<br>TTGCCUAGAUG**U**GGAAGUUCU-5 | ++ | 42% | 2% |

Table 172

| gene | Rat BIRC5(NM_022274)(SEQ ID NO:8) SIRNA | Locus Stability | 183-201 BP Expression inhibition ratio | Growth inhibition ratio |
|---|---|---|---|---|
| 172-1 | Unmodified siRNA | | | |
| | 5-GCUUUAAGGAACUGGAAGGTT<br>TTCGAAAUUCCUUGACCUUCC-5 | + | 77% | 0% |
| 172-2 | Random modification (2'-O-methyl) | | | |
| | 5-**G**C**U**UU**A**AG**G**A**A**C**U**G**G**A**A**G**G**TT<br>TTC**G**AAAUUCCUU**G**ACCUUCC-5 | ++ | 51% | 29% |
| 172-3A | Specific modification (2'-O-methyl) | | | |

(continued)

| gene | Rat BIRC5(NM_022274)(SEQ ID NO:8) | Locus | 183-201 BP | |
|---|---|---|---|---|
| | SIRNA | Stability | Expression inhibition ratio | Growth inhibition ratio |
| | 5-GCUU**UA**AGGAAC**U**GGAAGGTT<br>TTCGAA**AU**UCCUUGA**C**CUUCC-5 | ++ | 72% | 8% |
| 172-3B | Specific modification (2'-O-methyl) | | | |
| | 5-GCUUU**A**AGGAAC**U**GGAAGGTT<br>TTCGAAAUUCCUUGA**C**CUUCC-5 | ++ | 74% | 1% |

Table 173

| gene | Mice (NM_008960.2)(SEQ ID NO:9) | Locus | 290-308 BP | |
|---|---|---|---|---|
| | SIRNA | Stability | Expression inhibition ratio | Growth inhibition ratio |
| 173-1 | Unmodified siRNA | | | |
| | 5-GAGAGAGACGGUGGAAGAATT<br>TTCUCUCUCUGCCACCUUCUU-5 | + | 75% | 0% |
| 173-2 | Random modification (2'-O-methyl) | | | |
| | 5-**G**A**G**A**G**A**G**A**C**G**G**U**G**G**A**A**G**A**A**TT<br>TTCUCUCUCU**G**CCACCUUCUU-5 | +++ | 56% | 22% |
| 173-3A | Specific modification (2'-O-methyl) | | | |
| | 5-GAGAGAGACGG**UG**GAAGAATT<br>TTCUCUCUCUGCCA**C**CUUCUU-5 | +++ | 72% | 4% |
| 173-3B | Specific modification (2'-O-methyl) | | | |
| | 5-GAGAGAGACGGUGGAAGAATT<br>TTCUCUCUCUGCC**A**CCUUCUU-5 | +++ | 74% | 1% |

Table 174

| gene | Mice (NM_008960.2)(SEQ ID NO:9) | Locus | 791-809 BP | |
|---|---|---|---|---|
| | SIRNA | Stability | Expression inhibition ratio | Growth inhibition ratio |
| 174-1 | Unmodified siRNA | + | 53% | 0% |
| | 5-AUCCUGCAGAAGAAGCCUCTT<br>TTUAGGACGUCUUCUUCGGAG-5 | | | |
| 174-2 | Random modification (2'-O-methyl) | | | |
| | 5-**A**U**C**C**U**G**C**A**G**A**A**G**A**A**G**C**C**U**C**TT<br>TTUAGGA**C**GU**C**UU**C**UU**C**GGAG-5 | ++ | 37% | 24% |

(continued)

| gene | Mice (NM_008960.2)(SEQ ID NO:9) SIRNA | Locus Stability | 791-809 BP Expression inhibition ratio | Growth inhibition ratio |
|---|---|---|---|---|
| 174-3A | Specific modification (2'-O-methyl)<br>5-AUCC**U**G**C**AGAAGAA**G**CCUCTT<br>TTUAGGA**C**G**U**CUUCUUCGGAG-5 | ++ | 50% | 6% |
| 174-3B | Specific modification (2'-O-methyl)<br>5-AUCC**U**G**C**AGAAGAAGCCUCTT<br>TTUAGGACGUCUUCUUCGGAG-5 | ++ | 74% | 1% |

Table 175

| gene | Mice (NM_008960.2)(SEQ ID NO:9) SIRNA | Locus Stability | 247-265 BP Expression inhibition ratio | Growth inhibition ratio |
|---|---|---|---|---|
| 175-1 | Unmodified siRNA<br>5-UCCUCGUCGUCUGUUCUAATT<br>TTAGGAGCAGCAGACAAGAUU-5 | + | 84% | 0% |
| 175-2 | Random modification (2'-deoxy-2'-fluoro)<br>5-**U**C**C**U**C**G**U**C**G**U**C**U**G**U**U**C**U**A**A**TT<br>TTAGGAG**C**AG**C**AGA**C**AAGAUU-5 | ++ | 49% | 23% |
| 175-3A | Specific modification (2'-deoxy-2'-fluoro)<br>5-UCCUCGUC**G**UC**UG**UUC**U**AATT<br>TTAGGAGCAGCAGA**C**AAGA**U**U-5 | ++ | 77% | 6% |
| 175-3B | Specific modification (2'-deoxy-2'-fluoro)<br>5-UCCUCGUCGUCUGUUC**U**AATT<br>TTAGGAGCAGCAGA**C**AAGA**U**U-5 | ++ | 74% | 1% |

Table 176

| gene | Mice (NM_008960.2)(SEQ ID NO:9) SIRNA | Locus Stability | 1148-1166 BP Expression inhibition ratio | Growth inhibition ratio |
|---|---|---|---|---|
| 176-1 | Unmodified siRNA<br>5-AACCCACCACAGCUAGAACUU<br>UUUUGGGUGGUGUCGAUCUUG-5 | + | 89% | 0% |

(continued)

| gene | Mice (NM_008960.2)(SEQ ID NO:9) SIRNA | Locus Stability | 1148-1166 BP Expression inhibition ratio | Growth inhibition ratio |
|---|---|---|---|---|
| 176-2 | Random modification (2'-deoxy-2'-fluoro)<br>5-AACCCACCACAGCUAGAACUU<br>UUUUGGGUGGUGUCGAUCUUG-5 | +++ | 57% | 21% |
| 176-3A | Specific modification (2'-deoxy-2'-fluoro)<br>5-AACCCACCACAGCUAGAACUU<br>UUUUGGGUGGUGUCGAUCUUG-5 | +++ | 79% | 9% |
| 176-3B | Specific modification (2'-deoxy-2'-fluoro)<br>5-AACCCACCACAGCUAGAACUU<br>UUUUGGGUGGUGUCGAUCUUG-5 | +++ | 86% | 1% |

Table 177

| gene | Mice (NM_008960.2)(SEQ ID NO:9) SIRNA | Locus Stability | 3694-3712 BP Expression inhibition ratio | Growth inhibition ratio |
|---|---|---|---|---|
| 177-1 | Unmodified siRNA<br>5-UUACAAAGGAUCUCCUCCCTT<br>TTAAUGUUUCCUAGAGGAGGG-5 | + | 77% | 0% |
| 177-2 | Random modification (2'-O-methyl)<br>5-UUACAAAGGAUCUCCUCCCTT<br>TTAAUGUUUCCUAGAGGAGGG-5 | ++ | 51% | 29% |
| 177-3A | Specific modification (2'-O-methyl)<br>5-UUACAAAGGAUCUCCUCCCTT<br>TTAAUGUUUCCUAGAGGAGGG-5 | ++ | 72% | 6% |
| 177-3B | Specific modification (2'-O-methyl)<br>5-UUACAAAGGAUCUCCUCCCTT<br>TTAAUGUUUCCUAGAGGAGGG-5 | ++ | 74% | 1% |

Table 178

| gene | Aspergillus AF293 protein(XM_748400) (SEQ ID NO:10) SIRNA | Locus Stability | 75-93 BP Expression inhibition ratio | Growth inhibition ratio |
|---|---|---|---|---|
| 178-1 | Unmodified siRNA | | | |

(continued)

| gene | Aspergillus AF293 protein(XM_748400) (SEQ ID NO:10) | Locus | 75-93 BP | |
|---|---|---|---|---|
| | SIRNA | Stability | Expression inhibition ratio | Growth inhibition ratio |
| | 5-AAACGAGAGAGCCCACCUUTT<br>TTUUUGCUCUCUCGGGUGGAA-5 | + | 58% | 0% |
| 178-2 | Random modification (2'-O-methyl)<br>5-**A**A**A**C**G**A**G**A**G**C**C**C**A**C**C**U**U**TT<br>TTUUUG**C**U**C**U**C**U**C**GGGUGGAA-5 | +++ | 37% | 27% |
| 178-3A | Specific modification (2'-O-methyl)<br>5-AAACGA**G**AGAGCC**C**ACCUUTT<br>TTUUUGCUCUCUCGG**GU**GGAA-5 | +++ | 56% | 4% |
| 178-3B | Specific modification (2'-O-methyl)<br>5-AAACGAGAGAGCCCACCUUTT<br>TTUUUGCUCUCUCGGG**U**GGAA-5 | +++ | 57% | 1% |

Table 179

| gene | Aspergillus AF293 protein(XM_748400) (SEQ ID NO: 10) | Locus | 137-155 BP | |
|---|---|---|---|---|
| | SIRNA | Stability | Expression inhibition ratio | Growth inhibition ratio |
| 179-1 | Unmodified siRNA<br>5-AAAGCGGUGGAUCAGCUCUTT<br>TTUUUCGCCACCUAGUCGAGA-5 | + | 69% | 0% |
| 179-2 | Random modification (2'-O-methyl)<br>5-**A**A**A**G**C**G**G**U**G**G**A**U**C**A**G**C**U**C**U**TT<br>TTUUUC**G**CCACCUAGUC**G**A**G**A-5 | ++ | 43% | 24% |
| 179-3A | Specific modification (2'-O-methyl)<br>5-AAAGCGG**UG**GAU**C**AGCUCUTT<br>TTUUUCGCC**AC**CUAG**U**CGAGA-5 | ++ | 63% | 10% |
| 179-3B | Specific modification (2'-O-methyl)<br>5-AAAGCGGUGGAUCAGCUCUTT<br>TTUUUCGCCA**C**CUAG**U**CGAGA-5 | ++ | 67% | 1% |

Table 180

| gene | Aspergillus AF293 protein(XM_748400) (SEQ ID NO:10) | Locus | 360-378 | BP |
|---|---|---|---|---|
| | SIRNA | Stability | Expression inhibition ratio | Growth inhibition ratio |
| 180-1 | Unmodified siRNA<br>5-UCCAAGCUUCCUCGUUGGUTT<br>TTAGGUUCGAAGGAGCAACCA-5 | + | 71% | 0% |
| 180-2 | Random modification (2'-deoxy-2'-fluoro)<br>5-**U**C**C**A**A**G**C**U**U**C**C**U**C**G**U**U**G**G**U**TT<br>TTAGG**UU**CGAAGGAGCAACCA-5 | ++ | 51% | 28% |
| 180-3A | Specific modification (2'-deoxy-2'-fluoro)<br>5-UC**CA**AGCUUCCUCGU**UG**GUTT<br>TTAG**GU**UCGAAGGAGCA**AC**CA-5 | ++ | 65% | 7% |
| 180-3B | Specific modification (2'-deoxy-2'-fluoro)<br>5-UCCAAGCUUCCUCGUUGGUTT<br>TTAGG**U**UCGAAGGAGCAA**C**CA-5 | ++ | 71% | 0% |

Table 181

| gene | Aspergillus AF293 protein (XM_748400) (SEQ ID NO:10) | Locus | 14-32 BP | |
|---|---|---|---|---|
| | SIRNA | Stability | Expression inhibition ratio | Growth inhibition ratio |
| 181-1 | Unmodified siRNA<br>5-AGAGAGGUGGAGGUAUCUCTT<br>TTUCUCUCCACCUCCAUAGAG-5 | + | 90% | 0% |
| 181-2 | Random modification (2'-deoxy-2'-fluoro)<br>5-**A**G**A**G**A**G**G**U**G**G**A**G**G**U**A**U**C**U**C**TT<br>TTUCUCUCC**A**CCUCC**A**U**A**G**A**G-5 | +++ | 68% | 26% |
| 181-3A | Specific modification (2'-deoxy-2'-fluoro)<br>5-AGAGAGG**U**GGAGG**U**AUC**U**CTT<br>TTUCUCUCC**AC**CUCCA**U**AGAG-5 | +++ | 85% | 7% |
| 181-3B | Specific modification (2'-deoxy-2'-fluoro) | | | |

(continued)

| gene | Aspergillus AF293 protein (XM_748400) (SEQ ID NO:10) | Locus | 14-32 BP | |
|---|---|---|---|---|
| | SIRNA | Stability | Expression inhibition ratio | Growth inhibition ratio |
| | 5-AGAGAGG**U**GGAGGUAUCUCTT<br>TTUCUCUCCACCUCCA**U**AGAG-5 | +++ | 88% | 1% |

Table 182

| gene | Aspergillus AF293 protein(XM_748400) (SEQ ID NO: 10) | Locus | 1238-1256 BP | |
|---|---|---|---|---|
| | SIRNA | Stability | Expression inhibition ratio | Growth inhibition ratio |
| 182-1 | Unmodified siRNA<br>5-CCGAAGACGAAGCUAUGAATT<br>TTGGCUUCUGCUUCGAUACUU-5 | + | 73% | 0% |
| 182-2 | Random modification (2'-O-methyl)<br>5-**C**C**G**A**A**G**A**C**G**A**A**G**C**U**A**U**G**A**A**TT<br>TT**GG**CUUCU**G**CUUC**G**AUACUU-5 | +++ | 51% | 29% |
| 182-3A | Specific modification (2'-O-methyl)<br>5-CCGAAGACG**AA**GC**U**A**U**GAATT<br>TTGGCUUCUGCUUCGA**U**A**C**UU-5 | +++ | 69% | 6% |
| 182-3B | Specific modification (2'-O-methyl)<br>5-CCGAAGACGAAGC**U**AUGAATT<br>TTGGCUUCUGCUUCGAUA**C**UU-5 | +++ | 71% | 1% |

In tables 1-182:
Chemically modified nucleotides were indicated by bold letters of "A", "U", "G" or "C";
"+" indicates the situation that after serum treatment, the main siRNA band disappeared completely, while evident siRNA degradation bands were observed;
"++" indicates the situation that after serum treatment, the main siRNA band remains visible although degradaion occurs, at the same time, siRNA degradation bands are easily observed;
"+++" indicates the situation that after serum treatment, no obvious degradation occurs with the main siRNA band, no siRNA degradation band is observed.
In addition, as shown in table 1-182, the specific siRNA modification strategy provided by the present invention greatly improves serum stability of the siRNAs; while at the same time, reduces their cytotoxicity and does not compromise their gene silencing activity.
Thus, the present invention can achieve siRNA stabilization with minimal chemical modifications, decreases their potential cytotoxicity and influence on gene silencing activity.

## Claims

1. A modified oligonucleotide comprising a first nucleic acid fragment and a second nucleic acid fragment, the first nucleic acid fragment and the second nucleic acid fragment can form double-stranded region, wherein the modified oligonucleotide has at least one of the following modifications, the modification(s) improve the stability of the modified oligonucleotide in mammalian body fluids;

   (a) The first nucleic acid fragment contains at least one contiguous UA sequence, the second nucleic acid fragment contains at least one contiguous UA sequence complementary to the UA sequence in the first nucleic acid fragment, the UA sequence(s) of the first nucleic acid fragment and the complementary UA sequence(s) of the second nucleic acid fragment pair to form UA/UA site(s); wherein at least one of the nucleotides in at least one of the UA/UA site(s) is a modified nucleotide;
   (b) The first nucleic acid fragment contains at least one contiguous CA or UG sequence, the second nucleic acid fragment contains at least one contiguous CA or UG sequence complementary to the CA or UG sequence in the first nucleic acid fragment, the CA or UG sequence(s) of the first nucleic acid fragment and the CA or UG sequence(s) of the second nucleic acid fragment pair to form CA/UG and/or UG/CA site(s); wherein at least one of the nucleotides in at least one of the CA/UG and/or UG/CA site(s) is a modified nucleotide;
   (c) The first nucleic acid fragment contains at least one contiguous UA sequence and at least one contiguous CA or UG sequence, the second nucleic acid fragment contains at least one contiguous UA sequence complementary to the UA sequence of the first nucleic acid fragment and one contiguous CA or UG sequence complementary to the CA or UG sequence of the first nucleic acid fragment, the UA sequence(s) of the first nucleic acid fragment and the UA sequence(s) of the second nucleic acid fragment pair to form UA/UA site(s), the CA or UG sequence(s) of the first nucleic acid fragment and the UG or CA sequence(s) of the second nucleic acid

fragment pair to form CA/UG or UG/CA site(s); wherein at least one of the nucleotides in at least one of the UA/UA site(s) is a modified nucleotide, and at least one of the nucleotides in at least one of the CA/UG or UG/CA site(s) is a modified nucleotide.

**2.** The modified oligonucleotide of claim 1, wherein the oligonucleotide contains at least one UA/UA and/or CA/UG and/or UG/CA site(s).

**3.** The modified oligonucleotide of claim 1, wherein only one uridine nucleotide in all occurrences of the UA/UA site (s) is a modified nucleotide.

**4.** The modified oligonucleotide of claim 1, wherein only cytidine nucleotide(s) in all occurrences of the CA/UG or UG/CA site is/are modified nucleotide(s).

**5.** The modified oligonucleotide of any one of claims 1 to 4, wherein all the nucleotides are unmodified nucleotides except the nucleotides in said CA/UG and/or UA/UA and/or UG/CA site(s).

**6.** The modified oligonucleotide of any one of claims 1 to 5, wherein the first nucleic acid fragment and the second nucleic acid fragment are in two separate nucleic acid strands or in a single nucleic acid strand.

**7.** The modified oligonucleotide of claim 6, wherein the modified oligonucleotide is a hairpin-structured and single-stranded nucleic acid molecule, the complementary nucleic acid sequences of the first nucleic acid fragment and the second nucleic acid fragment pair to form double-stranded region.

**8.** The modified oligonucleotide of claim 6, wherein the modified oligonucleotide contains a sense strand and an antisense strand, the sense strand and the antisense strand are contiguous nucleic acid fragments; the first nucleic acid fragment is in the sense strand, and the second nucleic acid fragment is in the antisense strand.

**9.** The modified oligonucleotide of claim 6, wherein the modified oligonucleotide contains a sense strand and an antisense strand, the sense strand contains two or more discontiguous sense-strand component fragments, the antisense strand is a contiguous nucleic acid fragment; the first nucleic acid fragment is in one or more sense-strand component fragments, the second nucleic acid fragment is in the antisense strand.

**10.** The modified oligonucleotide of any one of claims 1 to 5, wherein the modified oligonucleotide is a hybrid nucleic acid molecule comprising ribonucleotides and at least one deoxyribonucleotide.

**11.** The modified oligonucleotide of any one of claims 1 to 5, wherein the modified oligonucleotide has at least one of the following modifications:

(a) modifying the phosphodiester bond(s) connecting nucleotides in the oligonucleotide;
(b) modifying the sugar(s) of the nucleotide in the oligonucleotide;
(c) modying the base(s) of the nucleotide in the oligonucleotide.

**12.** The modified oligonucleotide of claim 11, wherein the modification is replacing a 2'-OH group on the sugar with a 2'-modified nucleotide.

**13.** The modified oligonucleotide of claim 12, wherein the modification is replacing a 2'-OH group on the sugar with a 2'-O-methyl group or a 2'-deoxy-2'-fluoro group.

**14.** A pharmaceutical composition for inhibiting the expression of a target gene in a mammal, wherein the pharmaceutical composition comprises at least one modified oligonucleotide of any one of claims 1 to 13, and a pharmaceutically acceptable carrier.

**15.** A method for preparing the pharmaceutical composition of claim 14, wherein the method comprises formulating the oligonucleotide of any one of claims 1 to 13 and a pharmaceutically acceptable carrier.

**16.** A method for inhibiting the expression of a target gene in a cell, compreseing:

(a) introducing the oligonucleotide of any one of claims 1 to 13 into the cell; and

(b) incubating the cell for a time sufficient to obtain inhibition of the expression of the target gene in the cell.

**17.** The method of claim 16, wherein the cell is a mammalian cell.

**18.** A method for preparing an oligonucleotide that is highly stable in a biological sample and can inhibite the expression of a target gene, comprising:

(a) selecting one or more nucleic acid sequences of between 18 and 30 nucleotides in length from the nucleotide sequence of the mRNA resulting from the transcription of the target gene; and
(b) synthesizing the selected sequences, wherein one strand comprises a sequence complementary to selected sequence in (a); and
(c) testing one or more oligonucleotides of (b) for their activity to inhibit the expression of the target gene in a biological sample; and
(d) selecting one or more oligonucleotides of (c) possessing the activity to inhibit the expression of the target gene in a biological sample; and
(e) in the oligonucleotides selected in (d), identifying in the nucleotide sequences of all occurrences of the UA/UA, CA/UG and UG/CA site(s); and
(f) synthesizing one or more oligonucleotides selected in (d), wherein at least one nucleotide in the UA/UA, CA/UG and UG/CA site(s) identified in (e) is replaced by a corresponding modified nucleotide.

**19.** The method of claim 18, wherein only cytidine nucleotide(s) in all occurrences of the CA/UG or UG/CA site is/are modified.

**20.** The method of claim 18, wherein only one uridine nucleotide in each UA/UA site is modified.

**21.** The method of claim 18, wherein all the nucleotides are unmodified nucleotides except the nucleotides in said CA/UG and/or UA/UA and/or UG/CA site(s).

**22.** A method of treating a disease caused by expression of a target gene in a subject, said method comprises administering to a said subject a pharmaceutical composition comprising the oligonucleotide of any one of claims 1 to 13 and a pharmaceutically acceptable carrier.

**23.** The method of claim 22, wherein the subject is human beings.

**24.** A method for preparing the oligonucleotide with nuclease resistance, said method comprises the steps of:

(a) identifying in the nucleotide sequence of the oligonucleotide all occurrences of UA/UA, CA/UG and UG/CA site(s); and
(b) synthesizing the oligonucleotide, wherein at least one nucleotide in the UA/UA, CA/UG and UG/CA site(s) identified in (a) is replaced by a modified corresponding nucleotide.

**25.** The method of claim 24, wherein only cytidine nucleotide(s) in all occurrences of CA/UG or UG/CA site is/are modified.

**26.** The method of claim 24, wherein only one uridine nucleotide in all occurrences of UA/UA site(s) is modified.

**27.** The method of claim 24, wherein all the nucleotides are unmodified nucleotides except the nucleotides in said CA/UG and/or UA/UA and/or UG/CA site(s).

**28.** A method to identify an oligonucleotide with stability in biological samples, comprises the steps of:

(a) providing a first modified oligonucleotide of claim 1, and a second oligonucleotide identical in sequence to the first oligonucleotide except that it dose not have modified nucleotide(s) of the oligonucleotide of claim 1; and
(b) determining the stability and the degradation process of said first and second oligonucleotide in a biological sample by contacting the two oligonucleotides with the biological sample under indentical conditions,

Whereby, where the first modified oligonucleotide is degraded less rapidly than the second oligonucleotide, an oligonucleotide with stability in a biological sample is identified.

| INTERNATIONAL SEARCH REPORT | International application No. PCT/CN2010/000405 |
|---|---|

**A. CLASSIFICATION OF SUBJECT MATTER**

**See extra sheet**

According to International Patent Classification (IPC) or to both national classification and IPC

B. FIELDS SEARCHED

Minimum documentation searched (classification system followed by classification symbols)

IPC: C12N, A61K, A61P, C12Q

Documentation searched other than minimum documentation to the extent that such documents are included in the fields searched

Electronic data base consulted during the international search (name of data base and, where practicable, search terms used)

WPI, EPODOC, CNPAT, CNKI, MEDLINE, BA, CA: UA, CA, UG, RNA, siRNA, RNAi, iRNA, dsRNA, oligonucleotide, modified, modifying, modification, interference, interfering

C. DOCUMENTS CONSIDERED TO BE RELEVANT

| Category* | Citation of document, with indication, where appropriate, of the relevant passages | Relevant to claim No. |
|---|---|---|
| X | WO2008152131A2 (NOVARTIS AG), 18 Dec. 2008 (18.12.2008), see page 2, line 10 to page 3, line 19; page 9, line 18 to line 28; page 19, line 16 to page 21, line 25; page 26, line 16 to page 28, line 27; table A and 1A-1D; example 3; claims 1-22 | 1-2, 6-8, 10-18, 24, 28 |
| X | CN101370818A (ALNYLAM PHARM INC), 18 Feb. 2009 (18.02.2009), see page 3, line 3 to page 4, line 7; page 12, line 6 to line 21; page 52, line 23 to page 53, line 6; page 56, line 17 to page 58, line 19; page 62, line 16 to | 1-2, 6-8, 10-18, 24, 28 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 28 May 2010 (28.05.2010) | **17 Jun. 2010 (17.06.2010)** |
| Name and mailing address of the ISA/CN<br>The State Intellectual Property Office, the P.R.China<br>6 Xitucheng Rd., Jimen Bridge, Haidian District, Beijing, China 100088<br>Facsimile No. 86-10-62019451 | Authorized officer<br>SONG, Zhigang<br>Telephone No. (86-10)62411078 |

Form PCT/ISA /210 (second sheet) (July 2009)

## INTERNATIONAL SEARCH REPORT

International application No. PCT/CN2010/000405

C (Continuation). DOCUMENTS CONSIDERED TO BE RELEVANT

| Category* | Citation of document, with indication, where appropriate, of the relevant passages | Relevant to claim No. |
|---|---|---|
| | page 64, line 22; table 1B, 1H and 3; example 3; claims 1-23 | |
| X | CN1663957A(UNIV ZHEJIANG),<br>7 Sep. 2005 (07.09.2005), see pages 2-3; examples 2-4; table 1; claims 1-8 | 1-2, 6-8, 10-17 |
| A | CN1860228A (ANGES MG INC),<br>8 Nov. 2006 (08.11.2006), see the whole document | 1-21,24-28 |

Form PCT/ISA /210 (continuation of second sheet ) (July 2009)

## INTERNATIONAL SEARCH REPORT

International application No.
PCT/CN2010/000405

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 22-23
because they relate to subject matter not required to be searched by this Authority, namely:
claims 22-23 are directed to a method of treatment of the human/animal body

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fees, this Authority did not invite payment of additional fee.
3. ☐ As only some of the required additional search fees were timely paid by the applicant, this international search report covers only those claims for which fees were paid, specifically claims Nos.:
4. ☐ No required additional search fees were timely paid by the applicant. Consequently, this international search report is restricted to the invention first mentioned in the claims; it is covered by claims Nos.:

**Remark on protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA /210 (continuation of first sheet (2)) (July 2009)

**INTERNATIONAL SEARCH REPORT**
Information on patent family members

International application No.
PCT/CN2010/000405

| Patent Documents referred in the Report | Publication Date | Patent Family | Publication Date |
|---|---|---|---|
| WO2008152131A2 | 18-12-2008 | CA2690674A | 18-12-2008 |
| | | AU2008263876A1 | 18-12-2008 |
| | | TW200911989 A | 16-03-2009 |
| | | WO2008152131 A3 | 28-05-2009 |
| | | US2009156529A1 | 18-06-2009 |
| | | PE09422009A | 05-08-2009 |
| | | AR066984A | 23-09-2009 |
| | | CR11136A | 20-01-2010 |
| | | ECSP099794A | 29-01-2010 |
| | | EP2171059 A | 07-04-2010 |
| CN101370818A | 18-02-2009 | WO2007053696A2 | 10-05-2007 |
| | | CA2627585A1 | 10-05-2007 |
| | | AU2006308716A1 | 10-05-2007 |
| | | US2007197460A1 | 23-08-2007 |
| | | WO2007053696 A3 | 17-01-2008 |
| | | MXPA08005572 A | 31-07-2008 |
| | | EP1951737A2 | 06-08-2008 |
| | | INDELNP200803627E | 15-08-2008 |
| | | KR20080086440A | 25-09-2008 |
| | | JP2009513716T | 02-04-2009 |
| | | RU2008121953A | 10-12-2009 |
| CN1663957A | 07-09-2005 | CN1314698C | 09-05-2007 |
| CN1860228A | 08-11-2006 | WO2005030960A1 | 07-04-2005 |
| | | CA2538215A | 07-04-2005 |
| | | AU2004276684A1 | 07-04-2005 |
| | | EP1669450A1 | 14-06-2006 |
| | | JP2005514320T2 | 07-12-2006 |
| | | US2006276421A | 07-12-2006 |
| | | US7595301B | 29-09-2009 |

Form PCT/ISA /210 (patent family annex) (July 2009)

**INTERNATIONAL SEARCH REPORT**

International application No.
PCT/CN2010/000405

**A. CLASSIFICATION OF SUBJECT MATTER**

C12N 15/113 (2010.01) i
A61K 48/00 (2006.01) i
A61K 31/7115 (2006.01) i
A61K 31/712 (2006.01) i
A61K 31/7125 (2006.01) i
A61K 31/713 (2006.01) i
A61P 35/00 (2006.01) i
C12Q 1/68 (2006.01) i

Form PCT/ISA /210 (extra sheet) (July 2009)